# EUROPEAN PATENT APPLICATION

(11) **EP 2 769 723 A1**
(43) Date of publication of application: **27.08.2014**
(21) Application number: 13156371.0
(22) Date of filing: 22.02.2013
(51) Int. Cl.: A61K 31/517, A61P 31/18

(54) **Compounds for use in inhibiting HIV capsid assembly**

(71) Applicant: Ruprecht-Karls-Universität Heidelberg, 69117 Heidelberg (DE); Institute Of Organic Chemistry And Biochemistry As CR, V.V.I., 16610 Praha 6 (CZ)
(72) Inventor: Kräusslich, Hans-Georg, 68120 Heidelberg (DE); Sticht, Jana, 14195 Berlin (DE); Wildova, Marcela, 16500 Prag 6-Suchdol (CZ); Lux, Vanda, 40625 Düsseldorf (DE); Konvalinka, Jan, 16100 Prague 6 (CZ); Kotora, Martin, 153 00 Prague 5 (CZ); Grantz Sasková, Klára, 270 07 Mutejovice (CZ); Kozisek, Milan, 182 00 Prague 8 (CZ); Stepánek, Ondrej, 460 06 Liberec 6 (CZ); Parkan, Kamil, 130 00 Prague 3 (CZ); Machara, Ale, 169 00 Prague 6 (CZ)
(74) Representative: Dick, Alexander

(57) **Abstract**

The present invention relates to a compound or a pharmaceutically acceptable salt or solvate thereof for use in inhibiting HIV capsid assembly, the compound comprising the core structure 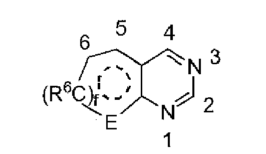 wherein E is CR⁷or S, and wherein f is 0 or 1, and wherein in case E is S, f is 0, and wherein the core structure is at least substituted in 2 and 4 position, and wherein the residue R6 and R⁷, are, independently of each other, selected from the group consisting of -H, -D, -alkyl, alkoxy, alkenyl, alkynyl, halides, -NO₂, - OH, - NH₂, -NHR^{4#}, -CN, -S(O)R^{4#}, -SO₂R^{4#}, -P(O)R^{4#}R^{5#}, -P(O)(OR^{4#})R^{5#}, - P(O)(OR^{4#})(OR^{5#}), -C(O)NR^{4#}R^{5#}, -C(O)SR^{4#}, -C(O)R^{4#}, -C(O)O-R^{4#}, alkoxy and glycol chains; and wherein R⁶ may optionally form a cyclic residue, with a further substituent present 5 or 6 position, and wherein R^{4#} and R^{5#} are, independently of each other, selected from the group consisting of -H, -alkyl, -alkenyl, - heterocycloalkyl, aryl and heteroaryl.

## Description

The present invention relates to compounds a pharmaceutically acceptable salts or solvates thereof for use in inhibiting HIV capsid assembly. The conjugates or pharmaceutically acceptable salt or solvate thereof comprise the following core structure 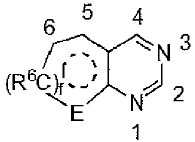 wherein E is S or CR⁷, and wherein f is 0 or 1, and wherein in case E is N, O or S, preferably S, f is 0, and wherein the core structure is at least substituted in 2 and 4 position.

The family of retroviruses comprises small enveloped virus particles (diameter ~100 nm) carrying two copies of a single-stranded positive sense RNA genome. All retroviruses possess three major genes encoding for the polyproteins Gag (structural proteins), Pol (enzymes) and Env (surface glycoproteins). The most studied member of the retroviral family is human immunodeficiency virus type 1 (HIV-1), the causative agent of acquired immunodeficiency syndrome (AIDS) with approx. 33.4 million people infected worldwide and 2 million deaths due to AIDS in 2008 alone. In the past two decades highly active antiretroviral therapy (HAART) has been implemented. There are two types of HIV, HIV-1 and HIV-2, with HIV-1 accounting for the majority of infections. Currently there are available many anti-HIV-1 drugs targeting mainly viral enzymes (e.g. nucleoside and non-nucleoside reverse transcriptase inhibitors, protease inhibitors, integrase inhibitors) or the viral entry into the host cell (fusion inhibitors and attachment inhibitors). Available drugs are nowadays used preferentially in combinations; still the occurrence of drug-resistant variants is the main problem in HIV-1 treatment. Therefore any new target in the HIV-1 life cycle would be useful tool in anti-retroviral therapy.

The Gag polyprotein alone is necessary and sufficient to form virus like particles. Its multimerization is mainly driven by intermolecular interactions between the capsid (CA), spacer peptide 1 (sp1) and nucleocapsid (NC) region of neighboring Gag molecules. The Gag polyprotein precursor radially lines the inside of the viral membrane and forms the spherical immature core. Concomitant with, or shortly after release, Gag is processed into matrix, CA, sp1, NC, spacer peptide 2 (sp2) and protein of 6 kDa (p6). CA then builds up the conical core of the mature virion which encloses the viral genome. Only after this maturation process the virus becomes infectious.

In 2005 was published by Jana Sticht et al. (Nature structural and molecular biology 12:671-677, 2005) a 12-mer peptide, capsid assembly inhibitor (CAI), that binds the capsid (CA) domain of Gag and inhibits assembly of immature- and mature-like particles *in vitro.* CAI was identified by phage display screening among a group of peptides with similar sequences that bind to a single reactive site in CA. Its binding site was mapped to CA residues 169-191, with an additional contribution from the last helix of CA. This result was confirmed by a separate X-ray structure analysis at the 1.7-Å-resolution showing that CAI inserts into a conserved hydrophobic groove and alters the CA dimer interface (F. Ternois et al. Nature structural and molecular biology 12:678-682, 2005). The CAI binding site was suggested as a new target for antiviral development, and CAI was the first known inhibitor directed against assembly of immature HIV-1.

The detail interaction between C-terminal CA (C-CA) and CAI was further investigated by Vanda Bartonova (JBC 283:32024-32033, 2008) and in this study were identified the amino acid residues essential for C-CA/CAI interaction. CA variants carrying mutations Y169A, L211A, or L211S had a reduced affinity for CAI and were unable to form mature-like particles *in vitro.* These mutations also blocked morphological conversion to mature virions in tissue culture and abolished infectivity. X-ray crystallographic analyses of the variant C-CA domains revealed that these alterations induced the same allosteric changes at the dimer interface observed in the C-CA/CAI complex.

Peptide inhibitors, however, have inherent drawbacks, as they usually have a short half-life and poor bioavailability. Accordingly it was shown that neither addition of the CAI peptide to virus-producing cells in culture nor peptide transfection caused any inhibition of HIV-1 release (J. Sticht et al. Nature structural and molecular biology 12:671-677, 2005). Thus, CAI cannot be directly used as an antiviral agent. Thus, there is a need for HIV capsid assembly inhibitors not having the drawbacks of the known capsid assembly inhibitors.

US 7,618,975 describes the use of 4-arylamino-quinazolines and of analogs thereof as activators of caspases and inducers of apoptosis. The compounds described in US 7,618,975 allow for induction of apoptosis, for inhibiting the assembly of tubulin and for inhibiting topoisomerase II. However, the document does not disclose that 4-arylamino-quinazolines can be used for inhibiting HIV capsid assembly.

Tian et al. (Bioorg. Med. Chem. Lett. 19 (2009) 2162-2167) discloses the use of acylhydrazone derivatives for inhibiting HIV-1 capsid assembly in vitro.

Li et al. (Bioorganic & Medicinal Chemistry 17 (2009) 3177-3188) discloses that thiourea compounds for inhibiting HIV-1 capsid assembly in vitro.

There is a need for effective HIV capsid assembly inhibitors. Thus, the present invention relates to a compound or pharmaceutically acceptable salts or solvates thereof for use in inhibiting HIV capsid assembly, the compounds comprising the following core structure 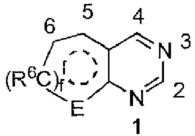 wherein E is S or CR⁷, and wherein f is 0 or 1, and wherein in case E is S, f is 0, and wherein the core structure is at least substituted in 2 and 4 position. As regards residue R⁶ and R⁷, said residues, are independently of each other, selected from the group consisting of -H, -D, -alkyl, alkoxy, alkenyl, alkynyl, halides, -NO₂, -OH, -NH₂, -NHR^{4#}, -CN, - S(O)R^{4#}, -SO₂R^{4#}, -P(O)R^{4#}R^{5#}, -P(O)(OR^{4#})R^{5#}, -P(O)(OR^{4#})(OR^{5#}), -C(O)NR^{4#}R^{5#}, - C(O)SR^{4#}, -C(O)R^{4#}, -C(O)O-R^{4#}, alkoxy and glycol chains; wherein R⁶ may optionally form a cyclic residue, with a further substituent present in 5 or 6 position, and wherein R^{4#} and R^{5#} are, independently of each other, selected from the group consisting of -H, -alkyl, -alkenyl, -heterocycloalkyl, aryl and heteroaryl,

Further, the present invention relates to a pharmaceutical composition for inhibiting HIV capsid assembly comprising the compound, as described above, in a therapeutically affective amount, and the use of a compound, as described above, for the manufacture of a medicament for inhibiting HIV capsid assembly.

Further, the present invention relates to a method of treating a patient suffering from HIV comprising administering a therapeutically effective amount of the compound as defined above, as well as the use of this compound for treating a patient suffering from HIV.

### The core structure:

The term "core structure" as used in the context of the present invention is denoted to mean that - besides the substitutions in 2 and 4 position, the shown structure may be further suitably substituted.

Within the meaning of the present invention, the term "alkyl" relates to non-branched alkyl residues and branched alkyl residues, as well as residues comprising one or more heteroatoms or functional groups, such as, by way of example, -O-, -S-, -NH-, -NH-C(=O)-, -C(=O)-NH-, and the like. The term also encompasses alkyl groups which are further substituted by one or more suitable substituent. Preferably, the alkyl groups according to the invention have from 1 to 20 carbon atoms, more preferably from 1 to 10 carbon atoms. Such alkyl groups are hereinunder also referred to as C1-20alkyl and C1-10alkyl, respectively.

The term "substituted" as used in the context of the present invention preferably refers to organic groups in which a hydrogen group, in any position, is replaced by one or more substituents, preferably by 1, 2, 3, 4, 5 or 6 substituents, more preferably by 1, 2, or 3 substituents. If two or more substituents are present, each substituent may be the same or may be different from the at least one other substituent. There are in general no limitations as to the substituent. The substituents may be, for example, selected from the group consisting of aryl, alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxy, phosphate, phosphonato, phosphinato, amino, acylamino, including alkylcarbonylamino, arylcarbonylamino, carbamoyl and ureido, amidino, nitro, imino, sulfhydryl, alkylthio, arylthio, thiocarboxylate, sulfates, alkylsulfinyl, sulfonate, sulfamoyl, sulfonamido, trifluoromethyl, cyano, azido, cycloalkyl such as e.g. cyclopentyl or cyclohexyl, heterocycloalkyl such as e.g. morpholino, piperazinyl or piperidinyl, alkylaryl, arylalkyl and heteroaryl. Preferred substituents of such organic residues are, for example, halogens, such as in particular fluorine or iodine, amino groups, hydroxyl groups, carbonyl groups, thiol groups and carboxyl groups.

Within the meaning of the present invention, the term "cyclic alkyl" or "cycloalkyl" relates to optionally suitably substituted to 8-membered single-ring alkyl groups as well as optionally suitably substituted multicyclic alkyl residues preferably having up to 20, more preferably up to 10 carbon atoms. Cycloalkyl groups residues having up to 20, preferably up to 10 carbon atoms, also referred to as C1-20cycloalkyl and C1-10cyclocloalkyl, respectively. By way of example, the following cycloalkyl groups are mentioned: cyclopentyl, cyclohexyl, cycloheptyl cyclopropylmethyl, cyclopentylmethyl, cyclohexylmethyl, cyclohexylcyclopentylmethyl, dicyclohexylmethyl, 1-cyclopentylethyl, 1-cyclohexylethyl, 2-cyclopropylethyl, 2-cyclopentylethyl, 2-cyclohexylethyl, 2-cycloheptylethyl, 1-cyclohexyl-1-methylethyl, 1-cyclohexylpropyl, 2-cyclopentylpropyl, 3-cyclobutylpropyl, 3-cyclopentylpropyl, 3- cyclohexylpropyl, 3-cycloheptylpropyl, 1-cyclopropyl-lmethylpropyl, 1-cyclohexyl-2-methylpropyl, 1-cyclopentyl butyl, 1-cyclohexylbutyl, 3-cyclohexylbutyl, 4-cyclopropylputyl, 4-cyclobutylbutyl, 4-cyclopentylbutyl, 1-cyclohexyl 1-methylbutyl, 1-cyclopentyl-2-ethylbutyl, 1-cyclohexyl-3methylbutyl, 1-cyclopentylpentyl, 1-cyclohexylpentyl, 1-cyclohexylmethylpentyl, 2-cyclohexylpentyl, 2-cyclohexylmethylpentyl, 3-cyclopentylpentyl, 1-cyclohexyl-4methylpentyl, 5-cyclopentylpentyl, 1-cyclopentylhexyl, 1-cyclohexylhexyl, 1-cyclopentylmethylhexyl, 2-cyclopentylhexyl, 2-cyclopropylethylhexyl, 3-cyclopentylhexyl, 1-cyclohexylheptyl, 1-cyclopentyl-1-methylheptyl, 1-cyclohexyl-1,6-dimethylheptyl, 1-cycloheptyloctyl, 2-cyclopentyloctyl, 3-cyclohexyloctyl, 2-cyclopentylmethyloctyl, 1-cyclopentylnonyl, 1-cyclohexylnonyl, 3-cyclopropylnonyl, 1-cyclopentyldecyl, 1-cyclohexylundecyl, 1-cyclopentyl tridecyl, 2-cyclohexyltridecyl, and the like

Within the meaning of the present invention, the term "heterocycloalkyl" relates to optionally suitably substituted 3 to 8-membered single-ring alkyl groups as well as optionally suitably substituted multicyclic alkyl residues having preferably up to 20, more preferably up to 10 carbon atoms, wherein the cyclic alkyl groups comprise one or more, preferably from 1 to 4 such as 1, 2, 3 or 4, heteroatoms, wherein in case the cycloalkyl residue comprises more than 1 heteroatom, the heteroatoms may be the same or different. Heterocycloalkyl groups residues having up to 20, preferably up to 10 carbon atoms, also referred to as C1-20heterocycloalkyl and C1-10heterocyclocloalkyl, respectively.

Preferably, the heteroatoms are selected from the group consisting of O, S and N.

The term "alkyl", "cycloalkyl", "cyclic alkyl" and "heterocycloalkyl", also encompasses groups which are further substituted by one or more suitable substituent.

Within the meaning of the present invention, the term "aryl" refers to, but is not limited to, optionally suitably substituted 5- and 6-membered single-ring aromatic groups as well as optionally suitably substituted multicyclic groups, for example bicyclic or tricyclic aryl groups. The term "aryl" thus includes, for example, optionally substituted phenyl groups or optionally suitably substituted naphthyl groups. Aryl groups can also be fused or bridged with alicyclic or heterocycloalkyl rings are not aromatic so as to form a polycycle, e.g., benzodioxolyl or tetraline. The term "aryl" further includes aromatic groups which linked via a single bond to further aromatic groups so as to form, e.g., biphenyl groups.

The term "heteroaryl," as used within the meaning of the present invention includes optionally suitably substituted 5- and 6-membered single-ring aromatic groups as well as substituted or unsubstituted multicyclic aryl groups, for example bicyclic or tricyclic aryl groups, comprising one or more, preferably from 1 to 4 such as 1, 2, 3 or 4, heteroatoms, wherein in case the aryl residue comprises more than 1 heteroatom, the heteroatoms may be the same or different. Such heteroaryl groups including from 1 to 4 heteroatoms are, for example, benzodioxolyl, pyrrolyl, furanyl, thiophenyl, thiazolyl, isothiazolyl, imidazolyl, triazolyl, tetrazolyl, pyrazolyl, oxazolyl, isoxazolyl, pyridinyl, pyrazinyl, pyridazinyl, benzoxazolyl, benzodioxazolyl, benzothiazolyl, benzoimidazolyl, benzothiophenyl, methylenedioxyphenylyl, napthyridinyl, quinolinyl, isoquinolinyl, benztriazyl, isoindolyl, benzofuranyl, purinyl, deazapurinyl, pteridinyl, or indolizinyl.

The term "substituted aryl" and the term "substituted heteroaryl" as used in the context of the present invention describes moieties having substituents replacing a hydrogen on one or more atoms, e.g. C or N, of an aryl or heteroaryl moiety. Again, there are in general no limitations as to the substituent. The substituents may be, for example, selected from the group consisting of alkyl, alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxy, phosphate, phosphonato, phosphinato, amino, acylamino, including alkylcarbonylamino, arylcarbonylamino, carbamoyl and ureido, amidino, nitro, imino, sulfhydryl, alkylthio, arylthio, thiocarboxylate, sulfates, alkylsulfinyl, sulfonate, sulfamoyl, sulfonamido, trifluoromethyl, cyano, azido, cycloalkyl such as e.g. cyclopentyl or cyclohexyl, heterocycloalkyl such as e.g. morpholino, piperazinyl or piperidinyl, alkylaryl, arylalkyl and heteroaryl.

The term "alkenyl" as used in the context of the present invention refers to unsaturated alkyl groups having at least one double bond. The term also encompasses alkenyl groups which are substituted by one or more suitable substituent.

The term "cycloalkenyl" as used in the context of the present invention refers to unsaturated cycloalkyl groups having at least one double bond. The term also encompasses alkenyl groups which are substituted by one or more suitable substituent. By way of example, the following groups are mentioned: cyclopentenyl, cyclohexenyl, cycloheptenyl, cyclooctenyl, cyclopentadienyl, cyclohexadienyl, cycloheptadienyl, cyclooctadienyl, and the like. Preferred are those with 5-7 carbon atoms, including cyclopentenyl, cyclohexenyl, and cycloheptenyl.

The term "alkynyl" refers to unsaturated alkyl groups having at least one triple bond. The term also encompasses alkynyl groups which are substituted by one or more suitable substituent.

The term "alkoxy", as used herein, refers to an alkyl residue being linked via an oxygen group to the respective parent molecule, thus to a residue having the structure -O-alkyl, wherein this term is meant to also encompass groups in which the alkyl group bears further substituents, as mentioned above.

### The group E

As mentioned above, the group E present in the core structure is CR⁷ or S, and f is 0 or 1, and wherein in case E is S, f is 0. Thus, the following preferred core structures are mentioned:

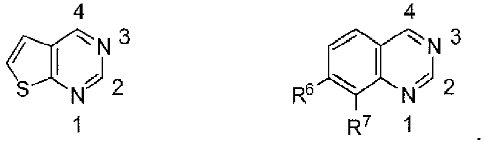

Most preferably E is CR⁷ and f is 1, thus, the compound of the invention preferably comprises the core structure

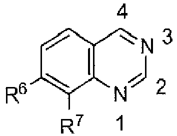

As mentioned above, the core structure comprised in the compounds according to the invention is at least substituted in 2 and in 4 position.

According to a preferred embodiment of the invention, the core structure is substituted with Z in 2 position, Z being selected from the group consisting of aryl, heteroaryl, -X^{Z}-aryl and -X^{Z}-heteroaryl, wherein X^{Z} is selected from the group consisting of O, NH, N(Me) and S, and wherein the core structure is further substituted with a group 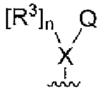 in 4 position, wherein X is selected from the group consisting of N, O or S, and wherein R³ is selected from the group consisting of H, alkyl, cycloalkyl, aryl, heteroaryl and heterocycloalkyl, wherein integer n is 0 or 1, and wherein Q is selected from the group consisting of alkyl, alkoxy, cycloalkyl, heterocycloalkyl, aryl, alkoxyalkyl and heteroaryl.

Thus, the compound according to the invention preferably comprises a structure according to the following formula (I), 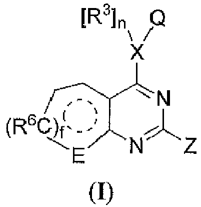 such as a structure according to one of the following formulas:

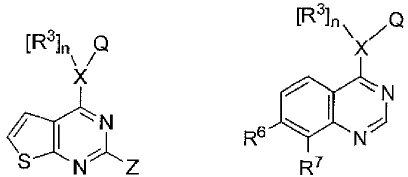

More preferably, the compound according to the invention, described above, has a structure according to following formula, including salts and solvates thereof, 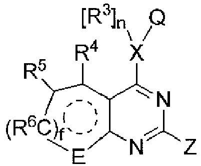 more preferably according to one of the following formulas, 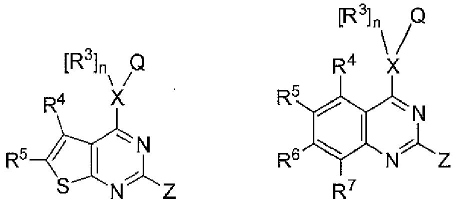 and most preferably according to the following formula (II): 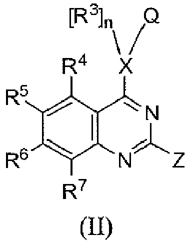 wherein R⁴, R⁵, R⁶, and R⁷ are, independently of each other, selected from the group consisting of -H, -D, -alkyl, alkoxy, alkenyl, alkynyl, halides, -NO₂, OH, NH₂, NHR^{4#} CN, S(O)R^{4#}, SO₂R^{4#}, P(O)R^{4#}R^{5#}, P(O)(OR^{4#})R^{5#}, P(O)(OR^{4#})(OR^{5#}), C(O)NR^{4#}R^{5#} C(O)SR^{4#}, -C(O)R^{4#}, -C(O)O-R^{4#}, alkoxy and glycol chains; and wherein R⁴ and R⁵ or R⁵ and R⁶ can form a cyclic residue, and wherein R^{4#} and R^{5#} are, independently of each other, selected from the group consisting of -H, -alkyl, -alkenyl, -heterocycloalkyl, aryl and heteroaryl.

### Residues R⁴, R⁵, R⁶, and R⁷

As mentioned above, R⁴, R⁵, R⁶, and R⁷, are, independently of each other, selected from the group consisting of H, -D, -alkyl, alkoxy, alkenyl, alkynyl, halides, -NO₂, -OH, -NH₂, -NHR^{4#} -CN, -S(O)R^{4#}, -SO₂R^{4#}, -P(O)R^{4#}R^{5#}, -P(O)(OR^{4#})R^{5#}, -P(O)(OR^{4#})(OR^{5#}), - C(O)NR^{4#}R^{5#}, -C(O)SR^{4#}, -C(O)R^{4#}, -C(O)O-R^{4#}, alkoxy and glycol chains, wherein R⁴ and R⁵ or R⁵ and R⁶ can form a cyclic residue.

It is to be understood that in case the compound comprises more than one residue denote herein as R^{4#}, each R^{4#} may be the same or may be different from each other, provided that R^{4#} is selected from the group consisting of -H, -alkyl, -alkenyl, -heterocycloalkyl, aryl and heteroaryl. In the same way, in case the compound comprises more than one residue denote herein as R^{5#}, each R^{5#} may be the same or may be different from each other, provided that R^{5#} is selected from the group consisting of -H, -alkyl, -alkenyl, - heterocycloalkyl, aryl and heteroaryl.

According to a preferred embodiment of the invention, R⁴, R⁵, R⁶, and R⁷, are, independently of each other, selected from the group consisting of -H, -OH, alkyl, alkoxy, in particular methoxy, -NO₂, -C(O)O-R^{4#}, glycol chains, wherein in case, any of R⁴, R⁵, R⁶, and R⁷ is an alkyl group, the alkyl group is preferably a, optionally substituted, C1-20alkyl group, more preferably a, optionally substituted, C1-10alkyl group, more preferably a, optionally substituted, methyl, ethyl, propyl, or butyl group, and most preferably a methyl group comprising at least one fluorine substituent, such as a, -CF₃, CHF₂, CH₂F. R^{4#} is preferably alkyl, more preferably C1-20alkyl, more preferably C1-10 alkyl, more preferably methyl, ethyl, propyl, or butyl. In case, any of R⁴, R⁵, R⁶, and R⁷ is an alkoxy group, the alkoxy group is preferably a, optionally substituted, C1-20alkoxy group, more preferably a, optionally substituted, C1-10alkoxy group, more preferably a ethoxy or methoxy group.

The term "glycol chain" preferably refers to an "ethylene glycol chain" or "oligoethylene glycol chain", i.e. to a group having a repeating ethylene glycol unit and optionally being capped with a methoxy group. However, other glycol groups known to the skilled person are also encompassed. The ethylene glycol chain can be any length, however typically it includes from 1 to about 100 and more typically from 1 to about 10 ethylene glycol units. Preferably, the glycol chain according to the invention has the following structure: -O-[CH₂-CH₂-O]_{g}-Me with integer g being preferably of from 1 to 100, more preferably of from 1 to 10. According to a particular preferred embodiment, g is 1.

As to residue R⁴, according to a preferred embodiment of the invention, this residue is H.

As to residues R⁵ and R⁶, according to a preferred embodiment of the invention, these residues are preferably, independently of each other, selected from the group consisting of -H, -CF₃, CHF₂, CH₂F, H, -OH, C1-10alkyl, C1-10alkoxy, in particular methoxy or ethoxy, -NO₂, -C(O)O-R^{4#}, with R^{4#} being C1-10alkyl, and glycol chains, in particular -O-[CH₂-CH₂-O]_{g}-Me with integer g being preferably of from 1 to 10.

As to residue R⁷, according to a preferred embodiment of the invention, this residue H or alkoxy, such as selected from the group consisting of H, methoxy and ethoxy, more preferably H or methoxy, most preferably H.

**Table 1: Preferred combinations of groups R⁴, R⁵, R⁶, and R⁷, by way of example:**

| Structure | R⁴ | R⁵ | R⁶ | R⁷ |
|---|---|---|---|---|
| 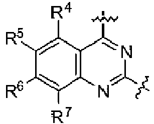 | H | -H, -CF₃, CHF₂, CH₂F, H, -OH, C1-10alkyl, methoxy, ethoxy, NO₂, -C(O)O-R^{4#}, with R^{4#} being C1-10alkyl, and -O-[CH₂-CH₂-O]_{g}-Me | -CF₃ | H |
| | H | methoxy | -H, -CF₃, CHF₂, CH₂F, H, -OH, C1-10alkyl, methoxy, ethoxy, NO₂, -C(O)O-R^{4#}, with R^{4#} being C1-10alkyl, or -O-[CH₂-CH₂-O]_{g}-Me | H |
| | H | methoxy | -H, -CF₃, CHF₂, CH₂F, H, -OH, C1-10alkyl, methoxy, ethoxy, NO₂, -C(O)O-R^{4#}, with R^{4#} being C1-10alkyl, or -O-[CH₂-CH₂-O]_{g}-Me | methoxy |
| | H | -H, -CF₃, CHF₂, CH₂F, H, -OH, C1-10alkyl, methoxy, ethoxy, NO₂, -C(O)O-R^{4#}, with R^{4#} being C1-10alkyl, or -O-[CH₂-CH₂-O]_{g}-Me | methoxy | methoxy |

### The residue Z:

Preferably, the substituent in 2 position is Z, wherein Z is preferably selected from the group consisting of aryl, heteroraryl, -X^{Z}-aryl and -X^{Z}-heteroaryl, as described above. According to a preferred embodiment Z has a structure according the following formulas, 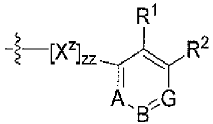 wherein X^{Z} is selected from the group consisting of O, NH, N(Me) and S, and wherein integer zz is 0 or 1, and wherein R¹ and R², are, suitable substituents, in particular, R¹ and R², are independently of each other, selected from the group consisting of H, D, -alkyl, cycloalkyl, cycloalkenyl, heterocycloalkyl, heterocycloalkenyl, aryl, heteroaryl, alkenyl, alkoxy, halides, -C(O)R^{1#}, -C(O)OR^{1#}, -S(O)R^{1#}, -SO₂R^{1#}, -P(O)R^{1#}R^{2#}, -P(O)(OR^{1#})R^{2#}, - P(O)(OR^{1#})(OR^{2#}), -C(O)NR^{1#}R^{2#}, -C(O)NR^{1#} and -C(O)SR^{1#}, wherein R^{1#} and R^{2#} are, independently of each other, selected from the group consisting of -H, -alkyl, -alkenyl, - heterocycloalkyl, aryl and heteroaryl, and wherein, A is N or C-R⁸, and wherein B is N or C-R⁹, and wherein G is N or C-R¹⁰, R⁸, R⁹ and R¹⁰, are, independently of each other, selected from the group consisting of H, -D, -alkyl, cycloalkyl, cycloalkenyl, heterocycloalkyl, heterocycloalkenyl, aryl, heteroaryl, alkenyl, alkoxy, halides, -C(O)R^{1#},-C(O)OR^{8#}, -S(O)R^{8#}, -SO₂R^{8#}, -P(O)R^{8#}R^{9#}, -P(O)(OR^{8#})R^{9#}, -P(O)(OR^{8#})(OR^{9#}), - C(O)NR^{8#}R^{9#}, -C(O)NR^{8#} and -C(O)SR^{8#}, and wherein R^{8#} and R^{9#} are, independently of each other, selected from the group consisting of -H, -alkyl, -alkenyl, -heterocycloalkyl, aryl and heteroaryl.

More preferably integer zz is 0, thus preferably Z has a structure according to the following formula:

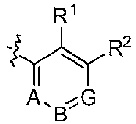

Thus, the present invention also describes a compound for inhibiting HIV capsid assembly, the conjugate having a structure according to the following formula 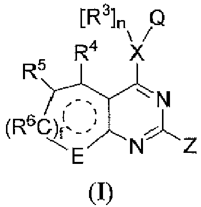 or a pharmaceutically acceptable salt or solvate thereof for use in inhibiting HIV capsid assembly, wherein Z is 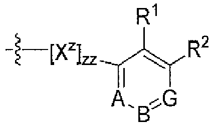 wherein X^{Z} is selected from the group consisting of O, NH, N(Me) and S, and wherein integer zz is 0 or 1, more preferably wherein Z is 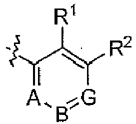 and wherein R¹ and R², are, independently of each other, selected from the group consisting of H, D, -alkyl, cycloalkyl, cycloalkenyl, heterocycloalkyl, aryl, heteroaryl, alkenyl, alkoxy, halides, -C(O)R^{1#}, -C(O)OR^{1#}, -S(O)R^{1#}, -SO₂R^{1#}, -P(O)R^{1#}R^{2#}, - P(O)(OR^{1#})R^{2#}, -P(O)(OR^{#})(OR^{2#}), -C(O)NR^{1#}R^{2#}, -C(O)NR^{1#} and -C(O)SR^{1#}, wherein R^{1#} and R^{2#} are, independently of each other, selected from the group consisting of -H, - alkyl, -alkenyl, -heterocycloalkyl, aryl and heteroaryl, wherein E is CR⁷ or S, and wherein f is 0 or 1, and wherein in case E is S, f is 0, and wherein, A is N or C-R⁸, and wherein B is N or C-R⁹, and wherein G is N or C-R¹⁰, and wherein R⁸, R⁹ and R¹⁰, are, independently of each other, selected from the group consisting of H, -D, -alkyl, cycloalkyl, cycloalkenyl, heterocycloalkyl, heterocycloalkenyl, aryl, heteroaryl, alkenyl, alkoxy, halides, -C(O)R^{1#}, -C(O)OR^{8#}, -S(O)R^{8#}, -SO₂R^{8#}, -P(O)R^{8#}R^{9#}, -P(O)(OR^{8#})R^{9#}, - P(O)(OR^{8#})(OR^{9#}), -C(O)NR^{8#}R^{9#}, -C(O)NR^{8#} and -C(O)SR^{8#}, and wherein R^{8#} and R^{9#} are, independently of each other, selected from the group consisting of -H, -alkyl, - alkenyl, -heterocycloalkyl, aryl and heteroaryl, preferably selected from the group consisting of -H, -alkyl, -O-alkyl, -halides, wherein X is selected from the group consisting of N, O or S, wherein R³ is selected from the group consisting of H, alkyl, cycloalkyl, aryl, heteroaryl and heterocycloalkyl, wherein integer n is 0 or 1, and wherein R⁴, R⁵, R⁶, and R⁷ are, independently of each other, selected from the group consisting of - H, -D, -alkyl, alkoxy, alkenyl, alkynyl, halides, -NO₂, -OH, -NH₂, -NHR^{4#}, -CN, - S(O)R^{4#}, -SO₂R^{4#}, -P(O)R^{4#}R^{5#}, -P(O)(OR^{4#})R^{5#}, -P(O)(OR^{4#})(OR^{5#}), -C(O)NR^{4#}R^{5#}, - C(O)SR^{4#}, -C(O)R^{4#}, -C(O)O-R^{4#}, alkoxy and glycol chains; and wherein R⁴ and R⁵ or R⁵ and R⁶ can form a cyclic residue, and wherein R^{4#} and R^{5#} are, independently of each other, selected from the group consisting of -H, -alkyl, -alkenyl, -heterocycloalkyl, aryl and heteroaryl, and wherein Q is selected from the group consisting of alkyl, alkoxy, cycloalkyl, heterocycloalkyl, aryl, alkoxyalkyl and heteroaryl, wherein E is CR⁷ or S, and wherein f is 0 or 1, and wherein in case E is S, f is 0, with the proviso that in case X is S, n is 0, and in case X is N, n is 1. Further, the present invention relates to the use of a compound, as described above, for the manufacture of a medicament for inhibiting HIV capsid assembly. Further, the present invention relates to a method of treating a patient suffering from HIV comprising administering a therapeutically effective amount of the compound, as defined above, as well as the use of this compound for treating a patient suffering from HIV.

In case, X^{Z} is present, X^{Z} is preferably NH. Thus, according to this embodiment, Z has preferably the structure

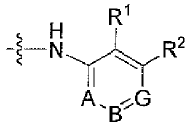

### Residues R¹ and R²

As mentioned above, R¹ and R², are, independently of each other, selected from the group consisting of H, D, -alkyl, cycloalkyl, cycloalkenyl, heterocycloalkyl, aryl, heteroaryl, alkenyl, alkoxy, halides, -C(O)R^{1#}, -C(O)OR^{1#}, -S(O)R^{1#}, -SO₂R^{1#}, -P(O)R^{1#}R^{2#}, - P(O)(OR^{1#})R^{2#}, -P(O)(OR^{#})(OR^{2#}), -C(O)NR^{1#}R^{2#}, -C(O)NR^{1#} and -C(O)SR^{1#}, wherein R^{1#} and R^{2#} are, independently of each other, selected from the group consisting of -H, - alkyl, -alkenyl, -heterocycloalkyl, aryl, heteroaryl. Preferably R¹ and R², are, independently of each other, selected from the group consisting alkyl cycloalkyl, heterocycloalkyl, cycloalkenyl, alkenyl, aryl, heteroaryl, H, halides, -D, C(O)R^{1#}, C(O)OR^{1#} and alkoxy, wherein R^{1#} is C₁₋₁₀alkyl, more preferably from the group consisting of C1-20alkyl, in particular C1-10alkyl, C3-10cycloalkyl, C3-10heterocycloalkyl, C3-10cycloalkenyl, C2-20alkenyl, C6-10aryl, C5-10heteroaryl, C(O)R^{1#}, C(O)OR^{1#}, -H, -D, -C1-10alkoxy, and -F, wherein R^{1#} is C₁₋₁₀alkyl, more preferably methyl, ethyl or butyl.

In case any one of R¹ or R² is C1-10alkyl , the following C₁₋₁₀alkyl groups are preferred: methyl, ethyl, propyl, butyl, pentyl, hexyl or octyl.

In case any one of R¹ or R² is C3-10cycloalkyl, the following preferred C3-10cycloalkyl groups are mentioned, by way of example: cyclopentyl, cyclohexyl, cycloheptyl cyclopropylmethyl, cyclopentylmethyl, cyclohexylmethyl, cyclohexylcyclopentylmethyl, dicyclohexylmethyl, 1-cyclopentylethyl, 1-cyclohexylethyl, 2-cyclopropylethyl, 2-cyclopentylethyl, 2-cyclohexylethyl, 2-cycloheptylethyl, 1-cyclohexyl-1-methylethyl, 1-cyclohexylpropyl, 2-cyclopentylpropyl, 3-cyclobutylpropyl, 3-cyclopentylpropyl, 3-cyclohexylpropyl, 3-cycloheptylpropyl, 1-cyclopropyl-lmethylpropyl, 1-cyclohexyl-2-methylpropyl, 1-cyclopentyl butyl, 1-cyclohexylbutyl, 3-cyclohexylbutyl, 4-cyclopropylputyl, 4-cyclobutylbutyl, 4-cyclopentylbutyl, 1-cyclohexyl 1-methylbutyl, 1-cyclopentyl-2-ethylbutyl, 1-cyclohexyl-3methylbutyl, 1-cyclopentylpentyl, 1-cyclohexylpentyl, 1-cyclohexylmethylpentyl, 2-cyclohexylpentyl, 2-cyclohexylmethylpentyl, 3-cyclopentylpentyl, 1-cyclohexyl-4methylpentyl, 5-cyclopentylpentyl, 1-cyclopentylhexyl, 1-cyclohexylhexyl, 1-cyclopentyhnethylhexyl, 2-cyclopentylhexyl, 2-cyclopropylethylhexyl, 3-cyclopentylbexyl, 1-cyclohexylheptyl, 1-cyclopentyl-1-methylheptyl, 1-cydohexyl-1,6-dimethylheptyl, 1-cycloheptyloctyl, 2-cyclopentyloctyl, 3-cyclohexyloctyl, 2-cyclopentylmethyloctyl, 1-cyclopentylnonyl, 1-cyclohexylnonyl, 3-cyclopropylnonyl, 1-cyclopentyldecyl, 1-cyclohexylundecyl, 1-cyclopentyl tridecyl, 2-cyclohexyltridecyl, and the like

In case any one of R¹ or R² is C3-10heterocycloalkyl the following preferred C3-10heterocycloalkyl groups are mentioned by way of example: morpholino, piperazinyl, piperidinyl tetrahydrofuran, pyrrolidin, pyrrolidin-2-on.

In case any one of R¹ or R² is C3-10cycloalkenyl, the following preferred C3-10cycloalkenyl groups are mentioned by way of example: cyclopentenyl, cyclohexenyl, and cycloheptenyl.

Further, the following preferred C2-20alkenyl groups are mentioned: ethenyl, propenyl, butenyl, pentenyl.

Further, the following preferred C6-10aryl groups are mentioned: phenyl (including substituted phenyl groups), benzyl (including substituted benzyl groups), naphthyl, anthryl, phenanthryl, biphenyl, and the like.

Further, the following preferred C5-10heteroaryl are mentioned: benzodioxolyl, pyrrolyl, furanyl, thiophenyl, thiazolyl, isothiazolyl, imidazolyl, triazolyl, tetrazolyl, pyrazolyl, oxazolyl, isoxazolyl, pyridinyl, pyrazinyl, pyridazinyl, benzoxazolyl, benzodioxazolyl, benzothiazolyl, benzoimidazolyl, benzothiophenyl, methylenedioxyphenylyl, napthyridinyl, quinolinyl, isoquinolinyl, indolyl, benzofuranyl, purinyl, deazapurinyl, or indolizinyl.

Most preferably, R¹ or are selected from the group consisting of C1-20alkyl, C3-10cycloalkyl, C3-10heterocycloalkyl, C3-10cycloalkenyl, C2-20alkenyl, C6-10aryl, C5-10heteroaryl, C(O)R^{1#}, C(O)OR^{1#}, -H, -D, -C1-10alkoxy, and -F, wherein R^{1#} is C₁₋₁₀alkyl.

According to a particularly preferred embodiment, R¹ and R², are both -H.

### A, B and G

As regards, A, B and G, preferably at least one of these atoms is N. Thus, Z preferably has one of the following structures: 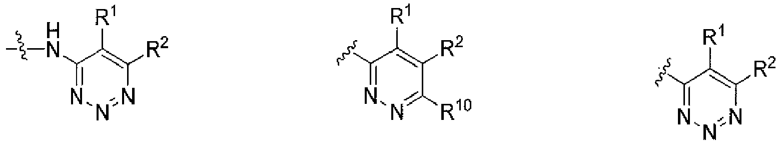 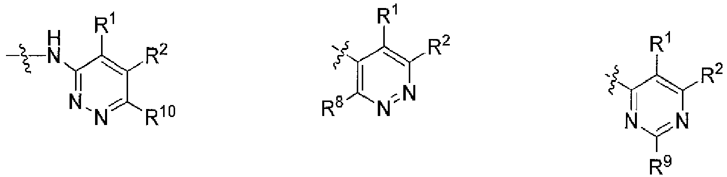 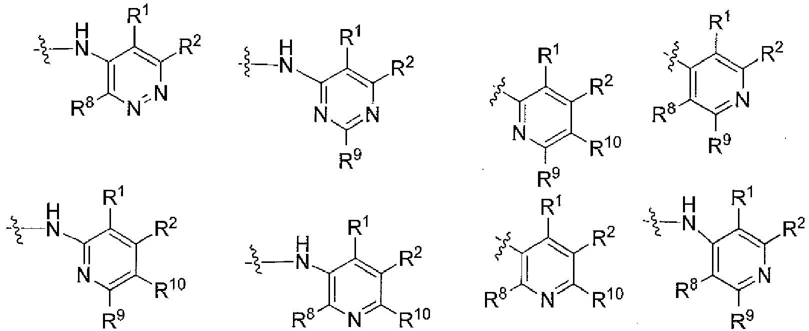 more preferably one of the following structures;

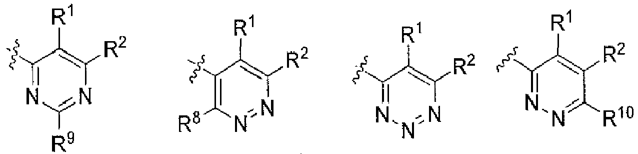

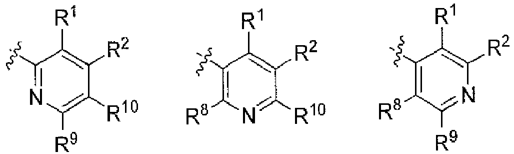

According to a preferred embodiment of the invention, only one of A, B and G is N, and the respective other groups are C-R⁸, C-R⁹, or C-R¹⁰, respectively. Thus, Z is preferably

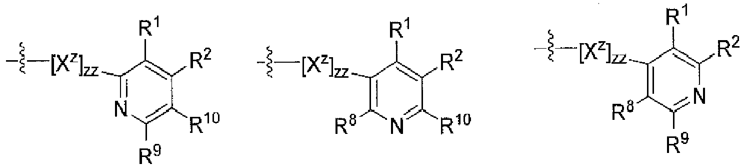

### Residues R⁹, R⁸ and R¹⁰

As regards, R⁸, R⁹ and R¹⁰, these residues are, preferably, independently of each other, selected from the group consisting of H, -D, -alkyl, cycloalkyl, cycloalkenyl, heterocycloalkyl, heterocycloalkenyl, aryl, heteroaryl, alkenyl, alkoxy, halides, -C(O)R^{1#}, C(O)OR^{8#}, -S(O)R^{8#}, -SO₂R^{8#}, -P(O)R^{8#}R^{9#}, -P(O)(OR^{8#})R^{9#}, -P(O)(OR^{8#})(OR^{9#}), - C(O)NR^{8#}R^{9#}, -C(O)NR^{8#} and -C(O)SR^{8#}, and wherein R^{8#} and R^{9#} are, independently of each other, selected from the group consisting of -H, -alkyl, -alkenyl, -heterocycloalkyl, aryl and heteroaryl.

More preferably, R⁸, R⁹ and R¹⁰, are, independently of each other -H, -alkyl, -O-alkyl and -halide. According to a preferred embodiment of the invention, R⁸, R⁹ and R¹⁰, are, independently of each other, selected from the group consisting of -H, C1-10alkyl, -O-[C1-10alkyl], -I, -Br, -Cl and -F, more preferably selected from the group consisting of -H, methyl, ethyl, propyl, trifluoromethyl, methoxy, ethoxy, -I and -F.]

Thus, the following preferred groups Z are mentioned by way of example:

**Table 2: Preferred groups Z, by way of example:**

| Structure Z | [X^{Z}]_{zz} | R¹ | R² | R⁸ | R⁹ | R¹⁰ |
|---|---|---|---|---|---|---|
| 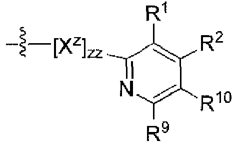 | zz=0 or zz = 1 and X^{Z} = NH | H | H | - | H | -H, C1-10alkyl, -O-[C1-10alkyl], -I, -Br, -Cl or -F |
| | zz=0 or zz = 1 and X^{Z} = NH | H | H | - | -H, C1-10alkyl, -O-[C1-10alkyl], -I, -Br, -Cl or -F | H |
| | zz = 0 or zz = 1 and X^{Z}=NH | H | -H, C1-10alkyl, -O-[C1-10alkyl], -I, -Br, -Cl or -F | - | H | H |
| | zz = 0 or zz = 1 and X^{Z} = NH | -H, C1-10alkyl, -O-[C1-10alkyl], -I, -Br, -Cl or -F | H | - | H | H |
| 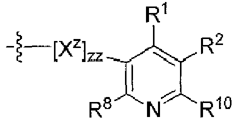 | zz = 0 or zz = 1 and X^{Z} = NH | H | -H, C1-10alkyl, -O-[C1-10alkyl], -I, -Br, -Cl or -F | H | - | H |
| | zz=0 or zz= 1 and X^{Z} = NH | H | H | -H, C1-10alkyl, -O-[C1-10alkyl], -I, -Br, -Cl or -F | - | H |
| | zz=0 or zz = 1 and | -H, Cl-10alkyl, -O-[C1-10alkyl],-I, -Br, -Cl | H | H | - | H |
| | X^{Z} = NH | or -F | | | | |
| | zz=0 or zz = 1 and X^{Z} = NH | H | H | H | - | -H, C1-10alkyl, -O-[C1-10alkyl], -I, -Br, -Cl or -F |
| 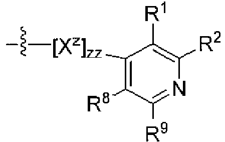 | zz=0 or zz = 1 and X^{Z} = NH | -H, C1-10alkyl, -O-[C1-10alkyl], -I, -Br, -Cl or -F | H | H | H | - |
| | zz = 0 or zz= 1 and X^{Z} = NH | H | -H, C1-10alkyl, -O-[C1-10alkyl, -I, Br, -Cl or -F | H | H | - |
| | zz = 0 or zz = 1 and X²=NH | H | H | -H, C1-10alkyl, -O-[C1-10alkyl],-I, -Br, -Cl or -F | | - |
| | zz = 0 or zz = 1 and X^{Z}=NH | H | H | H | -H, C1-10alkyl,-O-[C1-10alkyl], -I, -Br, -Cl or-F | - |

According to a further preferred embodiment of the present invention, Z has as structure according to the following formula, 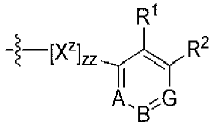 wherein zz is 0 or 1, and wherein A is C-R⁸, B is C-R⁹, and C-R¹⁰, Z thus having the following structure:

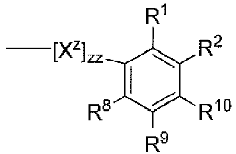

In this case, integer zz is preferably 0. Thus, the present invention also describes a compound for inhibiting HIV capsid assembly, as described above, the conjugate having a structure according to the following formula, 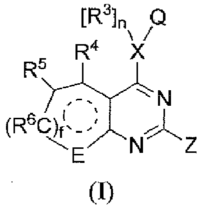 or a pharmaceutically acceptable salt or solvate thereof for use in inhibiting HIV capsid assembly, wherein Z is

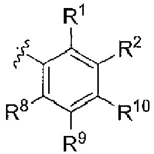

Thus, the following further preferred groups Z are mentioned by way of example.

**Table 3: Further preferred groups Z, by way of example**

| Structure Z | [X^{Z}]_{zz} | R¹ | R² | R⁸ | R⁹ | R¹⁰ |
|---|---|---|---|---|---|---|
| 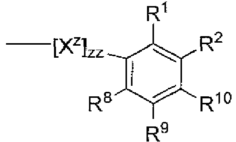 | zz = 0 | H | H | H | H | -H, C1-10alkyl, -O-[C1-10alkyl], -I, -Br, -Cl or -F |
| | zz = 0 | H | F | H | F | -H, C1-10alkyl, -O-[C1-10alkyl], -I, -Br, -Cl or -F |
| | zz = 1 and X^{Z}=NH | H | F | H | F | -H, C1-10alkyl, -O-[C1-10alkyl, -I, -Br, -Cl or -F |

Most preferably, Z is selected from the group consisting of

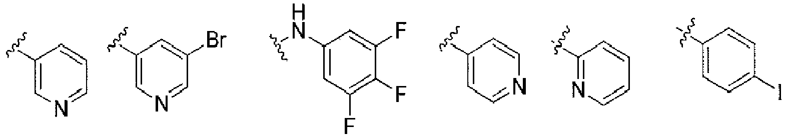

### The structural unit -X[Q][R³]ₙ

### The group X

As mentioned above, the group X present in the structural moiety 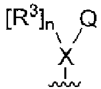 is selected from the group consisting of N, O or S, with the proviso that in case X is S, n is 0, and in case X is N, n is 1. Thus, the present invention also describes a compound for inhibiting HIV capsid assembly, the conjugate having a structure according to the one of the following formula 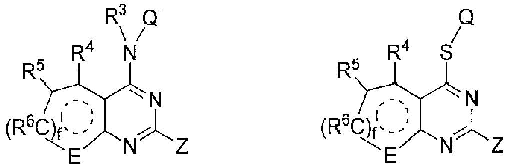 or a pharmaceutically acceptable salt or solvate thereof for use in inhibiting HIV capsid assembly, wherein Z is 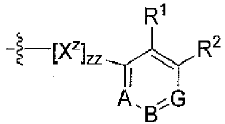 wherein X^{Z} is selected from the group consisting of O, NH, N(Me) and S, and wherein integer zz is 0 or 1, more preferably wherein Z is 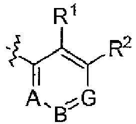 and wherein R¹ and R², are, independently of each other, selected from the group consisting of H, D, -alkyl, cycloalkyl, cycloalkenyl, heterocycloalkyl, aryl, heteroaryl, alkenyl, alkoxy, halides, -C(O)R^{1#}, -C(O)OR^{1#}, -S(O)R^{1#}, -SO₂R^{1#}, -P(O)R^{1#}R^{2#}, - P(O)(OR^{1#})R^{2#}, -P(O)(OR^{#})(OR^{2#}), -C(O)NR^{1#}R^{2#}, -C(O)NR^{1#} and -C(O)SR^{1#}, wherein R^{1#} and R^{2#} are, independently of each other, selected from the group consisting of -H, - alkyl, -alkenyl, -heterocycloalkyl, aryl and heteroaryl, wherein E is CR⁷or S, and wherein f is 0 or 1, and wherein in case E is S, f is 0, and wherein, A is N or C-R⁸, and wherein B is N or C-R⁹, and wherein G is N or C-R¹⁰, and wherein R⁸, R⁹ and R^{10,} are, independently of each other, selected from the group consisting of -H, -alkyl, -O-alkyl, -halides, wherein R³ is selected from the group consisting of H, alkyl, cycloalkyl, aryl, heteroaryl and heterocycloalkyl, and wherein R⁴, R⁵, R⁶, and R⁷ are, independently of each other, selected from the group consisting of -H, -D, -alkyl, alkoxy, alkenyl, alkynyl, halides, -NO₂, -OH, -NH₂, -NHR^{4#}, -CN, -S(O)R^{4#}, -SO₂R^{4#}, -P(O)R^{4#}R^{5#}, -P(O)(OR^{4#})R^{5#}, - P(O)(OR^{4#})(OR^{5#}), -C(O)NR^{4#}R^{5#}, -C(O)SR^{4#}, -C(O)R^{4#}, -C(O)O-R^{4#}, alkoxy and glycol chains; and wherein R⁴ and R⁵ or R⁵ and R⁶ can form a cyclic residue, and wherein R^{4#} and R^{5#} are, independently of each other, selected from the group consisting of -H, -alkyl, -alkenyl, -heterocycloalkyl, aryl and heteroaryl, and wherein Q is selected from the group consisting of alkyl, alkoxy, cycloalkyl, heterocycloalkyl, aryl, glycol chains and heteroaryl, wherein E is CR⁷ or S, and wherein f is 0 or 1, and wherein in case E is S, f is 0.

According to a preferred embodiment of the present invention, X is N and n is 1, the compound thus having the

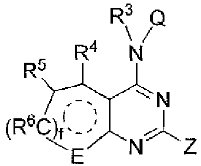

### Residue R³:

R³ is selected from the group consisting of H, alkyl, cycloalkyl, aryl, heteroaryl and heterocycloalkyl. Preferably R³ is selected from the group consisting of H, C1-10alkyl, C3-10cycloalkyl, C3-10heterocycloalkyl, C6-10aryl and C5-10heteroaryl, more preferably H or C1-10alkyl. It is to be understood that all mentioned residues mentioned may be substituted or unsubstituted. In particular, R³ is selected from the group consisting of H, C1-10alkyl and C3-10cycloalky, such as H, methyl, ethyl, propyl, butyl, pentyl, cyclopentyl, cylohexyl and hexyl. This includes the respective substituted residues. More preferably R³ is H or a C1-10alkyl being substituted with at least on halogen, such as with 1, 2 or 3 halogens, preferably with 1 or 2 or 3 fluorides, such as a 2-fluoroethyl group, or a C3-10cycloalkyl group being substituted with at least on halogen, such as with 1, 2 or 3 halogens, preferably with 1 or 2 or 3 fluorides. [ More preferably, R³ is H or methyl.

Thus, the present invention also relate to a compound, as described above, having the structure 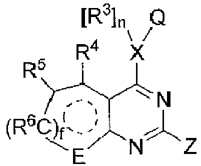 preferably a structure according to one of the following formulas, 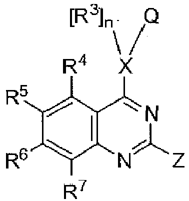 wherein X is N, n is 1 and R³ is H or alkyl or cycloalkyl, preferably H or methyl, more preferably H.

### Residue Q:

As regards residue Q, this residue is preferably selected from the group consisting of alkyl, alkoxy, cycloalkyl, heterocycloalkyl, aryl, alkoxyalkyl and heteroaryl.

Preferably Q is selected from the group consisting of C1-10alkyl, C1-10alkoxy, C3-10cycloalkyl, C3-10heterocycloalkyl, C6-10aryl, C1-4alkoxy-C1-10alkyl and C5-10heteroaryl. In case Q is an aryl group, Q is preferably phenyl, or a benzocyclopentadienyl group, or a benzocyclopentyl group.. In case Q is an alkyl group, Q is preferably selected from the group consisting of methyl, ethyl, propyl, -CF₃, -CHF₂, - CH₂F, -CH₂-CF₃, -CH₂-CHF₂, -CH₂-CH₂F, -CHF-CH₃, more methyl or -CF₃. In case Q is a heteroaryl group, Q is preferably selected from the group consisting of indolyl, benzimidazolyl, pyridinyl, benztriazyl pyrimidinyl and pyrazinyl. In case Q is an cycloalkyl group, Q is preferably selected cyclohexane or cyclopentane. In case Q is an cycloheteroalkyl group, Q is preferably selected from the group consisting of morpholino, piperazinyl, piperidinyl tetrahydrofuran, pyrrolidin and pyrrolidin-2-on It is to be understood that all above mentioned residues mentioned may be substituted or unsubstituted.

Thus according to a preferred embodiment, Q is selected from the group consisting phenyl, benzocyclopentadienyl group, benzocyclopentyl group, methyl, ethyl, propyl, - CF₃, -CHF₂, -CH₂F, -CH₂-CF₃, -CH₂-CHF₂, -CH₂-CH₂F, indolyl, benzimidazolyl, pyridinyl, benztriazyl, pyrimidinyl, pyrazinyl, cyclohexane, cyclopentane, morpholino, piperazinyl, piperidinyl, tetrahydrofuran, pyrrolidin, pyrrolidin-2-on, wherein all residues mentioned (except for CF₃, -CHF₂, -CH₂F, -CH₂-CF₃, -CH₂-CHF₂, -CH₂-CH₂F) may be substituted or unsubstituted.

According to a one preferred embodiment of the invention, Q is selected from the group consisting of a C1-10alkyl group, which is optionally substituted with at least one fluorine group.

According to a further preferred embodiment of the invention, Q is selected from residues having one of the following structures: 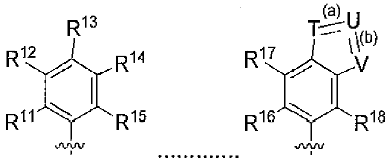 wherein R¹¹, R¹², R¹³, R¹⁴, and R¹⁵ are, independently of each other, selected from the group consisting of -H, -alkyl, -halides, alkoxy, alkynyl, alkenyl, -NO₂, -CN, -C(=Y)-R^{11#}, -C(=Y)-H, -C(=Y)-Y¹-R^{11#}, -C(=Y)-OH, , -Y-R^{11#}, -NH₂, -NHR^{11#}, -CN, -F, -CF₃, - S(O)R^{11#}, -SO₂R^{11#}, -P(O)R^{11#}R^{12#}, -P(O)(OR^{11#})R^{12#}, -P(O)(OR^{11#})(OR^{12#}), wherein Y is O, S or NH, and wherein Y¹ is O, S or NR*, wherein R* is H or alkyl, and wherein R^{11#} and R^{12#} are, independently of each other, selected from the group consisting of -H, -alkyl, -alkenyl, -heterocycloalkyl, aryl and heteroaryl, and wherein T is -C-R¹⁹, N or N-Y^{T}, with Y^{T} being H or alkyl, wherein U is -C-R²⁰, N or N-Y^{U}, with Y^{U} being H or alkyl, and wherein V is -C-R²¹, N or N-Y^{Y}, with Y^{V} being H or alkyl, the bond (a) is a single or double bond, the bond (b) is a single or double bond, with eth proviso that one of (a) and (b) is a double bond, R¹⁹, R²⁰, and R²¹ are, independently of each other H or -alkyl, and wherein at R¹⁶, R¹⁷ and R¹⁸, are independently of each other selected from the group consisting of -H, -alkyl, -halides, alkoxy, alkynyl, alkenyl, -NO₂, -C(=Y)-alkyl, -C(=Y)-H, -C(=Y)-Y¹-alkyl, -C(=Y)-OH and -Y-alkyl.

Thus, the present invention also describes a compound for inhibiting HIV capsid assembly, as described above, the conjugate having a structure according to the following formula 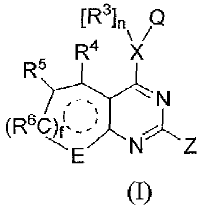 or a pharmaceutically acceptable salt or solvate thereof for use in inhibiting HIV capsid assembly, wherein Q is 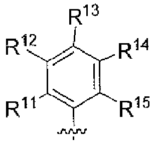 wherein R¹¹, R¹², R¹³, R¹⁴, and R¹⁵ are, independently of each other, selected from the group consisting of -H, -alkyl, -halides, alkoxy, alkynyl, alkenyl, -NO₂, -CN, -C(=Y)-R^{11#}, -C(=Y)-H, -C(=Y)-Y¹-R^{11#}, -C(=Y)-OH, , -Y-R^{11#}, -NH₂, -NHR^{11#}, -CN, -S(O)R^{11#}, -SO₂R^{11#}, -P(O)R^{11#}R^{12#}, -P(O)(OR^{11#})R^{12#}, -P(O)(OR^{11#})(OR^{12#}),
wherein Y is O, S or NH, and wherein Y¹ is O, S or NR*, wherein R* is H or alkyl, and wherein R^{11#} and R^{12#} are, independently of each other, selected from the group consisting of -H, -alkyl, -alkenyl, -heterocycloalkyl, aryl and heteroaryl.

Preferably, R¹¹, R¹², R¹³, R¹⁴, and R¹⁵ are, independently of each other, selected from the group consisting of -H, -C1-10alkyl, -F, -I, -Cl, -Br, C1-10alkoxy, C2-10alkynyl, C2-10alkenyl, --NO₂, -CN, -C(=O)-alkyl, -C(=O)-H, -C(=O)-O-alkyl, C(=O)-NH-alkyl (C(=O)-NH- alkyl substituted with 1, 2 or 3 halogens, in particular F, in particular -C(=O)-NH(-CH₂-CH₂F)), -C(=O)-N(alkyl)₂ (such as C(=O)-N(-CH₂-CH₂F)₂), -C(=O)-OH, -O-alkyl, -NH₂, -NH-alkyl, -CN, -S(O)alkyl, -SO₂alkyl (such as -SO₂-alkyl substituted with 1, 2 or 3 halogens, in particular F), -C(=O)-NH-cycloalkyl (such as -C(=O)-NH-cycloalkyl substituted with 1, 2 or 3 halogens, in particular F), -C(=O)-N(cycloalkyl)₂ (such as - C(=O)-N(cycloalkyl)₂ substituted with 1, 2 or 3 halogens, in particular F. It is to be understood that all above mentioned residues denoted as alkyl, alkoxy, alkynyl, alkenyl may be substituted or unsubstituted. In case any one of R¹¹, R¹², R¹³, R¹⁴, and R¹⁵ is an alkyl group, this alkyl group is preferably selected from the group consisting of methyl, ethyl, propyl, -CF₃, -CHF₂, -CH₂F, -CH₂-CF₃, -CH₂-CHF₂, -CH₂-CH₂F and -CHF-CH₃, more methyl or -CF₃.

E.g., R¹¹ R¹², R¹³, R¹⁴, and R¹⁵ are, independently of each other, selected from the group consisting of -F, -I, NO₂, -C(=O)-alkyl, -C(=O)-OH, -C(=O)-O-alkyl, -C(=Y)-OH and - NH-alkyl.

In case any one of R¹¹, R¹², R¹³, R¹⁴, and R¹⁵ is selected from the group consisting of - C(=O)-alkyl, -C(=O)-O-alkyl and C(=O)-NH-alkyl, the alkyl group present in this residues is preferably, a optionally substituted, methyl group, ethyl group, propyl group....

Most favorable is a substitution in which at least one of R¹¹, R¹², R¹³, R¹⁴, and R¹⁵ is an electron withdrawing group. The term "electron-withdrawing group" is recognized in the art, and denotes the tendency of a functional group to attract valence electrons from neighboring atoms by means of a difference in electronegativity with respect to the neighbouring atom (inductive effect) or by withdrawal of π-electrons via conjugation (mesomeric effect). Examples for suitable withdrawing groups are the above mentioned groups, -F, -I, -NO₂, -C(=O)-alkyl, -C(=O)-NH-alkyl, - C(=O)-N(alkyl)₂, -C(=O)-OH, -C(=O)-O-alkyl, -C(=Y)-OH, preferably the electron withdrawing group is selected from the group consisting of -F, -I, -NO₂, -C(=O)-methyl, -C(=O)-ethyl, -COOH, -CF₃, -CHF₂, -CH_{2F}, -CH₂-CF₃, -CH₂-CHF₂ and -CH₂-CH₂F.

Preferably Q is selected from the group consisting of

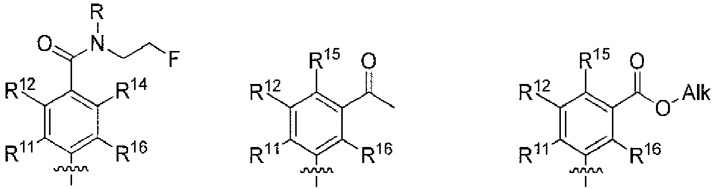

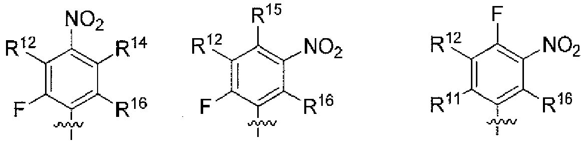

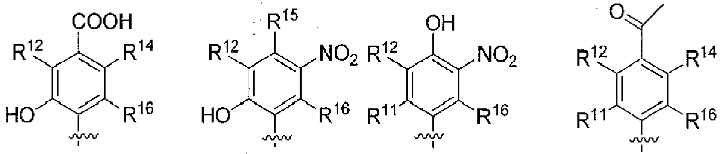

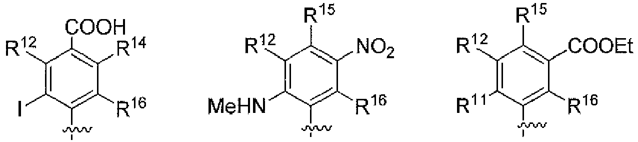

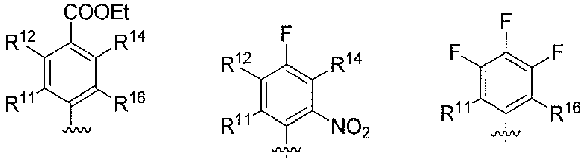

Wherein R in the above depicted formula is H or alkyl,

More preferably Q is selected from the group consisting of

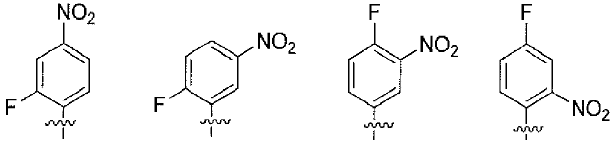

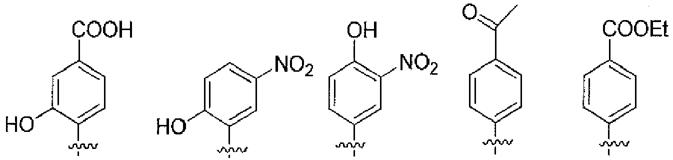

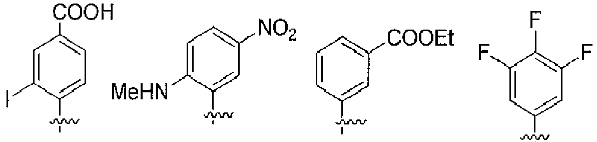

In particular at least one of one of R¹¹, R¹², R¹³, R¹⁴, and R¹⁵ is -F, more preferably at least R¹², R¹³ and R¹⁴ are -F, more preferably Q is

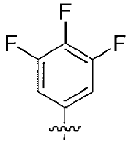

Thus, the present invention also describes a compound for inhibiting HIV capsid assembly, as described above, the conjugate having a structure according to the following formula 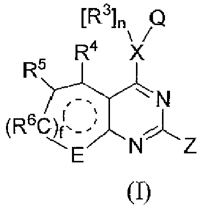 or a pharmaceutically acceptable salt or solvate thereof for use in inhibiting HIV capsid assembly, wherein Q is

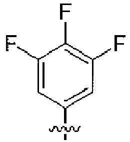

Furthermore , the present invention also describes a compound for inhibiting HIV capsid assembly, as described above, the conjugate having a structure according to the following formula 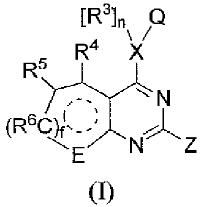 or a pharmaceutically acceptable salt or solvate thereof for use in inhibiting HIV capsid assembly, wherein Q is 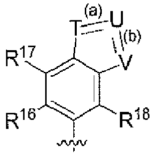 wherein T is -C-R¹⁹, N or N-Y^{T}, with Y^{T} being H or alkyl
wherein U is -C-R²⁰, N or N-Y^{U}, with Y^{U} being H or alkyl
wherein V is -C-R²¹, N or N-Y^{Y}, with Y^{V} being H or alkyl
the bond (a) is a single or double bond,
the bond (b) is a single or double bond,
with the proviso that one of (a) and (b) is a double bond,
and wherein R¹⁹, R²⁰, and R²¹ are, independently of each other selected from the group consisting of H and -alkyl and wherein R¹⁶, R¹⁷ and R¹⁸, are independently of each other selected from the group consisting of -H, -alkyl, -halides, alkoxy, alkynyl, alkenyl, -NO₂, - C(=Y)-alkyl, -C(=Y)-H, -C(=Y)-Y¹-alkyl, -C(=Y)-OH and -Y-alkyl.

Preferably, R¹⁶, R¹⁷ and R¹⁸, are independently of each other selected from the group consisting of -H, -C1-10alkyl, -F, -I, C1-10alkoxy, C2-10alkynyl, C2-10alkenyl, -NO₂, - C(=O)-C1-10alkyl, -C(=O)-H, -C(=O)-O-C1-10alkyl, -C(=O)-OH and -O-C1-10alkyl, more preferably R¹⁶, R¹⁷ and R¹⁸, are independently of each other selected from the group consisting of -H, -CH3, -CH2-CH3, CF₃, -CHF₂, -CH₂F, -CH₂-CF₃, -CH₂-CHF₂ and -CH₂-CH₂F, -F, -I, methoxy, ethoxy, -NO₂, -C(=O)-methyl, C(=O)- ethyl, -C(=O)-H, -C(=O)-O-methyl, -C(=O)-O-ethyl and -C(=O)-OH.

According to a preferred embodiment Q is selected from the group consisting o 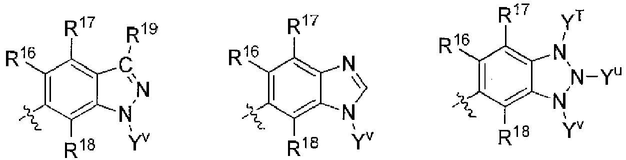 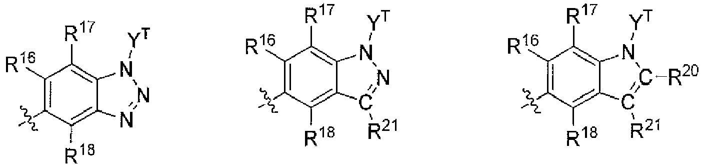 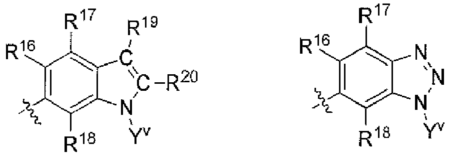 wherein R¹⁹, R²⁰, and R²¹ are, independently of each other selected from the group consisting of H and -alkyl and wherein R¹⁶, R¹⁷ and R¹⁸, are independently of each other selected from the group consisting of -H, -alkyl, -halides, alkoxy, alkynyl, alkenyl, -NO₂, - C(=Y)-alkyl, -C(=Y)-H, -C(=Y)-Y¹-alkyl, -C(=Y)-OH and -Y-alkyl, more preferably R¹⁶, R¹⁷ and R¹⁸, are independently of each other selected from the group consisting of -H, - CH3, -CH2-CH3, CF₃, -CHF₂, -CH₂F, -CH₂-CF₃, -CH₂-CHF₂ and -CH₂-CH₂F, -F, -I, methoxy, ethoxy, -NO₂, -C(=O)-methyl, C(=O)- ethyl, -C(=O)-H, -C(=O)-O-methyl, - C(=O)-O-ethyl and -C(=O)-OH.

As regards, Y^{T}, Y^{U} and Y^{V}, these groups are, if present, independently from each other H or alkyl, preferably H or C1-10 alkyl, more preferably selected from the group consisting of H, methyl and ethyl, most preferably H of methyl.

In particular, Q is selected from the following formulas 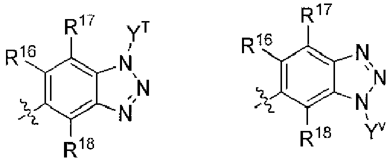 more preferably from one of the following formulas:

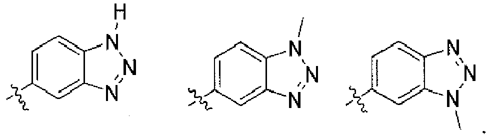

Thus, the present invention also describes a compound for inhibiting HIV capsid assembly, as described above, the conjugate having a structure according to the following formula 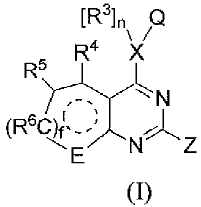 or a pharmaceutically acceptable salt or solvate thereof for use in inhibiting HIV capsid assembly, wherein Q is selected from the group consisting of

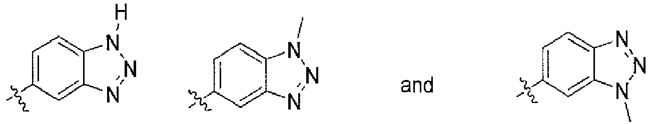

In a preferred embodiment of the present invention, the compound of the present invention is combined with at least one further antiretroviral agent. Preferred further antiretroviral agents are disclosed elsewhere herein. Thus, the present invention also envisages a composition comprising the compound of the present invention and at least one further antiretroviral agent.

### Pharmaceutically acceptable salt

As described above, the compounds of the present invention can be formulated as pharmaceutically acceptable salt or solvate.

The term "pharmaceutically acceptable" refers to those compounds or salts and solvates thereof as well as pharmaceutical, compositions, and/or dosage forms which are, within the scope of sound medical judgment, suitable for treatment of human beings and animals without excessive toxicity, irritation, allergic response, or other problem complications commensurate with a reasonable benefit/ risk ratio.

Typical pharmaceutically acceptable salts include those salts prepared by reaction of the compounds of the present invention with a pharmaceutically acceptable mineral or organic acid or an organic or inorganic base. Such salts are known as acid addition and base addition salts. Acids commonly employed to form acid addition salts are inorganic acids such as hydrochloric acid, hydrobromic acid, hydroiodic acid, sulfuric acid, phosphoric acid, and the like, and organic acids such as p-toluenesulfonic acid, methanesulfonic acid, oxalic acid, p-bromophenylsulfonic acid, carbonic acid, succinic acid, citric acid, benzoic acid, acetic acid, and the like. Examples of such pharmaceutically acceptable salts are the sulfate, pyrosulfate, bisulfate, sulfite, bisulfite, phosphate, monohydrogenphosphate, dihydrogenphosphate, metaphosphate, pyrophosphate, bromide, iodide, acetate, propionate, decanoate, caprylate, acrylate, formate, hydrochloride, dihydrochloride, isobutyrate, caproate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, hydroxybenzoate, methoxybenzoate, phthalate, xylenesulfonate, phenylacetate, phenylpropionate, phenylbutyrate, citrate, lactate, gamma-hydroxybutyrate, glycolate, tartrate, methanesulfonate, propanesulfonate, naphthalene-1-sulfonate, napththalene-2-sulfonate, mandelate and the like. Preferred pharmaceutically acceptable acid addition salts are those formed with mineral acids such as hydrochloric acid and hydrobromic acid, and those formed with organic acids such as maleic acid and methanesulfonic acid. Salts of amine groups may also comprise quaternary ammonium salts in which the amino nitrogen carries a suitable organic group such as an alkyl, alkenyl, alkynyl, or aralkyl moiety. Base addition salts include those derived from inorganic bases, such as ammonium or alkali or alkaline earth metal hydroxides, carbonates, bicarbonates, and the like. Such bases useful in preparing the salts of this invention thus include sodium hydroxide, potassium hydroxide, ammonium hydroxide, potassium carbonate, sodium carbonate, sodium bicarbonate, potassium bicarbonate, calcium hydroxide, calcium carbonate, and the like. The potassium and sodium salt forms are particularly preferred. It should be recognized that the particular counter ion forming a part of any salt of this invention is usually not of a critical nature, so long as the salt as a whole is pharmacologically acceptable and as long as the counter ion does not contribute undesired qualities to the salt as a whole.

The term acceptable solvate encompasses also pharmaceutically acceptable solvates of the compounds of the invention, wherein the compound combines with a solvent such as water, methanol, ethanol or acetonitrile to form a pharmaceutically acceptable solvate such as the corresponding hydrate, methanolate, ethanolate or acetonitrilate.

### Pharmaceutical Composition

As described above, the present invention also relates to a pharmaceutical composition comprising in a therapeutically effective amount the compound of the invention..

The term "therapeutically effective amount", as used herein, preferably refers to an amount of the compound, as defined herein, that effectively inhibits the HIV capsid assembly.

As far as the pharmaceutical compositions according to the present invention, are concerned, the conjugate may be used in combination with a pharmaceutical excipient. Generally, the compound will be in a solid form which can be combined with a suitable pharmaceutical excipient that can be in either solid or liquid form. As excipients, carbohydrates, inorganic salts, antimicrobial agents, antioxidants, surfactants, buffers, acids, bases, and combinations thereof may be mentioned. A carbohydrate such as a sugar, a derivatized sugar such as an alditol, aldonic acid, an esterified sugar, and/or a sugar polymer may be present as an excipient. Specific carbohydrate excipients include, for example: monosaccharides, such as fructose, maltose, galactose, glucose, D-mannose, sorbose, and the like; disaccharides, such as lactose, sucrose, trehalose, cellobiose, and the like; polysaccharides, such as raffmose, melezitose, maltodextrins, dextrans, starches, and the like; and alditols, such as mannitol, xylitol, maltitol, lactitol, sorbitol (glucitol), pyranosyl sorbitol, myoinositol, and the like. The excipient may also include an inorganic salt or buffer such as citric acid, sodium chloride, potassium chloride, sodium sulfate, potassium nitrate, sodium phosphate monobasic, sodium phosphate dibasic, and combinations thereof. The pharmaceutical composition according to the present invention may also comprise an antimicrobial agent for preventing or determining microbial growth, such as, e.g., benzalkonium chloride, benzethonium chloride, benzyl alcohol, cetylpyridinium chloride, chlorobutanol, phenol, phenylethyl alcohol, phenylmercuric nitrate, thimersol, and combinations thereof.

The pharmaceutical composition according to the present invention may also comprise an antioxidant, such as, e.g., ascorbyl palmitate, butylated hydroxyanisole, butylated hydroxytoluene, hypophosphorous acid, monothioglycerol, propyl gallate, sodium bisulfite, sodium formaldehyde sulfoxylate, sodium metabisulfite, and combinations thereof.

The pharmaceutical composition according to the present invention may also comprise a surfactant, such as, e.g., polysorbates, or pluronics sorbitan esters; lipids, such as phospholipids and lecithin and other phosphatidylcholines, phosphatidylethanolamines, acids and fatty esters; steroids, such as cholesterol; and chelating agents, such as EDTA or zinc.

The pharmaceutical composition according to the present invention may also comprise acids or bases such as, e.g., hydrochloric acid, acetic acid, phosphoric acid, citric acid, malic acid, lactic acid, formic acid, trichloroacetic acid, nitric acid, perchloric acid, phosphoric acid, sulfuric acid, fumaric acid, and combinations thereof, and/or sodium hydroxide, sodium acetate, ammonium hydroxide, potassium hydroxide, ammonium acetate, potassium acetate, sodium phosphate, potassium phosphate, sodium citrate, sodium formate, sodium sulfate, potassium sulfate, potassium fumarate, and combinations thereof.

### Inhibition of the assembly of the HIV capsid

The compound of the present invention shall inhibit the assembly of the HIV capsid, in particular, of the HIV-1 capsid. Preferably, the term "capsid" refers to the protein shell of the HIV virus. The capsid of HIV is well known in the art, and, e.g. described by Engelman, A. and Cherepanov, P. Nature Reviews Microbiology 10, 279-290 (2012). A major component of the HIV capsid, is the so called CA protein (capsid protein) which originated from the Gag polyprotein which is processed during maturation into various polypeptides.

Preferably, the pharmaceutical composition according to the present invention comprises at least one further antiretroviral agent as set forth herein below.

Moreover, the present invention relates to use of a compound of the present invention for the manufacture of a HIV capsid inhibitor, and, thus, for a medicament for inhibiting HIV capsid assembly.

Further envisaged by the present invention is method of treating a patient suffering from HIV comprising administering a therapeutically effective amount of the compound or the pharmaceutical composition of the present invention to said subject.

The term "patient" as used herein, preferably, relates to a human. Moreover, the patient according to the present invention is preferably infected with the human immunodeficiency virus (HIV). As set forth elsewhere herein, the human immunodeficiency virus is a retrovirus that may cause acquired immunodeficiency syndrome (AIDS), a condition which may lead to life-threatening opportunistic infections. HIV in the context of the present invention, preferably, refers to HIV-1 and HIV-2. However, it is particularly contemplated that the term "HIV" refers to HIV-1.

Antiretroviral medicaments may be also used for pre-exposure and post-exposure prophylaxis (see Morbidity and Mortality Weekly Reports 2011;60 (03);65-68). Therefore, is it also contemplated that the compound or the pharmaceutical composition of the present invention is used for pre-exposure and post-exposure prophylaxis. Thus, the compound or the pharmaceutical composition of the present invention may also be administered to subjects which are not infected with HIV (in case of pre-exposure prophylaxis), or in subjects which are suspected of having contracted HIV recently (in case of post-exposure prophylaxis). Thereby, HIV infection may be prevented.

The compound or composition of the present invention may be administered by any way deemed appropriate. Preferably, the compound or composition of the present invention is administered orally, parenterally, intracisternally, intrasternally, intravaginally, intraperitoneally or topically. It is particularly preferred to administer the compound or composition of the present invention orally or parentally. Parental administration as used herein, preferably, refers to modes of administration which include intravenous, intramuscular, intraperitoneal, intracisternal, intrasternal, subcutaneous and intraarticular injection and infusion.

Preferably, the compound or composition of the present invention is administered in combination with at least one further antiretroviral HIV medicament. Preferably, the at least one further antiretroviral medicament is selected from the group consisting of: a nucleoside reverse transcriptase inhibitor (NRTI), a non-nucleoside reverse transcriptase inhibitor (NNRTI) a protease inhibitor and an integrase inhibitor.

In the following especially preferred embodiments of the present invention are described:
1. As compound or a pharmaceutically acceptable salt or solvate thereof for use in inhibiting HIV capsid assembly, the compound comprising the core structure 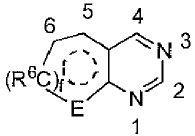 wherein E is CR⁷or S, and wherein f is 0 or 1, and wherein in case E is S, f is 0, and wherein the core structure is at least substituted in 2 and 4 position, and wherein the residue R6 and R⁷, are, independently of each other, selected from the group consisting of -H, -D, -alkyl, alkoxy, alkenyl, alkynyl, halides, -NO₂ -OH, - NH₂, -NH^{4#} -CN, -S(O)^{R#}, -SO₂R^{4#}, -P(O)R^{4#}R^{5#}, -P(O)(OR^{4#})R^{5#}, - P(O)(OR^{4#})(OR^{5#}), -C(O)NR^{4#}R^{5#}, -C(O)SR^{4#}, -C(O)R^{4#}, -C(O)O-R^{4#}, alkoxy and glycol chains; and wherein R⁶ may optionally form a cyclic residue, with a further substituent present 5 or 6 position, and wherein R^{4#} and R^{5#} are, independently of each other, selected from the group consisting of -H, -alkyl, -alkenyl, - heterocycloalkyl, aryl and heteroaryl.
2. The compound according to embodiment 1 having a structure according to formula (I) 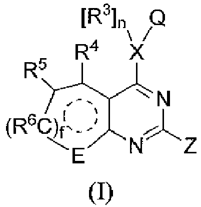 or a pharmaceutically acceptable salt or solvate thereof, wherein Z is 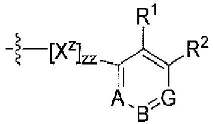 wherein X^{Z} is selected from the group consisting of O, NH, N(Me) and S, and wherein integer zz is 0 or 1, more preferably wherein Z is 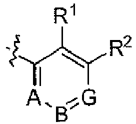 and wherein R¹ and R², are, independently of each other, selected from the group consisting of H, D, -alkyl, cycloalkyl, cycloalkenyl, heterocycloalkyl, aryl, heteroaryl, alkenyl, alkoxy, halides, -C(O)R^{1#}, -C(O)OR^{1#}, -S(O)R^{1#}, -SO₂R^{1#}, - P(O)R^{1#}R^{2#}, -P(O)(OR^{1#})R^{2#}, -P(O)(OR^{#})(OR^{2#}), -C(O)NR^{1#}R^{2#}, -C(O)NR^{1#} and - C(O)SR^{1#}, wherein R1# and R2# are, independently of each other, selected from the group consisting of -H, -alkyl, -alkenyl, -heterocyclaalkyl, aryl and heteroaryl,
   and wherein, A is N or C-R⁸, and wherein B is N or C-R⁹, and wherein C is N or C-R¹⁰
   and wherein R⁸, R⁹ and R¹⁰, are, independently of each other, selected from the group consisting of -H, -D, -alkyl, cycloalkyl, cycloalkenyl, heterocycloalkyl, heterocycloalkenyl, aryl, heteroaryl, alkenyl, alkoxy, halides, -C(O)R^{1#}, -C(O)OR^{8#}, -S(O)R^{8#}, -SO₂R^{8#}, -P(O)R^{8#}R^{9#}, -P(O)(OR^{8#})R^{9#}, -P(O)(OR^{8#})(OR^{9#}), - C(O)NR^{8#}R^{9#}, -C(O)NR^{8#} and -C(O)SR^{8#}, and wherein R^{8#} and R^{9#} are, independently of each other, selected from the group consisting of -H, -alkyl, - alkenyl, -heterocycloalkyl, aryl and heteroaryl,
   X is selected from the group consisting of N, O or S,
   wherein R³ is selected from the group consisting of H, alkyl, cycloalkyl, aryl, heteroaryl and heterocycloalkyl,
   wherein integer n is 0 or 1,
   and wherein R⁴, R⁵, are, independently of each other, selected from the group consisting of -H, -D, -alkyl, alkoxy, alkenyl, alkynyl, halides, -NO₂, -OH, -NH₂, - NHR^{4#}, -CN, -S(O)R^{4#}, -SO₂R^{4#}, -P(O)R^{4#}R^{5#}, -P(O)(OR^{4#})R^{5#}, -P(O)(OR^{4#})(OR^{5#}), -C(O)NR^{4#}R^{5#}, -C(O)SR^{4#}, -C(O)R^{4#}, -C(O)O-R^{4#}, alkoxy and glycol chains;
   wherein R⁴ and R⁵ or R⁵ and R⁶ can form a cyclic residue,
   and wherein R^{4#} and R^{5#} are, independently of each other, selected from the group consisting of -H, -alkyl, -alkenyl, -heterocycloalkyl, aryl and heteroaryl,
   and wherein Q is selected from the group consisting of alkyl, alkoxy, cycloalkyl, heterocycloalkyl, aryl, alkoxyalkyl and heteroaryl,
   with the proviso that in case X is S, n is 0, and in case X is N, n is 1.
3. The compound according to embodiment 2, wherein R¹ and R², are, independently of each other, selected from the group consisting of C1-20alkyl, C3-10cycloalkyl, C3-10heterocycloalkyl, C3-10cycloalkenyl, C2-20alkenyl, C6-10aryl, C5-10heteroaryl, C(O)R^{1#}, C(O)OR^{1#}, -H, -D, -C1-10alkoxy, and -F, wherein R^{1#} is C₁₋₁₀alkyl.
4. The compound according to any one of embodiments 2 or 3, wherein R⁸, R⁹ and R¹⁰, are, independently of each other, selected from the group consisting of -H, C1-10alkyl, -O-[C1-10alkyl], -I, -Br, -Cl and -F.
5. The compound according to any one of embodiments 2 to 4, having a structure according to formula (II) 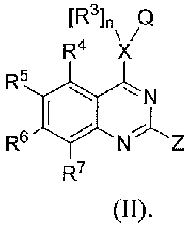
6. The compound according to any one of embodiments 2 to 4, wherein at least one of A, B and G is N.
7. The compound according to any one of embodiments 2 to 6, wherein X is N and n is 1, the compound thus having the 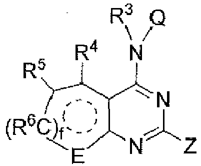
8. The compound according embodiment 5 or 7, wherein R5 and R6 are, independently of each other, selected from the group consisting of -H, -CF₃, CHF₂, CH₂F, H, -OH, C1-10alkyl, C1-10alkoxy, in particular methoxy or ethoxy, -NO₂,- - C(O)O-R^{4#}, with R^{4#} being C1-10alkyl, and glycol chains, in particular -O-[CH₂-CH₂-O]_{g}-Me with integer g being preferably of from 1 to 10.
9. The compound The compound according embodiment 5 or 7 or 8, wherein R⁷, is H or alkoxy, and preferably selected from the group consisting of H, methoxy and ethoxy, more preferably H or methoxy, most preferably H.
10. The compound according to any one of embodiments 2 to 9, wherein X is N, n is 1 and R³ is H or alkyl, preferably H or methyl, more preferably H.
11. The compound according to any one of embodiments 2 to 10, wherein Q is selected from the group consisting of C1-10alkyl, C1-10alkoxy, C3-10cycloalkyl, C3-10heterocycloalkyl, C6-10aryl and C5-10heteroaryl-.
12. The compound according to any one of embodiments 2 to 11, wherein Q is selected from the group consisting of an C1-10alkyl group, which is optionally substituted with at least one fluoride, the group 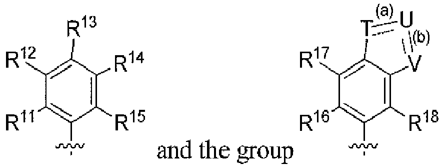 wherein R¹¹, R¹², R¹³, R¹⁴, and R¹⁵ are, independently of each other, selected from the group consisting of -H, -alkyl, -halides, alkoxy, alkynyl, alkenyl, -NO₂, -CN, - C(=Y)- R^{11#}, -C(=Y)-H, -C(=Y)-Y¹-R^{11#}, -C(=Y)-OH, , -Y-R^{11#}, -NH₂, -NHR^{11#},-CN, -F, -CF₃, -S(O)R^{11#}, -SO₂R^{11#}, -P(O)R^{11#}R^{12#}, -P(O)(OR^{11#})R^{12#}, - P(O)(OR^{11#})(OR^{12#}),
   wherein Y is O, S or NH, and wherein Y¹ is O, S or NR*, wherein R* is H or alkyl, and wherein R^{11#} and R^{12#} are, independently of each other, selected from the group consisting of -H, -alkyl, -alkenyl, -heterocycloalkyl, aryl and heteroaryl,
   wherein T is -C-R¹⁹, N or N-Y^{T}, with Y^{T} being H or alkyl,
   wherein U is -C-R²⁰, N or N-Y^{U}, with Y^{U} being H or alkyl, and
   wherein V is -C-R²¹, N or N-Y^{Y}, with Y^{V} being H or alkyl
   the bond (a) is a single or double bond,
   the bond (b) is a single or double bond,
   with eth proviso that one of (a) and (b) is a double bond,
   R¹⁹, R²⁰, and R²¹ are, independently of each other, H or -alkyl, and wherein R¹⁶, R¹⁷ and R¹⁸, are independently of each other selected from the group consisting of
   -H, -alkyl, -halides, alkoxy, alkynyl, alkenyl, -NO₂, -C(=Y)-alkyl, -C(=Y)-H, - C(=Y)-Y¹-alkyl, -C(=Y)-OH and -Y-alkyl-.
13. The compound according to any one of embodiments 2 to 12, wherein Q is 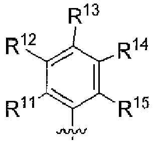 wherein R¹¹, R¹², R¹³, R¹⁴, and R¹⁵ are, independently of each other, selected from the group consisting of -H, -alkyl, -halides, alkoxy, alkynyl, alkenyl, -NO₂, -CN, - C(=Y)- R^{11#}, -C(=Y)-H, -C(=Y)-Y¹,R^{11#}, -C(=Y)-OH, , -Y-R^{11#}, -NH₂, -NHR^{11#},-CN, -F, -CF₃, -S(O)R^{11#}, -SO₂R^{11#}, -P(O)R^{11#}R^{12#}, -P(O)(OR^{11#})R^{12#}, - P(O)(OR^{11#})(OR^{12#}),
   wherein Y is O, S or NH, and wherein Y¹ is O, S or NR*, wherein R* is H or alkyl, and wherein R^{11#} and R^{12#} are, independently of each other, selected from the group consisting of -H, -alkyl, -alkenyl, -heterocycloalkyl, aryl and heteroaryl.
14. The compound according to embodiment 13, wherein at least one of R¹¹, R¹², R¹³, R¹⁴, and R¹⁵ is selected from the group consisting of -F, -I, NO₂, -C(=O)-alkyl, - C(=O)-OH, -C(=O)-O-alkyl, -C(=Y)-OH and -NH-alkyl.
15. The compound according to embodiment 13 or 14, wherein at least one of R¹¹, R¹², R¹³, R¹⁴, and R¹⁵ is -F.
16. The compound according to embodiment 13 or 14 or 15, wherein Q is selected from the group consisting 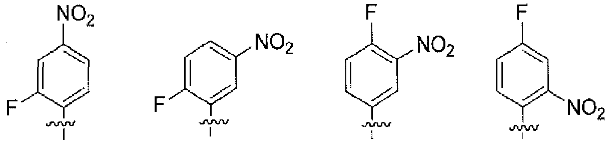 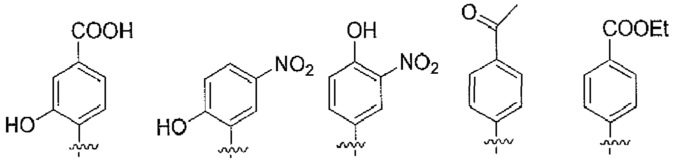 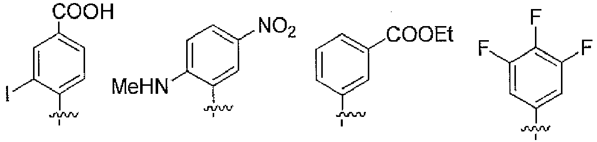
17. The compound according to any one of embodiments 2 to 12, wherein Q is 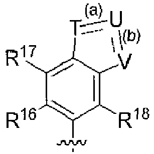 wherein T is -C-R¹⁹, N or N-Y^{T}, with Y^{T} being H or alkyl wherein U is -C-R²⁰, N or N-Y^{U}, with Y^{U} being H or alkyl wherein V is -C-R²¹, N or N-Y^{Y}, with Y^{V} being H or alkyl the bond (a) is a single or double bond,
   the bond (b) is a single or double bond,
   with eth proviso that one of (a) and (b) is a double bond,
   R¹⁹, R²⁰, and R²¹ are, independently of each other selected from the group consisting of H, -alkyl,
   and wherein R¹⁶, R¹⁷ and R¹⁸, are independently of each other selected from the group consisting of -H, -alkyl, -halides, alkoxy, alkynyl, alkenyl, -NO₂, -C(=Y)-alkyl, -C(=Y)-H, -C(=Y)-Y¹-alkyl, -C(=Y)-OH and -Y-alkyl.
18. The compound according embodiment 17, wherein Q is selected from the group consisting of 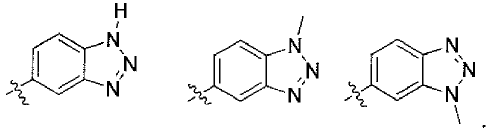
19. The compound according to any one of embodiments 2 to 18, wherein Z is selected from the group consisting of 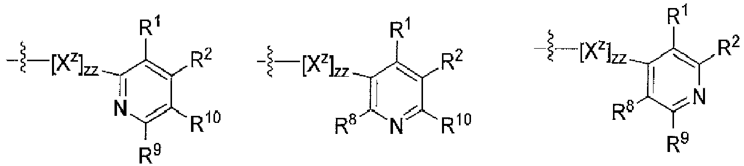
20. The compound according any one of embodiments 2 to 19, wherein Z has the following structure: 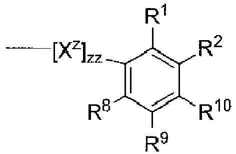
21. The compound according to any one of embodiments 2 to 20, wherein Z is selected from the group consisting of 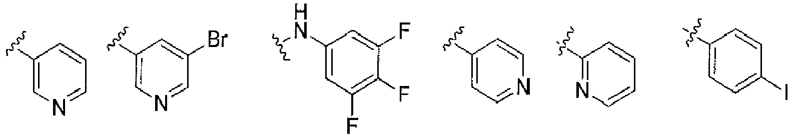
22. A compound according any one of embodiments 1 to 21, wherein HIV is HIV-1.
21. A pharmaceutical composition comprising in a therapeutically effective amount a compound according to any one of embodiments 1 to 22.
23. Use of a compound as defined in any one of embodiments 1 to 22 for the manufacture of a medicament for inhibiting HIV capsid assembly.
24. A method of treating a patient suffering from HIV comprising administering a therapeutically effective amount of the compound as defined in any one of embodiments 1 to 13.

### Figures:

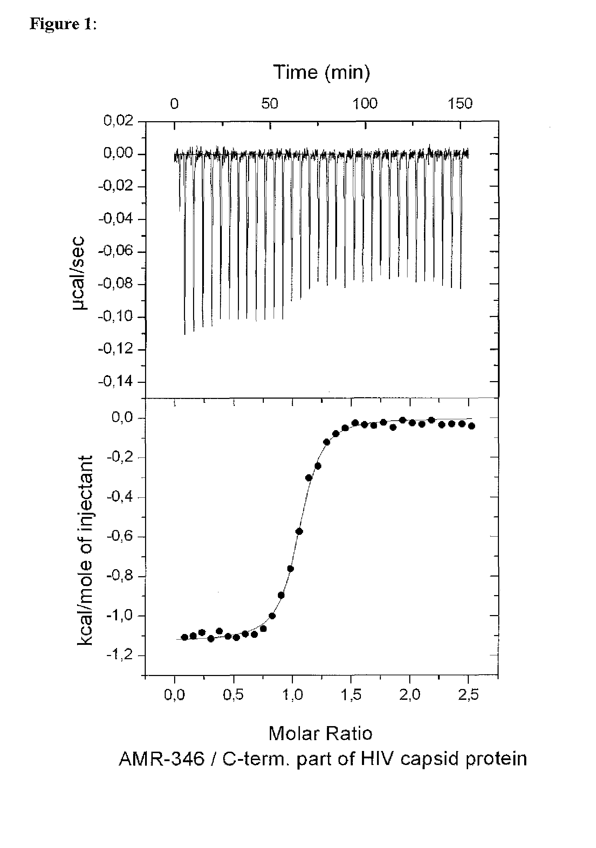

**Figure 1** shows the isothermal titration of CA ligand AMR-346 with HIV capsid protein C-terminal part. Upper graph: raw experimental data. Lower graph: Fit (line) to the integrated heats (full circles) from each injection, corrected for the heat of dilution of CA ligand AMR-346.

### Examples:

### I. General procedures of synthesis of the compounds according to the invention

### General scheme of the synthesis of the compounds of interest

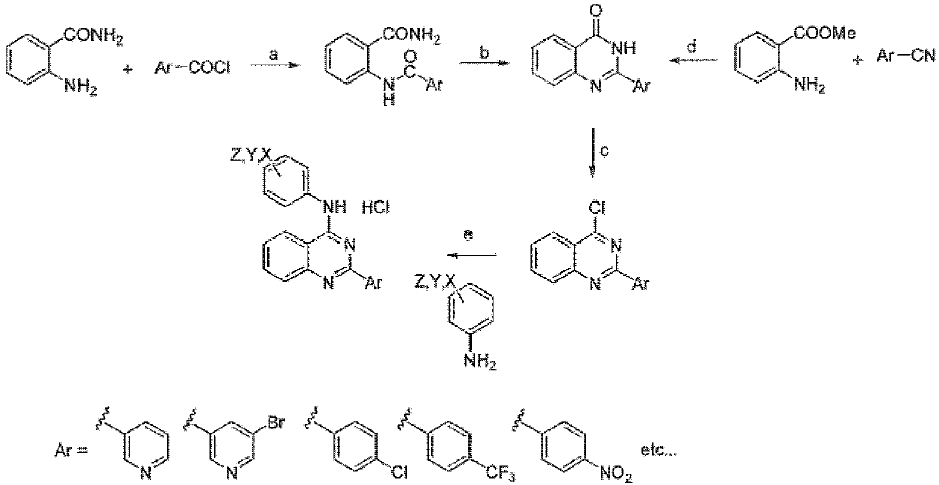

X, Y, Z = H, X, OR, COOR, NO₂ , etc ...
reaction conditions:
a: Et₃N / dioxane, CH₂Cl₂, reflux; b: 1 M NaOH in H₂O, reflux; c: POCl₃ / DMF, 90 °C; d: 6 M HCl in dioxane, reflux; e: cat. HCl / dioxane, reflux or 1. t-BuONa / THF, 2. 3 M HCl

### I.1 General procedure A - synthesis of 4-chloro quinazolines

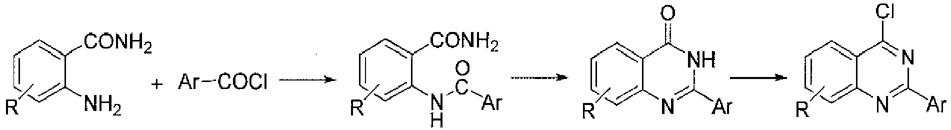

To a solution of 2-aminobenzamide (1.0 mmol) and triethylamine (10.0 mmol) in tetrahydrofurane (1.0 ml) and dichloromethane (1.0 ml) was added aromatic acid chloride (1.0 mmol) in one portion. The reaction mixture was heated to reflux with stirring. After 15 h the solvents were removed in vacuo and obtained solid was dissolved in ethyl acetate (10.0 ml) and extracted with water (3.0 ml) three times. After evaporation of solvent the appropriate N-(2-carbamoylphenyl)arylamide was obtained. This compound was then dissolved in aqueous KOH (1M, 3.0 ml) and stirred with heating to 100° C for 10 h. The resulting solution was diluted with water (20.0 ml) and the resulting 2-arylquinazolin-4(3*H*)-one was filtered off and washed with water (20.0 ml). Treating this compound with POCl₃ (10.0 mmol) in dimethylformamide (10.0 ml) at 80° C followed by decomposition of excess of POCl₃ on ice (100 g) and neutralization with sodium hydroxide (20% aqueous solution) led to crude product. Appropriate 4-chloroquinazoline derivative was obtained after chromatography (CHCl₃ with gradient of MeOH).

### I.2 General procedure B - synthesis of 2-arylquinazolin-4(3H)-ones

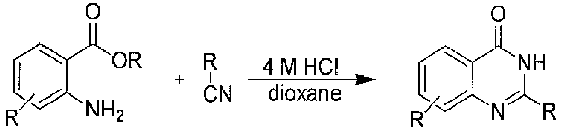

The appropriate aminoester (30 mmol), and the appropriate nitrile (30 mmol), were placed in 150 ml flask, and saturated HCl solution in dioxane (30 ml) was added dropwise. The reaction mixture was heated with stirring at 100 °C for 16 h. After it was cooled to rt, the reaction mixture was poured into water (100 mL). The formed precipitate was filtered and washed with a small amount of EtOH to give the title compound.

### I.3 General procedure C - synthesis of 4-chloro 2-arylquinazoline

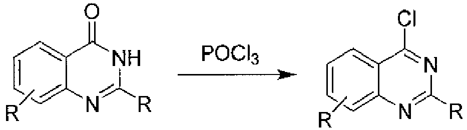

To a solution of 2-arylquinazolin-4(3*H*)-one (30 mmol) in dimethylformamide (30 ml) were added POCl₃ (300 mmol). The reaction mixture was heated with stirring at 80° C for 16 h. After it was cooled to rt, the reaction mixture was poured into ice-water, followed by neutralization with 2M NaOH leads to crude 4-chloroquinazoline derivative. Appropriate 4-chloroquinazoline derivative was obtained after chromatography (CHCl₃ with gradient of MeOH).

### I.4 General procedures of synthesis substituted quinazoline-4-amine analogues

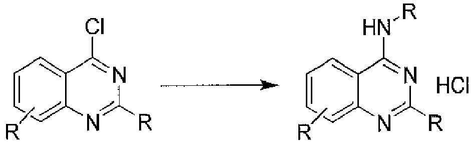

### I.5 General procedure D

To a solution of starting quinazoline derivative (1.0 mmol) and aryl amine (1.2 mmol) in dioxane is heated to 100° C. Then two drops of concentrated hydrochloric acid are added. Resulting solution is then heated to 100° C for 15 h or until disappearance of starting quinazoline derivative. Solvents are evaporated in vacuo and chromatography (CHCl₃ with gradient of MeOH) leads to a desired product.

### I.6 General procedure E

To a solution of starting quinazoline derivative (1.0 mmol) and aryl amine (1.2 mmol) in dry THF was added solution of sodium *tert*-butoxide (3 mmol) in THF. The reaction mixture was stirred at RT overnight or until disappearance of starting quinazoline derivative. The reaction mixture was concentrated and the residue was adsorbed on silica gel and purified by flash column chromatography on silica gel (CHCl₃ with gradient of MeOH) leads to a desired product, which was converted to hydrochloric salt by treatment with HCl ethanol.

### I.6 General procedure F

To a de-gassed mixture of quinazoline derivative (0.313 mmol), arylamine (0.376 mmol), and Cs₂CO₃ (0.438 mmol) in dioxane (5 ml) under argon was added Pd(OAc)₂ (0.016 mmol) and BINAP (0.023 mmol). The mixture was stirred at 80°C for 16 hours. The reaction mixture was concentrated and the residue was adsorbed on silica gel and purified by flash column chromatography on silica gel (eluents Hex/EA: 1/0 -->1/1) to afford title compound, which was converted to hydrochloric salt by treatment with HCl ethanol.

### II. Specifc examples:

### II.1 Example 1

### 2-(pyridin-3-yl)quinazoline-4(3H)-thione

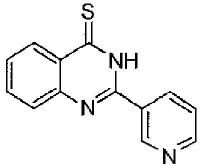

A solution of 2-(Pyridin-3-yl)quinazolin-4(3*H*)-one (1.5 g, 6.7 mmol) and Lawesson's reagent (2.2 g, 5.4 mmol) in pyridine (30 mL) was irradiated at 115°C (power input: 70 W) until completion (TLC monitoring, 45 min). After cooling, the solvent was removed under reduced pressure. The residue was then poured into boiling water and was filtered off. The collected yellow solid was dissolved into 1 M NaOH solution and the solution was filtered again. The filtrate was neutralised with a saturated NH₄Cl solution until complete crystallisation occurred. After filtration the title compound was obtained as pale yellow solid (1.3 g, 82 %).
¹H NMR (400 MHz, DMSO-d6) δ 14.16 (bs, 1H); 9.25 (bd, 1H, *J* = 2.0 Hz); 8.77 (dm, 1H, *J* = 5.1 Hz); 8.62 (dm, 1H, *J* = 8.2 Hz); 8.48 (dm, 1H, *J* = 8.1 Hz); 7.92 (m, 1H); 7.81 (bd, 1H, *J* = 8.1 Hz); 7.62 (m, 1H); 7.59 (m, 1H).
¹³C (125 MHz, DMSO-d6) δ 188.1; 152.0; 150.3; 149.5; 144.4; 136.4; 135.7; 129.5; 128.7; 128.5, 128.5; 128.0; 123.6.
HRMS: calcd for C₁₃H₈N₃S, 238.04444; found, 238.04392

### Example 2

### 4-(2-fluoro-4-nitrophenylthio)-2-(pyridin-3-yl)quinazoline hydrochloride

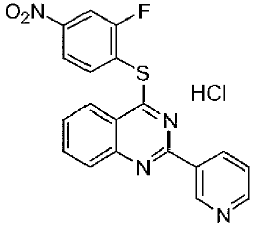

To a de-gassed mixture of 2-(pyridin-3-yl)quinazoline-4(3*H*)-thione (0.2 g, 0.836 mmol, example 1), 1,2-difluoro-4-nitrobenzene (0.093 ml, 0.836 mmol), and K₂CO₃ (0.347, 2.51 mmol) in DMF (7 ml) under argon. The mixture was stirred at rt for 16 hours. The reaction mixture was concentrated and the residue was adsorbed on silica gel and purified by flash column chromatography on silica gel (eluents Hex/EA: 1/0 --> 1/2) to afford title compound, which was converted to pale yellow hydrochloric salt (0.240 g, 76%) by treatment with HCl ethanol.
¹H NMR (400 MHz, DMSO-d6) δ 9.15 (bd, J= 2.3 Hz, 1H); 8.66 (dd, J = 4.8 Hz, J = 1.7 Hz, 1H); 8.48 (dd, J = 8.7 Hz, J = 2.4 Hz, 1H); 8.39 (dt, J = 8.0 Hz, J = 2.0 Hz, 1H); 9.34 (dm, J = 8.3 Hz, 1H); 8.31 (dd, J = 8.5 Hz, J= 2.4 Hz, 1H); 8.19 (dd, J = 8.5 Hz, J= 6.9 Hz, 1H); 8.10 - 8.14 (m, 2H); 7.85 (m, 1H); 7.50 (ddd, J= 8.0 Hz, J= 4.8 Hz, J= 0.9 Hz).
¹³C (125 MHz, DMSO-d6) δ 168.4; 156.7; 151,8; 149.2; 148.9; 138.8; 135.9; 135.3; 132.4; 129.1; 129.1; 124.2; 124.1; 123.1 (d, J = 18.5 Hz); 121.5; 120.4 (d, *J*=3.7 Hz); 112.0 (d, J=28.4).
HRMS: calcd for C₁₅H₁₁O₂N₅F, 312.0891; found, 312.0891.

### N-(2-fluoro-4-nitrophenyl)-6,7,8-trimethoxy-2-(pyridin-3-yl)quinazolin-4-amine hydrochloride

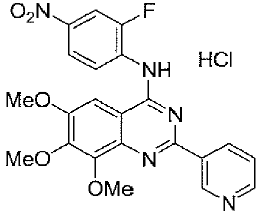

The title compound was prepared from 4-chloro-6,7,8-trimethoxy-2-(pyridin-3-yl)quinazoline (0.2 g, 0.604 mmol) and 2-fluoro-4-nitroaniline (0.113 g, 0.725 mmol) by using an analogous procedure to that described in general procedure F and was isolated as yellow solid (0.2 g, 68%).
¹H NMR (400 MHz, DMSO-d6) δ 9.53 (bd, 1H, *J* = 2.1 Hz); 9.17 (dt, 1H, *J* = 8.1 Hz, *J* = 1.8 Hz); 9.02 (dd, 1H, *J* = 5.5 Hz, *J* = 1.5 Hz); 8.93 (dd, 1H, *J* = 6.9 Hz, *J* = 2.9 Hz); 8.55 (d, 1H, *J* = 6.1 Hz); 8.10 (ddd, 1H, *J* = 8.1 Hz, *J* = 5.5 Hz, *J* = 0.7 Hz); 8.07 (ddd, 1H, *J* = 9.1 Hz, *J* = 3.9 Hz, *J* = 2.8 Hz); 7.62 (dd, 1H, *J* = 11.2 Hz, *J* = 9.1 Hz); 7.09 (d, 1H, *J* = 6.1 Hz).
¹³C (125 MHz, DMSO-d6) δ 160.3; 158.3; 154.5; 150.4 (d, *J* = 258.6 Hz); 145.6; 143.1; 142.1; 136.5 (d, *J* = 7.8 Hz); 136.1 (d, *J* = 3.1 Hz); 134.8; 127.7 (d, *J* = 8.2 Hz); 126.8; 119.2 (d, *J* = 22.2 Hz); 116.8 (d, *J* = 2.4 Hz); 108.0.
HRMS: calcd for C₂₂H₁₉FN₅O₅, 452.13693; found, 452.1365.

### N-(3-nitropyridin-4-yl)-2-(pyridin-3-yl)quinazolin-4-amine hydrochloride

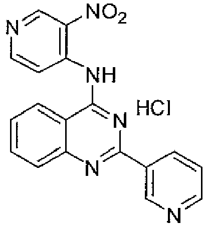

The title compound was prepared from 4-chloro-2-(pyridin-3-yl)quinazoline (0.15 g, 0.62 mmol) and 3-nitropyridin-4-amine (0.1 g, 0.745 mmol) by using an analogous procedure to that described in general procedure F and was isolated as yellow solid (0.147 g, 69 %). ¹H NMR (400 MHz, DMSO-d6) δ 11.73 (bs, 1H); 9.46 (bd, 1H, *J* = 2.0 Hz); 9.26 (bs, 1H); 9.16 (dt, 1H, *J* = 8.2 Hz, *J* = *J* = 1.7 Hz); 9.02 (bdd, 1H, *J* = 5.6 Hz, *J* = 1.4 Hz); 8.89 (d, 1H, *J* = 5.6 Hz); 8.74 (dm, 1H, *J* = 8.3 Hz); 8.19 (d, 1H, *J* = 5.7 Hz); 8.15 (bdd, 1H, *J* = 8.2 Hz, *J* = 5.5 Hz); 8.06-8.10 (m, 2H); 7.85 (m, 1H).
¹³C (125 MHz, DMSO-d6) δ 158.0; 154.2; 153.3; 150.3; 146.1; 144.3; 143.0; 142.3; 138.9; 135.5; 135.4; 128.6; 128.3; 127.2; 124.3; 119.5; 115.0.
HRMS: calcd for C₁₈H₁₃O₂ N₆,345.10945; found, 345.10943.

### N-(2-fluoro-4-nitrophenyl)-2-(pyridin-3-yl)quinolin-4-amine hydrochloride

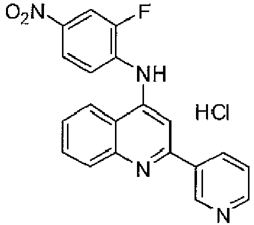

The title compound was prepared from 4-chloro-2-(pyridin-3-yl)quinoline (0.2 g, 0.931 mmol) and 2-fluoro-4-nitroaniline (0.139 g, 0.997 mmol) by using an analogous procedure to that described in general procedure F and was isolated as yellow solid (0.219 g, 73 %). ¹H NMR (400 MHz, DMSO-d6) δ 11.44 (bs, 1H); 9.27 (bd, 1H, *J*= 2.3 Hz); 8.96 (bd, 1H, *J*= 8.5 Hz); 891 (dd, 1H, *J*= 5.1 Hz, *J* = 1.4 Hz); 8.66 (dm, 1H, *J* = 8.1 Hz); 8.47 (bd, 1H, *J*= 8.6 Hz); 8.39 (dd, 1H, *J*= 10.5 Hz, *J*= 2.6 Hz); 8.27 (m, 1H); 8.10 (m, 1H); 7.96 (dd, 1H, *J*= 8.9 Hz, *J*= 7.9 Hz); 7.87 (m, 1H); 7.84 (dd, 1H, *J* = 8.1 Hz, *J*= 5.1 Hz); 7.23 (m, 1H).
¹³C (125 MHz, DMSO-d6) δ 155.6 (d, *J*= 252.9 Hz); 153.8; 150.7; 149.8; 147.4; 146.1; 139.7; 134.3; 132.5; 129.6; 128.3; 127.9; 124.9; 124.2; 122.1; 121.2; 117.6; 113.6 (d, *J*= 25.0 Hz); 102.2.
HRMS: calcd for C₂₀H₁₂O₂ N₄F, 359.09498; found, 359.09513.

### 2-(pyridin-3-yl)-N-(2,3,4,5-tetrafluorophenyl)quinazolin-4-amine hydrochloride

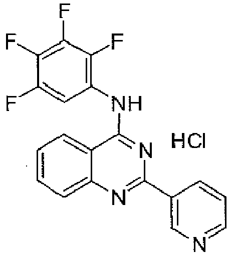

The title compound was prepared from 4-chloro-2-(pyridin-3-yl)qumazoline (0.2 g, 0.83 mmol) and 2,3,4,5-tetrafluoroaniline (0.164 g, 1.0 mmol) by using an analogous procedure to that described in general procedure E and was isolated as white solid (0.233 g, 76 %). ¹H NMR (400 MHz, DMSO-d6) δ 10.76 (bs, 1H); 9.43 (bd, 1H, *J*= 2.0 Hz); 9.05 (dt, 1H, *J* = 8.1 Hz, *J*= 1.8 Hz); 8.94 (dd, 1H, *J*= 5.5 Hz, *J*= 1.5 Hz); 8.65 (dm, 1H, *J*= 8.3 Hz); 8.00-8.05 (m, 3H); 7.74-7.80 (m, 2H).
¹³C (125 MHz, DMSO-d6) δ 159.1; 155.2; 149.3; 145.5; 143.5; 141.7; 135.4; 134.8; 128.0; 127.8; 126.6; 123.9; 114.1; 110.6 (bd, *J*= 21.3 Hz).
HRMS: calcd for C₁₉H₁₀ N₄ F₄, 370.0842; found, 370.0840.

### N-(5-bromo-2,4-difluorophenyl)-2-(pyridin-3-yl)quinazolin-4-amine hydrochloride

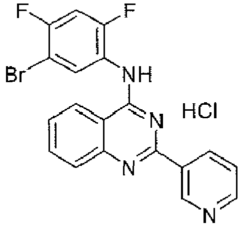

The title compound was prepared from 4-chloro-2-(pyridin-3-yl)pyrimidine quinazoline (0.2 g, 0.83 mmol) and 5-bromo-2,4-difluoroaniline (0.207 g, 1.0 mmol) by using an analogous procedure to that described in general procedure E and was isolated as yellow solid (0.233 g, 68 %).
¹H NMR (400 MHz, DMSO-d6) δ 11.43 (bs, 1H); 9.38 (bd, 1H, *J*= 2.0 Hz); 8.99 (dt, 1H, *J*= 8.2 Hz, *J*= 1.8 Hz); 8.91-8.95 (m, 2H); 8.15 (bd, 1H, *J*= 7.7 Hz); 8.05 (t, 1H, *J*= 7.7 Hz); 8.02 (m, 1H); 7.97 (bdd, 1H, *J*= 8.1 Hz, *J*= 5.4 Hz); 7.73-7.77 (m, 2H).
¹³C (125 MHz, DMSO-d6) δ 159.4; 155.5, 157.8; 155.2; 147.8; 146.4; 144.1; 141.0; 135.0; 134.2; 131.9; 127.9; 126.3, 123.4; 124.7; 123.8; 113.9; 106.2 (t, *J*=26.8 Hz); 102.7.
HRMS: calcd for C₁₉H₁₂ N₄BrF₂, 413.02079; found, 413.02070.

### N-(perfluorophenyl)-2-(pyridin-3-yl)quinazolin-4-amine hydrochloride

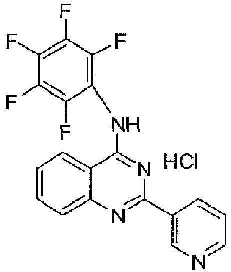

The title compound was prepared from 4-chloro-2-(pyridin-3-yl)pyrimidine (0.2 g, 0.83 mmol) and 2,3,4,5,6-pentafluoroaniline (0.182 g, 1.0 mmol) by using an analogous procedure to that described in general procedure E and was isolated as white solid (0.24 g, 75%).
¹H NMR (400 MHz, DMSO-d6) δ 10.50 (bs, 1H); 9.38 (bd, 1H, *J*= 2.0 Hz); 8.77-8.80 (m, 2H); 8.59 (bd, 1H, *J*= 8.3 Hz); 7.99-8.03 (m, 2H); 7.74-7.79 (m, 1H).
¹³C (125 MHz, DMSO-d6) δ 158.9; 156.9; 150.2; 150.0; 147.9; 136.9; 134.6; 133.7; 128.4; 127.6; 124.6; 123.5; 113.9.
HRMS: calcd for C₁₉H₉N₄F₅, 388.0747; found, 388.0751.

### N-(2-iodo-4-nitrophenyl)-2-(pyridin-3-yl)quinazolin-4-amine hydrochloride

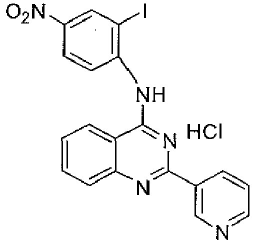

The title compound was prepared from 4-chloro-2-(pyridin-3-yl)pyrimidine (0.2 g, 0.83 mmol) and 2-iodo-4-nitroaniline (0.262 mg, 1.0 mmol) by using an analogous procedure to that described in general procedure E and was isolated as yellow solid (0.252 mg, 65%). ¹H NMR (400 MHz, DMSO-d6) δ 10.65 (bs, 1H); 9.36(bd, *J* = 1.9 Hz, 1H); 8.85-8.89 (m, 2H); 8.79 (d, *J* = 2.6 Hz, 1H); 8.64 (dm, *J* = 8.2 Hz, 1H); 8.42 (dd, *J* = 8.8 Hz, *J* = 2.6 Hz, 1H); 8.01 - 8.03 (m, 2H); 7.96 (d, *J* = 8.8 Hz, 1H); 7.89 (m, 1H); 7.78 (m, 1H).
¹³C (125 MHz, DMSO-d6) δ 158.7; 155.6; 149.2; 147.2; 147.0; 145.5; 145.0; 140.2; 134.7; 134.7; 134.6; 134.2; 128.7; 127.8; 127.7; 125.9; 124.2; 123.7; 114.2; 98.5. HRMS: calcd for C₁₉H₁₂N₅O₂I, 469.0036; found, 469.0044.

### 2-(pyridin-3-yl)-N-(pyrimidin-4-yl)quinazolin-4-amine hydrochloride

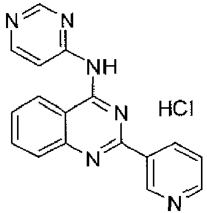

The title compound was prepared from 4-chloro-2-(pyridin-3-yl)pyrimidine (0.2 g, 0.83 mmol) and pyrimidin-4-amine (94 mg, 1.0 mmol) by using an analogous procedure to that described in general procedure E and was isolated as white solid (0.164 g, 66 %). ¹H NMR (400 MHz, DMSO-d6) δ 10.98 (bs, 1H); 9.60 (bd, 1H, *J*= 2.2 Hz); 9.00 (d, 1H, *J* = 1.3 Hz); 8.83 (d, 1H, *J*= 5.8 Hz); 8.74-8.77 (m, 3H); 8.57 (dd, 1H, *J*= 5.8 Hz, *J*= 1.3 Hz); 7.97-8.01 (m, 2H); 7.69 (m, 1H); 7.61 (dd, 1H, *J=* 7.9 Hz, *J*= 4.8 Hz).
¹³C (125 MHz, DMSO-d6) δ 159.6; 158.5; 158.2; 158.1; 157.38; 151.5; 150.9; 149.5; 135.7; 134.6; 133.5; 128.5; 127.5; 124.2, 124.1; 114.8; 111.7.
HRMS: calcd for C₁₇H₁₁N₆, 299.1045; found, 299.1044.

### N-(3,4,5-trifluorophenyl)quinazolin-4-amine hydrochloride

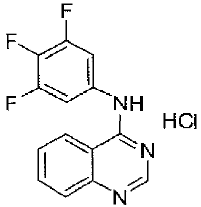

The title compound was prepared from 4-chloroquinazoline (0.4 g, 2.43 mmol) and 4-fluoro-3-nitroaniline (0:429 g, 2.92 mmol) by using an analogous procedure to that described in general procedure E and was isolated as white solid (0.455 g, 71 %).
¹H NMR (400 MHz, DMSO-d6) δ 11.62 (bs, 1H; 9.00 (s, 1H); 8.92 (bd, 1H, *J*= 8.5 Hz); 8.11 (ddd, 1H, *J*= 8.4 Hz, *J*= 7.1 Hz, *J*= 1.0 Hz); 7.98 (dd, 1H, *J*= 8.4 Hz, *J*= 1.0 Hz); 7.91 (m, 2H); 7.87 (ddd, 1H, *J*= 8.5 Hz, *J*= 7.1 Hz, *J=* 1.2 Hz).
¹³C (125 MHz, DMSO-d6) δ 159.8; 151.9; 150.0 (ddd, *J*=245.2 Hz, *J*= 10.1 Hz, *J*= 5.2 Hz); 105.0; 140.9; 136.8 (dt, *J*= 248.1 Hz, *J*= 15.9 Hz); 136.3; 133.8; 128.8; 124.8; 121.6; 114.0; 109.2 (d, *J*= 23.7 Hz); 103.2.
HRMS: calcd for C₁₄H₉N₃F₃, 276.07431; found, 276.07430.

### 2-(pyridin-3-yl)quinazolin-4-yl tetra-O-acetyl-β-D-1-thio-glucopyranoside

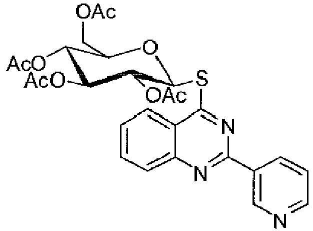

To a mixture of 2-(pyridin-3-yl)quinazoline-4(3*H*)-thione (0.15 g, 0.63 mmol, example 1) and KOH (5 mg, 0.63 mmol, in 10 ml of H₂O) in acetone (100 ml) was added acetobromo-α-D-glucose (0.28 g, 0.69 mmol). The mixture was stirred at rt for 16 hours. The reaction mixture was concentrated and the residue was adsorbed on silica gel and purified by flash column chromatography on silica gel (eluent CHCl₃/MeOH: 1/0 -->10/1) to afford title compound, which was converted to pale yellow salt (0.570 g, 55%).
¹H NMR (400 MHz, DMSO-d6) δ 9.80 (bs, 1H); 8.84 (dt, 1H, *J*= 7.9 Hz, *J*' = 1.8 Hz); 8.05 (bd, 1H, *J*= 8.4 Hz); 8.01 (dd, 1H, *J*= 8.4 Hz, *J*= 1.3 Hz); 7.91 (ddd, 1H, *J*= 8.5 Hz, *J*= 6.9 Hz, *J*= 1.4 Hz); 7.60 (ddd, 1H, *J*= 8.3 Hz, *J*= 6.9 Hz, *J*= 1.2 Hz); 7.52 (bdd, 1H, *J* = 7.8 Hz, *J*= 4.9 Hz); 6.26 (d, 1H, *J*= 10.4 Hz); 5.42-5.50 (m, 2H); 5.21 (t, 1H, *J*= 9.7 Hz); 4.24 (dd, 1H, *J* = 12.6 Hz, *J*= 6.0 Hz); 4.12-4.16 (m, 2H); 2.10 (s, 3H); 2.07 (s, 3H); 2.05 (s, 3H); 1.80 (s, 3H).
¹³C (125 MHz, DMSO-d6) δ 170.5, 170.0, 169.5, 169.5; 168.0; 157.0; 151.3; 150.0; 149.1; 135.6; 134.5; 133.2; 129.1; 127.7; 123.7; 123.5; 122.5; 80.0; 76.6; 74.0; 68.9; 68.2; 62.2; 21.0, 20.9, 20.8, 20.7.
HRMS: calcd for C₂₇H₂₈O₉N₃S, 570.15408; found, 570.15437.

### 2-phenyl-N-(3,4,5- trifluorophenyl)quinazolin-4-amine hydrochloride

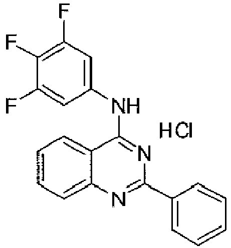

The title compound was prepared from 4-chloro-2-phenylquinazoline (0.4 g, 1.66 mmol) and 3,4,5-trifluoroaniline (0.293 g, 2.0 mmol) by using an analogous procedure to that described in general procedure E and was isolated as white solid (0.438 g, 75%).
¹H NMR (400 MHz, DMSO-d6) δ 11.21 (bs, 1H); 8.81 (bd, 1H, *J*= 8.3 Hz); 8.37 (m, 2H); 8.17 (bd, 1H, *J*= 8.4 Hz); 8.05 (m, 1H); 7.98 (m, 2H); 7.87 (m, 1H); 7.61-7.68 (m, 3H). ¹³C (125 MHz, DMSO-d6) δ 158.9; 158.1; 150.0; 150.0 (ddd, *J* = 244.8 Hz, *J*= 10.4 Hz, *J* = 5.6 Hz); 144.4; 136.0; 135.6; 134.4; 132.6; 129.1, 129.1; 128.9, 128.9; 127,9; 124.2; 123.7; 113.3; 108.3; 108.3.
HRMS: calcd for C₂₀H₁₃N₃F₃, 570.15408; found, 570.15437.

### 5-hydroxy-2-(2-(pyridin-3-yl)quinazolin-4-ylamino)benzoic acid hydrochloride

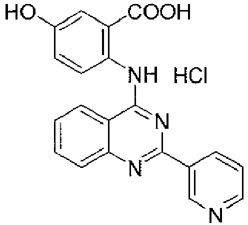

The reaction of 4-chloro-2-(pyridin-3-yl)quinazoline (400 mg, 1.65 mmol) and 2-amino-5-hydroxybenzoic acid (510 mg, 3.3 mmol) according to general procedure D gave the title compound (390 mg, 60 %) as a brown precipitate. Due its insolubility in DMSO the product was treated with NaOH to give free base for NMR experiments.
Free base ¹H NMR (500 MHz, DMSO-d6) δ 11.57 (bs, 1H), 9.49 (bs, 1H), 8.96 (bd, *J*= 8.0 Hz, 1H), 8.88 (bd, *J*= 5.1 Hz, 1H), 8.39 (d, *J*₅₋₆ = 8.4 Hz, 1H), 8.26 (bd, *J*= 8.2 Hz, 1H), 8.04-7.97 (m, 2H), 7.89 (m, 1H), 7.77 (m, 1H), 7.47 (d, *J*= 2.9 Hz, 1H), 7.19 (dd, *J*= 8.8 Hz, *J* = 2.9 Hz, 1H).
¹³C NMR (125 MHz, DMSO-d6) δ 169.08,158.28, 156.09, 154.23, 148.12, 147.59, 146.37, 139.30, 134.53, 133.85, 130.97, 127.97, 125.83, 125.32, 123.27, 122.70, 116.98, 114.22.
HRMS: calculated for C₂₀H₁₄N₄O₃, 358.1066, found 358.1059.

### 3,4-bis(2- methoxyethoxy) benzonitrile

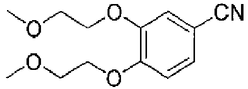

To a solution of 3,4-dihydroxybenzonitrile (10g, 74.0 mmol) and K₂CO₃ (30.7g, 222.0 mmol) in dimethylformamide was dropwise added 1-chloro-2-methoxyethane (28g, 296.0 mmol). The reaction mixture was heated with stirring to 70 °C overnight. Inorganic salts were removed by filtration and resulting solution was poured into the brine and then extracted with ethyl acetate. Solvents were removed in vacuo and title compound was obtained as white solid (13.2 g, 71 %).
Spectral data were in agreement with literature. Chandregowda, Venkateshappa; Rao, Gudapati Ventakeswara; Reddy, Goukanapalli Chandrasekara Heterocycles, 2007, vol.71, # 1 p.39-48.

### 4,5-bis(2-methoxyethoxy)-2-nitrobenzonitrile

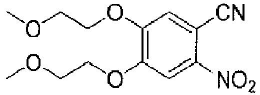

Nitric acid (65 %, 16.2 g, 254.5 mmol) was dropwise added directly into flask containing 3,4-bis(2-methoxyethoxy)benzonitrile (12.8 g, 50.9 mmol). The mixture was then heated with stirring to 50 °C until starting material was completely dissolved. After stirring for two days at RT the reaction mixture was dissolved with water (50 ml) and formed precipitate was filtered off and washed with water (100 ml). After crystallization from chloroform the title compound was obtained in form of white crystals (7.1 g, 47 %). Spectral data were in agreement with literature.Chandregowda, Venkateshappa; Rao, Gudapati Ventakeswara; Reddy, Goukanapalli Chandrasekara Heterocycles, 2007 , vol. 71, # 1 p. 39 - 48.

### 4,5-bis(2-methoxyethoxy)-2-nitrobenzamide

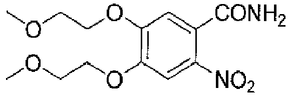

4,5-bis(2-methoxyethoxy)-2-nitrobenzonitrile (6.6 g, 23.3 mmol) was dissolved in ethanol. Then K₂CO₃ (0.5 g, 3.5 mmol) and NaOH (35.0 ml, 1M solution in water) were added. The resulting mixture was cooled to 0° C and then hydrogen peroxide (46.0 ml, 30% solution in water) was added dropwise with vigorous stirring. The reaction mixture was aged at RT overnight then water (100 ml) was added. Extraction with ethyl acetate afforded crude product (6.1 g, 83 %) which was used in the next reaction without additional purification.
Spectral data were in agreement with literature.

### 2-amino-4,5-bis(2-methoxyethoxy)benzanide

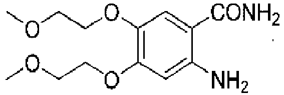

To a solution of 4,5-bis(2-methoxyethoxy)-2-nitrobenzatnide (6.1 g, 19.4 mmol) and ammonium formate (6.1 g, 102.4 mmol) in ethanol was added Pd/C (0.5 g) and the reaction mixture was heated with stirring to reflux for 2 h. Pd/C was then filtered off and solvent was removed in vacuo. Resulting crude product was suspended in water and filtered off to remove remaining ammonium formate. Obtained white solid (5.2 g, 95 %) used in the next reaction without additional purification.
Spectral data were in agreement with literature.

### N-(2-carbamoyl-4,5-bis(2-methoxyethoxy)phenyl)nicotinamide hydrochloride

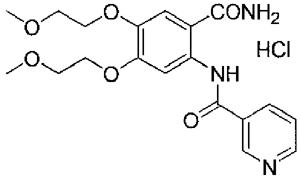

To a solution of 2-amino-4,5-bis(2-methoxyethoxy)benzamide (4.5 g, 15.8 mmol) and triethylamine (10 g, 97.8 mmol) in tetrahydrofurane and dichloromethane was added nicotinoyl chloride hydrochloride (3.1 g, 17.4 mmol) in one portion. The reaction mixture was heated to reflux with stirring. After 15 h the solvents were removed in vacuo and obtained solid was dissolved in ethyl acetate and extracted with water three times. After evaporation of solvent the title compound was obtained in form of white solid (3.2 g, 51 %).
¹H NMR (500 MHz, DMSO-d6) δ 13.44 (bs, 1H), 9.21 (dd, *J*= 2.4 Hz, *J*= 0.9 Hz, 1H), 8.78 (dd, *J*= 4.8 Hz, *J*= 1.7 Hz, 1H), 8.66 (s, 1H), 8.33 (ddd, *J*= 8.0 Hz, *J*= 2.4 Hz, *J*= 1.6 Hz, 1H), 7.94 (bs, 1H), 7.58 (ddd, *J* = 8.0 Hz,*J*= 4.8 Hz, *J* = 0.9 Hz, 1H), 7.56 (s, 1H), 7.06 (bs, 1H), 4.26 (m, 2H), 4.19 (m, 2H), 3.80 (m, 2H), 3.71 (m, 2H), 3.41 (s, 3H), 3.37 (s, 3H).
¹³C NMR (125 MHz, DMSO-d6) δ 172.27, 163.78, 153.53, 153.26, 149.39, 144.57, 137.67, 135.45, 13*J*.SS, 124.53, 115.30, 11.18, 106.15, 71.70, 71.32, 70.04, 69.15, 59.05, 58.99.
HRMS: calculated for C₁₉H₂₃N₃O₆, 389.1587; found, 389.1595

### 6,7-bis(2-methoxyethoxy)-2-(pyridin-3-yl)quinazolin-4(3H)-one

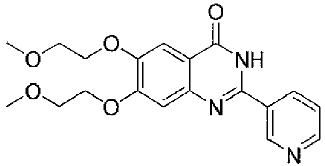

*N*-(2-carbamoyl-4,5-bis(2-methoxyethoxy)phenyl)nicotinamide hydrochloride (3.15 g, 8.1 mmol) was dissolved in aqueous KOH (25 ml, 1M) and stirred with heating to 100° C for 10 h. The resulting solution was diluted wit water and extracted six times with butanol. Solvents were removed in vacuo and title compound was obtained as white solid (3.0 g, 95 %). Sample for analysis was treated with HCl and then analysed as hydrochloride. Protonated product ¹H NMR (500 MHz, DMSO-d6) δ 12.60 (bs, 1H), 9.27 (dd, *J*= 2.4 Hz, *J*= 0.9 Hz, 1H), 8.72 (dd, *J* = 4.8 Hz, *J* = 1.6 Hz, 1H), 8.45 (ddd, *J*= 8.1 Hz, *J*= 2.4 Hz, *J*= 1.6 Hz, 1H), 7.56 (ddd, *J*= 8.1 Hz, *J*= 4.8 Hz, *J*= 0.9 Hz, 1H), 7.50 (s, 1H), 7.25 (s, 1H), 4.28 (m, 2H), 4.23 (m, 2H), 3.74-3.71 (m, 4H), 3.34 (s, 6H).
¹³C NMR (125 MHz, DMSO-d6) δ 161.63, 154.34, 151.74, 149.41, 148.75, 148.28, 144.81, 135.26, 129.01, 123.75, 114.45, 109.51, 106.55, 70.38, 70.29, 68.42, 68.33, 58.57, 58.56.
HRMS: calculated for C₁₉H₂₁N₃O₅, 371.1481; found, 371.1479

### 4-chloro-6,7-bis(2-methoxyethoxy)-2-(pyridin-3-yl)quinazoline

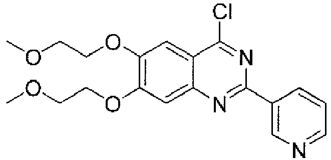

To a solution of 6,7-bis(2-methoxyethoxy)-2-(pyridin-3-yl)quinazolin-4(3H)-one (2.9 g, 7.8 mmol) in dimethylformamide cooled to 0 °C in ice bath is dropwise added POCl₃ (10.1, 65.9 mmol). Resulting solution is heated with stirring to 80° C for 8 h. The reaction mixture is then poured to an ice-water mixture and sodium hydroxide (50 ml, 20% solution in water) is then added with vigorous stirring. An orange precipitate obtained is then filtered of and washed with water to give a title compound (2.3 g, 75 %). ¹H NMR (500 MHz, DMSO-d6) δ 9.52 (dd, *J*= 2.2 Hz, J= 0.9 Hz, 1H), 8.71 (dd, *J*= 4.8 Hz, *J*= 1.7 Hz, 1H), 8.66 (ddd, *J*= 8.0 Hz, *J*= 2.3 Hz, *J*= 1.7 Hz 1H), 7.56 (ddd, *J*= 8.0 Hz, *J*= 4.8 Hz, *J* = 0.9 Hz 1H), 7.49 (s, 1H), 7.45 (s, 1H), 4,40 (m, 2H), 4,35 (m, 2H), 3.80-3.77 (m, 4H), 3.37 (s, 6H).
¹³C NMR (125 MHz, DMSO-d6) δ 158.63, 156.52, 156.00, 151.28, 150.85, 148.97, 148.91, 134.99, 132.05, 123.79, 117.42, 108.02, 104.02, 70.11, 69.98, 68.89, 68.77, 58.43. HRMS: calculated for C₁₉H₂₀ClN₃O₄, 389.1142; found, 389.1145

### 2-methylamino-5-nitroaniline

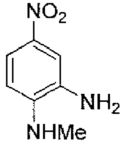

To a solution of 1-chloro-2,4-dinitrobenzene in tetrahydrofuran was added methylamine in portions. After stirring for 1 h at RT *N*-methyl-2,4-dinitroaniline precipitate from solution. The precipitate was filtered off, washed with water and cold ethanol. Than the a suspension of crude N-methyl-2,4-dinitroaniline in ethanol was added to solution of sodium sulphide nonahydrate and powder sulphur in water and heated to 100° C for 2 h. Diluting the reaction mixture a cooling it to 0° C lead to precipitation of crude product. After filtration and washing with water and cold ethanol the title compound was obtained (6.6 g, 68 %) as dark red precipitate.
Spectral data were in agreement with literature. Russ. J. Gen. Chem. 2006, 76, No 6, 1282-1287

### N²-(6,7-bis(2-methoxyethoxy)-2-(pyridin-3-yl)quinazolin-4-yl)-N⁴-methyl-4-nitrobenzene-1,2-diamine hydrochloride

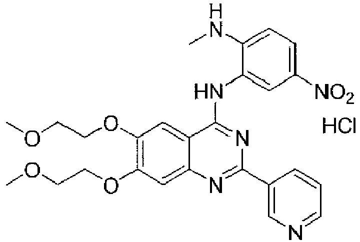

To a solution of 4-chloro-6,7-bis(2-methoxyethoxy)-2-(pyridin-3-yl)quinazoline (100 mg, 260 µmol) and 2-methylamino-5-nitroaniline (85 mg, 510 µmol) in dioxane were added 2 drops of concentrated hydrochloric acid. The resulting mixture was heated with stirring to reflux for 10 h. Formed precipitate was filtered off and washed sequentially with acetone to give the title compound (70 mg, 50 %).
¹H NMR (500 MHz, DMSO-d6) δ 9.47 (bs, 1H), 9.31 (dd, *J*= 2.2 Hz, *J=* 0.8 Hz, 1H),
8.57 (dd, *J*= 4.7 Hz, *J*= 1.7 Hz, 1H), 8.47 (dt, *J*= 8.0 Hz, *J*= 2.0 Hz,1H), 8.24 (bs, 1H), 8.12 (bdd, *J*= 9.2 Hz, *J*= 2.6 Hz, 1H), 7.92 (s, 1H), 7.44 (ddd, *J*= 7.9 Hz, *J*= 4.7 Hz, *J*= 0.9 Hz 1H), 7.30 (s, 1H), 6.86 (bs, 1H), 6.80 (d, *J*= 9.4 Hz, 1H), 4.33-4.29 (m, 4H), 3.80-3.76 (m, 4H), 3.38 (s, 3H), 3.36 (s, 3H), 2.87 (s, 3H).
¹³C NMR (125 MHz, DMSO-d6) δ 158.23, 156.21, 15-1.88, 151.58, 150.50, 149.04, 148.01, 147.40, 135.03, 134.66, 134.19, 124.35, 123.62, 123.22, 108.78, 108.58, 108.48, 104.48, 70.32, 72.31, 68.45, 68.26, 58.60, 58.56, 29.83.
HRMS: calculated for C₂₆H₂₈N₆O₆, 520.2070; found, 520.2074

### 4-chloro-5-fluoro-2-(pyridin-3-yl)quinazoline

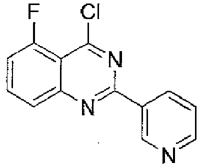

Reaction of 2-amino-6-fluorobenzamide (1 g, 6.5 mmol) with nicotinoyl chloride hydrochloride (1.15 g, 6.5 mmol) according to general procedure A gave the title compound (260 mg, 16 %) as white solid.
¹H NMR (500 MHz, DMSO-d6) δ 9.39 (bd, *J*= 2.0 Hz, 1H), 8.92 (dd, *J*= 5.2 Hz, *J*= 1.5 Hz, 1H), 8.79 (ddd, *J*= 8.1 Hz, *J*= 2.2 Hz, *J* = 1.5 Hz, 1H), 7.88 (bdd, *J=* 8.1 Hz, *J* = 5.2 Hz, 1H), 7.85 (td, *J*= 8.2 Hz, *J*= 5.6 Hz, 1H), 7.60 (dd, *J*= 8.2 Hz, *J*= 1.0 Hz, 1H), 7.32 (ddd, *J*= 10.9 Hz, *J*= 8.1 Hz, *J*= 1.0 Hz, 1H).
¹³C NMR (125 MHz, DMSO-d6) δ 161.55 (m), 150.90, 150.49, 148.65, 145.89, 139.67, 135.73 (d, *J* = 10.6 Hz), 129.92, 125.36, 123.92, 113.93 (d, *J* = 20.5 Hz), 110.91 (d, *J* = 6.7 Hz).
HRMS: calculated for C₁₃H₇ClFN₃, 259.0313; found, 259.0307

### phenyl(4-(2-(pyridin-4-yl)quinazolin-4-ylamino)phenyl)methanone hydrochloride

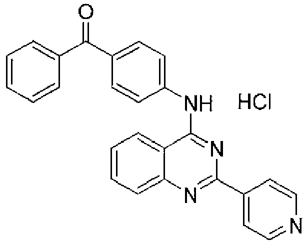

The reaction of 4-chloro-2-(pyridine-4-yl)quinazoline (200 mg, 0.83 mmol) with 4-aminobenzophenone (326 mg, 1.7 mmol) according to general procedure D gave the title compound (172 mg, 47 %) as white solid.
¹H NMR (500 MHz, DMSO-d6) δ 10.64 (bs), 9.01 (m, 2H), 8.84 (dt, *J*= 8.5 Hz, *J*= 1.0 Hz, 1H), 8.75 (m, 2H), 8.24 (m, 2H), 7.98-8.00 (m, 2H), 7.91 (m, 2H), 7.76-7.79 (m, 3H), 7.69 (m, 1H), 7.60 (m, 2H).
¹³C NMR (125 MHz, DMSO-d6) δ 194.90,158.44,155.01, 152.26, 149.91, 43.71, 143.49, 137.83, 134.51, 132.53, 131.94, 131.13, 129.64, 128.81, 128.69, 128.49, 124.68, 124.02, 121.59, 115.00.
HRMS: calculated for C₂₆H₁₉N₄O, 403.1559; found 403.1553

### (4-(4-chloroquinazolin-2-yl)phenyl)(phenyl)methanone

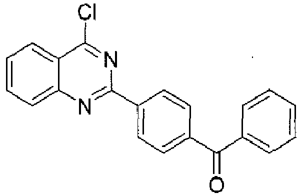

Reaction of 2-aminobenzamide (1.0 g, 7.3 mmol) with 4-benzoylbenzoyl chloride (1.79 g, 7.3 mmol) according to general procedure A gave the title compound (637 mg, 38 %) as white solid.
¹H NMR (400 MHz, CDCl₃) δ 8.78-8.64 (m, 2H), 8.29 (dd, *J*= 8.4 Hz, *J*= 0.8 Hz, 1H), 8.14 (d, *J*= 8.4, 1H), 8.01-7.97 (m, 1H), 7.97-7.93 (m, 2H), 7.88-7.83 (m, 2H), 7.72 (ddd, *J=* 8.2 Hz, *J*= 7.0 Hz, *J*=1.1 Hz, 1H), 7.65-7.59 (m, 1H), 7.52 (dd, *J*= 9.4 Hz, *J*= 5.7 Hz, 2H).
¹³C NMR (100 MHz, CDCl₃) δ 196.35, 162.73, 159.07, 151.80, 140.18,139.54, 137.50, 135.03, 132.60, 130.26, 130.08, 129.09, 128.78, 128.56, 128.35, 125.89, 122.69. HRMS: calculated for C₂₁H₁₃ClN₂O, 344.0716; found 344.0713.

### 2-fluoro-6-(2-(pyridin- 2-yl)quinazolin-4-ylamino)benzamide hydrochloride

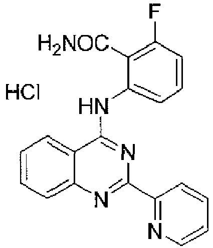

Reaction of 4-chloro-2-(pyridine-2-yl)quinazoline (100 mg, 0.41 mmol) with 2-amino-6-fluorobenzamide (63.2 mg, 0.41 mmol) according to general procedure D gave the title compound (78 mg, 48 %) as a brown hygroscopic solid.
¹H NMR (500 MHz, DMSO-d6) δ 12.32 (bs, 1H), 8.91 (ddd, *J* = 4.7 Hz, *J* = 1.7 Hz, *J* = 0.9 Hz, 1H), 8.84 (dd, *J* = 8.4 Hz, *J* = 1.2 Hz, 1H), 8.36 (dd, *J* = 8.6 Hz, *J* = 1.1 Hz, 1H), 8.28 (dt, *J* = 7.9 Hz, *J* 1.1 Hz, 1H), 8.14-8.18 (m, 3H), 7.91 (ddd, *J* = 8.4 Hz, *J* = 7.1 Hz, *J* = 1.1 Hz, 1H), 7.82 (ddd, *J*= 7.6 Hz, *J=* 4.8 Hz, *J=* 1.3 Hz, 1H), 7.80 (bs, 1H), 7.64-7.71 (m, 2H), 7.42 (ddd, *J* = 10.0 Hz, *J* = 8.2 Hz, *J* = 1.3 Hz, 1H).
¹³C NMR (125 MHz, DMSO-d6) δ 164.21, 160.68, 159.45 (d, *J*=248.2 Hz), 154.71, 149.49, 147.38, 140.01, 139.64, 136.94, 135.86 (d, *J*= 5.5 Hz), 131.62 (d, *J*= 5.5 Hz), 129.27, 128.65, 125.29, 124.86, 123.57, 121.83 (d, *J*=18.7 Hz), 121.24, 115.32 (d, *J*= 22.8 Hz), 113.28.
HRMS: calculated for C₂₀H₁₄FN₂ONa, 382.1080; found 382.1075.

### 4-chloro-2-(pyridin-3-yi)-7-(trifluoromethyl)quinazoline

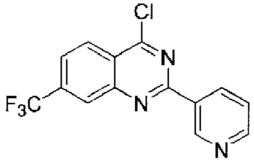

The reaction of 2-amino-4-(trifluoromethyl)benzamide (2.0 g, 9.8 mmol) with nicotinoyl chloride hydrochloride (1.75 g, 9.8 mmol) according to general procedure A gave the title compound (880 mg, 28 %) as white solid.
¹H NMR (500 MHz, DMSO-d6) δ 9.58 (dd, *J* = 2.2 Hz, *J* = 0.8 Hz, 1H), 8.79 (dd, *J*= 4.8 Hz, *J*= 1.7 Hz, 1H), 8.74 (ddd, *J* = 8.0 Hz, *J* = 2.2 Hz, *J* = 1.8 Hz, 1H), 8.49-8.51 (m, 2H), 8.12 (bdd, *J*= 8.8 Hz, *J*= 1.8 Hz, 1H), 7.64 (ddd, *J*= 8.0 Hz, *J* = 4.8 Hz, *J*= 0.9 Hz, 1H). ¹³C NMR (125 MHz, DMSO-d6) δ 162.94, 58.83, 152.52, 150.65, 149.60, 135.91, 135.33 (q, *J*= 32.8 Hz), 131.32, 128.22, 126.48 (q, *J*= 4.3 Hz), 125.00 (q, *J*= 3.1 Hz), 124.28, 124.14, 123.37 (q, *J*= 273.4 Hz).
HRMS: calculated for C₁₄H₇ClF₃N₃, 309.0281; found, 309.0279.

### N-cyclohexyl-2-(pyridin-3-yl)quinazolin-4-amine

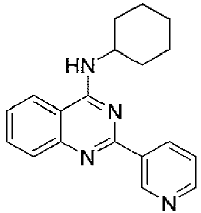

Reaction of 4-chloro-2-(pyridine-2-yl)quinazoline (50 mg, 0.21 mmol) with cyclohexylamine (21 mg mg, 0.21 mmol) according to general procedure D gave the title compound (10 mg, 16 %) as a white solid.
¹H NMR (500 MHz, DMSO-d6) δ 9.58 (dd, *J* = 2.2 Hz, *J* = 0.9 Hz, 1H), 8.72 (ddd, *J* = 8.0 Hz, *J*= 2.2 Hz, *J* = 1.7 Hz, 1H), 8.67 (dd, *J* = 4.7 Hz, *J* = 1.7 Hz, 1H), 8.38 (m, 1H), 8.07 (bd, *J* = 7.5 Hz, 1H), 7.75-7.78 (m, 2H), 7.53 (ddd, *J* = 8.0 Hz, *J* = 4.7 Hz, *J*= 0.9 Hz, 1H), 7.50 (m, 1H), 4.34 (m, 1H), 2.06 (m, 2H), 1.82 (m, 2H), 1.70 (m, 1H), 1.43-1.49 (m, 4H), 1.23 (m, 1H).
¹³C NMR (125 MHz, DMSO-d6) δ 159.11, 157.90, 150.88, 149.98, 149.36, 135.21, 134.20, 133.00, 127.97, 125.65, 123.71, 123.28, 114.18, 50.06, 32.19, 25.30, 23.96. HRMS: calculated for C₁₉H₂₁N₄, 305.1761; found, 305.1759.

### N-(2-fluoro-5-nitrophenyl)-2-(pyridin-3-yl)-7-(trifluoromethyl)quinazolin-4-amine

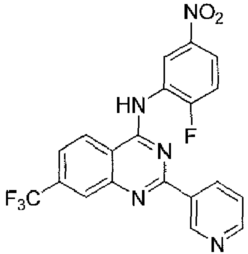

The reaction of 4-chloro-2-(pyridin-3-yl)-7-(trifluoromethyl)quinazoline (50 mg, 0.16 mmol) and 2-fluoro-5-nitroaniline (31 mg, 0.20 mmol) according to general procedure F gave the title compound (62 mg, 90 %) as a red-brown solid.
¹H NMR (500 MHz, DMSO-d6) δ 9.62 (bs, 1H), 9.21 (bdd, *J*= 6.6 Hz, *J*= 2.9 Hz, 1H), 8.76 (dt, *J*= 8.0 Hz, *J*=1.9 Hz, 1H), 8.72 (bd, *J* = 8.7 Hz, 1H), 8.70 (m, 1H), 8.24-8.27 (m, 2H), 7.89 (m, 1H), 7.64 (t, *J*=9.5 Hz, 1H), 7.49 (m, 1H).
¹³C NMR (125 MHz, DMSO-d6) δ 159.14-161.18 (m), 152.46, 151.60, 150.80, 145.16, 136.19, 135.30 (q, *J*= 32.8 Hz), 133-92,126.88 (q, *J*= 4.0 Hz), 125.47, 124.24, 122.69-122.99 (m), 117.63 (d, *J* = 23.1 Hz), 117.20.
HRMS calculated for C₂₀H₁₂F₄N₅O₂, 430.0922; found, 430.0920.

### 2-(pyridin-3-yl)-N-(3-(trifluoromethyl)phenyl)quinazolin-4-amine hydrochloride

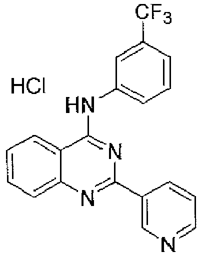

The reaction of 4-chloro-2-(pyridin-3-yl)quinazoline (50 mg, 0.21 mmol) and 3-(trifluoromethyl)aniline (40 mg, 0.25 mmol) according to general procedure D gave the title compound (26 mg, 31 %) as a pale yellow solid.
¹H NMR (500 MHz, DMSO-d6) δ 11.16 (bs, 1H), 9.53 (bd, *J*= 2.1 Hz, 1H), 9.03 (bd, *J*= 8.4 Hz, 1H), 8.98 (dm, *J =* 8.1 Hz, 1H), 8.87 (dd, *J* = 5.1 Hz, *J* = 1.5 Hz, 1H), 8.53 (m, 1H), 8.32 (dm, *J* = 8.1 Hz, 1H), 8.09 (m, 1H), 7.99 (m, 1H), 7.84 (bdd, *J* = 8.1 Hz, *J*= 5.2 Hz, 1H), 7.70-7.75 (m, 2H), 7.56 (dm, *J*= 7.9 Hz, 1H).
¹³C NMR (125 MHz, DMSO-d6) δ 158.69, 156.05, 148.4, 148.4, 146.4, 139.72, 139.0, 134.67, 133.9, 129.93, 129.38 (q, *J* = 31.5 Hz), 127.59, 126.81, 125.33, 124.51, 124.49 (q, *J* = 272.7 Hz), 120.82, 119.60, 114.34.
HRMS: calculated for C₂₀H₁₄F₃N₄, 367.11651; found, 367.11648.

### 2-(pyridin-3-yl)-N-(3,4,5-trifluorophenyl)quinazolin-4-amine hydrochloride

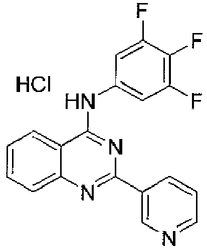

The reaction of 4-chloro-2-(pyridin-3-yl)quinazoline (50 mg, 0.21 mmol) and 3,4,5-trifluoroaniline (37 mg, 0.25 mmol) according to general procedure D gave the title compound (63 mg, 77 %) as a pale yellow solid.
¹H NMR (500 MHz, DMSO-d6) δ 11.23 (bs, 1H), 9.52 (bd, *J*= 2.1 Hz, 1H), 9.11 (dt, *J*= 8.1 Hz, *J* = 1.8 Hz, 1H), 9.07 (bd, *J* = 8.4 Hz, 1H), 8.96 (dd, *J* = 5.3 Hz, *J* = 1.6 Hz, 1H), 8.11 (m, 2H), 8.02 (dd, *J*= 8.1 Hz, *J*= 5.3 Hz, 1H), 7.99 (m, 1H), 7.72 (m, 1H).
¹³C NMR (125 MHz, DMSO-d6) δ 158.63, 155.40, 149.99 (ddd, *J*= 244.1 Hz, *J*=10.1 Hz, *J*= 5.5 Hz), 148.7, 146.3, 144.30, 141.00, 134.64-136.47 (m), 134.76, 127.79, 127.20, 126.30, 124.65, 114.38, 107.58 (m).
HRMS: calculated for C₁₉H₁₂F₃N₄, 353.10086; found, 353.10082.

### N-(2-acetylphenyl)nicotinamide

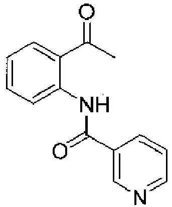

To a solution of 2-aminoacetophenone (5.0 g, 37 mmol) in dichloromethane (50 ml) at 0 °C was sequentially added nicotinoyl chloride hydrochloride (7.24 g, 40.7 mmol), DMAP (0.4 g) and pyridine (6.4 g, 81 mmol). The mixture was allowed to stir at 20 °C overnight. The reaction was quenched by addidion of water and crude product was extracted with CHCl₃. Combined organic layers were washed with water, brine and dried. Chromatography (hexane-EtOAc 1:1) gave the title compound 6.77 g (76%).
m.p. 116.7-118.3 °C.
¹H NMR (400 MHz, CDCl₃) δ 12.85 (s), 9.34 (d, *J*= 1.8, 1H), 8.97 (d, *J*= 7.5, 1H), 8.82 (dd, *J* = 4.8, 1.6, 1H), 8.45 - 8.24 (m, 1H), 8.01 (dd, *J*= 8.0, 1.5, 1H), 7.75 - 7.57 (m, 1H), 7.49 (dd, *J*= 8.0, 4.8, 1H), 7.27 - 7.18 (m, 1H), 2.76 (s, 3H).
¹³C NMR (101 MHz, CDCl₃) δ 203.38, 164.24, 152.60, 149.15, 140.98, 135.43, 134.87, 131.84, 130.39, 123.45, 122.97, 122.06, 120.90, 2$.51.
Jones, Carrie P.; Anderson, Kevin W.; Buchwald, Stephen L. Journal of Organic Chemistry, 2007, vol. 72, # 21 p. 7968 - 7973

### 2-(pyridin-3-yl)quinolin-4(1H)-one

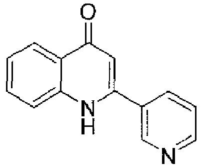

Suspension of *N*-(2-acetylphenyl)nicotinamide (4.4 g, 18.31 mmol) and NaOH (2.2 g, 55 mmol) in dioxane (70 ml) was refluxed for 4h and then allowed to cool down. Resulting precipitate was filtered off and solid was washed with hexane then water. The crude product was dried in vacuo to afford 4.0 g (98%) of title compound.
¹H NMR (400 MHz, DMSO) δ 11.56 (bs, 1H), 9.05 (s, 1H), 8.77 (d, *J =* 3.7, 1H), 8.26 (d, *J* = 7.9, 1H), 8.12 (d, *J* = 7.9, 1H), 7.76 (d, *J* = 8.2, 1H), 7.70 (t, *J* = 7.6, 1H), 7.62 (dd, *J* = 7.9, 4.8, 1H), 7.37 (t, *J*= 7.4, 1H), 6.44 (bs, 1H).
Jones, Carrie P.; Anderson, Kevin W.; Buchwald, Stephen L. Journal of Organic Chemistry, 2007 , vol. 72, # 21 p. 7968 - 7973

### 4-chloro-2-(pyridin-3'-yl)quinoline

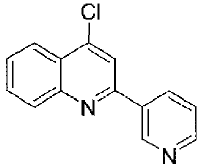

To a solution of 2-(pyridin-3-yl)quinolin-4(1*H*)-one (4.0 g, 18.0 mmol) in DMF (15 ml) was added POCl₃ (13.81 g, 90 mmol) under Ar. The reaction mixture was stirred at 80 °C for 3h and then poured into ice. Neutralization with 20 % KOH solution to pH = 9, extraction with CHCl₃ and evaporation of solvent lead to crude product. Chromatography (hexane-EtOAc 1:1) of the residue gave the title compound (3.21 g, 74%).
¹H NMR (400 MHz, CDCl₃) δ 9.37 (d, *J*= 1.7, 1H), 8.75 (dd, *J*= 4.8, 1.6, 1H), 8.57 - 8.46 (m, 1H), 8.28 (d, *J* = 8.4, 1H), 8.22 (d, *J* = 8.4, 1H), 8.01 (s, 1H), 7.89 - 7.76 (m, 1H), 7.74- 7.63 (m, 1H), 7.50 (dd, *J*= 8.0, 4.8, 1H).
¹³C NMR (101 MHz, CDCl₃) δ 154.57, 150.65, 149.13, 148.75, 143.60, 134.84, 134.12, 130.87, 130.13, 127.74, 125.53, 124.03, 123.67, 118.63.

### 2-(pyridin-3-yl)-N-(3,4,5-trifluorophenyl)quinolin-4-amine

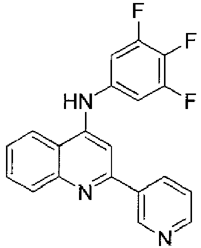

The reaction of 4-chloro-2-(pyridin-3-yl)quinoline (50 mg, 0.21 mmol) and and 3,4,5-trifluoroaniline (37 mg, 0.25 mmol) according to general procedure F gave the title compound (20 mg, 27 %) as a yellow solid.
¹H NMR (500 MHz, DMSO-d6) δ 9.28 (bs, 1H), 9.25 (bs, 1H), 8.65 (m, 1H), 8.46 (ddd, *J* = 8.0 Hz, *J*= 2.3 Hz, *J*= 1.7 Hz, 1H), 8.32 (dm, *J*= 8.5 Hz, 1H), 8.02 (dm, *J*= 8.5 Hz, 1H), 7.78 (m, 1H), 7.65 (s, 1H), 7.60 (m, 1H), 7.53 (ddd, *J*= 8.0 Hz, *J*= 4.8 Hz, *J*= 0.9 Hz, 1H), 7.37 (dd, *J*= 9.7 Hz, *J*= 6.3 Hz, 2H).
¹³C NMR (125 MHz, DMSO-d6) δ 154.81, 151.06 (ddd, *J=* 245.2 Hz, *J=* 10.2 Hz, *J* 5.7 Hz), 150.24, 149.03, 148.59, 147.85, 137.75 (m), 134.07-136.13 (m), 134.97, 134.86, 130.38,129.77, 125.66,123.91,122.19, 119.58, 106.12 (m), 100.83.
HRMS: calculated for C₂₀H₁₂F₃N₃, 350.0905; found, 350.0897.

### Methyl 2-amino-5-hydroxybenzoate

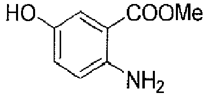

To a solution of 2-amino-5-hydroxybenzoic acid (1.0 g, 6.6 mmol) in methanol is dropwise added SOCl₂ (3.75 g, 33.0 mmol). The reaction mixture was stirred with reflux overnight. The volatiles and solvents were removed in vacuo to give the title compound (915mg, 83%).
¹H NMR (400 MHz, DMSO) δ 8.66 (s, 1H), 7.10 (d, *J*= 2.9 Hz, 1H), 6.80 (dd, *J*= 8.8 Hz and 2.9 Hz, 1H), 6.64 (d, *J*= 8.8 Hz, 1H), 6.07 (s, 2H), 3.76 (s, 3H).
¹³C NMR (101 MHz, DMSO) δ 167.52, 146.53, 144.59, 123.41, 117.75, 114.32, 108.81, 51.16.
HRMS: calculated for C₈H₉NO₃, 167.0582; found, 167.0583.

### 4-chloro-2-(pyridin-3-yl)quinazolin-6-ol

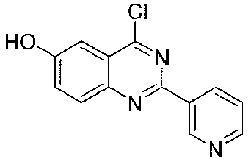

The reaction of methyl 2-amino-5-hydroxybenzoate (915 mg, 5.5 mmol)) and 3-cyanopyridine (570 mg, 5.5 mmol) according to general procedure B gave the title compound (450 mg, 32 % overall).
¹H NMR (400 MHz, DMSO) δ 9.52 (d, *J* = 1.7 Hz, 1H), 8.71 (dd, *J* = 4.7 Hz, *J* = 1.5 Hz, 1H), 8.70 - 8.65 (m, 1H), 8.03 (d, *J* = 9.1 Hz, 1H), 7.67 (dd, *J* = 9.1 Hz, *J* = 2.7 Hz, 1H), 7.58 (dd, *J* = 8.0 Hz, *J* = 4.8 Hz, 1H), 7.45 (d, *J* = 2.6 Hz, 1H).
¹³C NMR (101 MHz, DMSO) δ 159.53, 158,12, 154.32, 151,11, 148.64, 145.86, 134.81 , 131.81, 130.39, 128.30, 123.76, 123.32, 105.76.
HRMS: calculated for C₁₃H₉NO₃Cl, 258.04287; found, 258.04280.

### 2-(pyridin-3-yl)-4-(3,4,5-trifluorophenylamino)quinazolin-6-ol hydrochloride

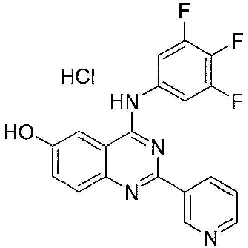

The reaction of 4-chloro-2-(pyridin-3-yl)quinazolin-6-ol (50 mg, 0.19 mmol) and 3,4,5-trifluoroaniline (30 mg, 0.20 mmol) according to general procedure D gave the title compound (12 mg, 15 %) as a beige solid.
¹H NMR (500 MHz, DMSO-d6) δ 10.46 (bs, 1H), 10.03 (bs, 1H), 9.48 (bd, *J* = 2.1 Hz, 1H), 8.66 (dd, *J* = 4.7 Hz, *J* = 1.6 Hz, 1H), 8.62 (td, *J* = 7.9 Hz, *J* = 1.9 Hz, 1H), 8.05 (m, 2H), 7.88 (d, *J* = 2.6 Hz, 1H), 7.80 (d, *J* = 9.0 Hz, 1H), 7.58 (dd, *J* = 9.0 Hz, *J* =2.6 Hz, 1H), 7.55 (bdd, *J* = 8.0 Hz, *J* = 4.7 Hz, 1H).
¹³C NMR (125 MHz, DMSO-d6) δ 156.90, 156.56, 154.44, 150.71, 150.01 (ddd, *J* = 242.9 Hz, 9.2 Hz, *J* = 5.3 Hz), 148.99,144.72, 136.31 (m), 134.78, 134.66 (dt, *J* = 244.5 Hz, *J* = 15.8 Hz), 133.94, 12992, 125.21, 123.86, 115.47, 105.94 (m), 105.55.
HRMS: calculated for C₁₃H₁₀N₄O₃F₃, 367.08122; found, 367.08147.

### N-(2-(pyridin-3-yl)quinazolin-4-yl)thiazol- 2-amine hydrochloride

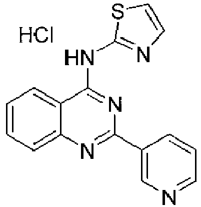

The reaction of 4-chloro-2(pyridine-3-yl)quinazoline (50 mg, 0.21 mmol) and thiazol-2-amine (25 mg, 0.25 mmol) according to general procedure E followed by treatment of resulting free base with 3 M HCl gave the title compound (17 mg, 26 %).
¹H NMR (500 MHz, DMSO-d6) δ 9.47 (dd, *J* = 2.2 Hz, *J* = 0.7 Hz, 1H), 9.50 (bs, 1H), 9.29 (dm, *J* = 8.1 Hz, 1H), 9.02 (dd, *J* = 5.3 Hz, *J* = 1.5 Hz, 1H), 8.64 (bd, *J* = 8.2 Hz, 1H), 8.15 (dm, *J* = 8.1 Hz, 1H), 8.04 (dd, *J* = 8.1 Hz, *J* = 5.3 Hz, 1H), 8.00 (m, 1H), 7.74 (m, 1H), 7.73 (d, *J* = 4.1 Hz, 1H), 7.48 (d, *J* = 4.1 Hz, 1H).
¹³C NMR (125 MHz, DMSO-d6) δ 163.7,159.24, 155.20,147.9,145.8,145.2,141.13, 134.99, 133.1, 130.7, 128.27, 126.05, 124.84, 124.1, 116.92, 115.07.
HRMS: calculated for C₁₆H₁₂N₅S, 308.08079; found, 308.08074.

### 4-ethoxy-2-(pyridin-3-yl)quinazoline

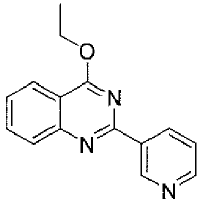

To a solution of 4-chloro-2(pyridine-3-yl)quinazoline (50 mg, 0.21 mmol) in dry ethanol (5 ml) was added sodium *tert*-butoxide (56 mg, 59 mmol) and the reaction mixture was stirred at RT overnight. The solvents were evaporated and chromatography (CHCl₃ with gradient of methanol) gave the title compound (36 mg, 68 %).
¹H NMR (500 MHz, DMSO-d6) δ 9.63 (bdd, *J* = 2.2 Hz, *J* = 0.9 Hz, 1H), 8.77 (ddd, *J* = 8.0 Hz, *J* = 2.2 Hz, *J* =1.8 Hz, 1H), 8.73 (dd, *J* = 4.8 Hz, *J* = 1.7 Hz, 1H), 8.16 (m, 1H), 7.95-7.99 (m, 2H), 7.58 (ddd, *J* = 7.9 Hz, *J* = 4.8 Hz, *J* = 0.9 Hz, 1H), 4.76 (q, *J* = 7.1 Hz, 2H), 1.52 (t, *J* = 7.1 Hz, 3H).
¹³C NMR (125 MHz, DMSO-d6) δ 166.59, 157.61, 151.55, 151.16, 149.46, 135.48, 134.59, 133.04, 127.86, 123.92, 123.53, 114.99, 63.33, 14.37.
HRMS: calculated for C₁₅H₁₃ON₃, 251.1059; found, 251.1060.

### 4-(dimethylamino)-2-(pyridin-3-yl)quinazolin-6-ol hydrochloride

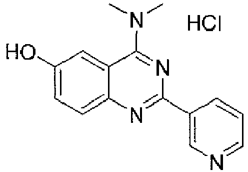

To a solution of 4-chloro-2-(pyridin-3-yl)quinazolin-6-ol (400 mg, 1.52 mmol) in acetone was added diethylamine (400 mg, 5.6 mmol) and the reaction mixture was stirred at RT overnight. Solvents were evaporated and free base was treated with excess of HCl (3 M) to give its salt. Crude product was dried in vacuo and chromatography of residue (CHCl₃ with gradient of MeOH) gave the title compound (335 mg, 73 %).
¹H NMR (500 MHz, DMSO-d6) δ 10.09 (bs, 1H), 9.56 (bd, *J* =1.9 Hz, 1H), 8.69 (dt, *J* = 7.9 Hz, *J* = 2.0 Hz, 1H), 8.64 (dd, *J* = 4.7 Hz, *J* =1.7 Hz, 1H), 7.47 (d, *J* = 9.0 Hz, 1H), 7.50 (ddd, *J* = 7.9 Hz, J = 4.7 Hz, *J* = 0.9 Hz, 1H), 7.46 (d, *J* = 2.6 Hz, 1H), 7.34 (dd, *J* = 8.9 Hz, *J* = 2.6 Hz, 1H), 3.35 (s, 6H).
¹³C NMR (125 MHz, DMSO-d6) δ 162.67, 154.59, 154.15, 150.47, 149.09, 146.26, 134.88, 134.09, 129.88, 123.99, 123.66, 115.88, 108.18, 41.55.
HRMS: calculated for C₁₅H₁₅ON₄, 257.12404; found, 257. 12383.

### Sodium 4-(dimethylamino)-2-(pyridin-3-yl)quinazolin-6-yl sulfate

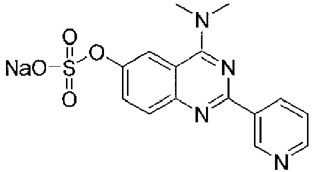

Pyridine (2 ml) was cooled to -20° C and chlorosulfonic acid (220 mg, 1.9 mmol) was slowly added. Resulting solution was cooled to -20° C again and 4-(dimethylamino)-2-(pyridin-3-yl)quinazolin-6-ol hydrochloride (300 mg, 1.2 mmol) in pyridine was added in one portion. The reaction mixture was stirred at RT overnight then the excess of pyridine vas evaporated and residue was neutralized with aqueous Na₂CO₃ to pH = 10. Evaporation of solvents and extraction of solid phase with hot acetone followed by chromatography (CHCl₃ with gradient of MeOH) gave the title compound (152 mg, 34 %).
¹H NMR (500 MHz, DMSO-d6) δ 9.59 (dd, *J* = 2.2 Hz, *J* = 0.9 Hz, 1H), 8.73 (ddd, *J* = 7.9 Hz, *J* = 2.2 Hz, *J* = 1.8 Hz, 1H), 8.66 (dd, *J* = 4.8 Hz, *J* = 1.7 Hz, 1H), 8.03 (d, *J* = 2.6 Hz, 1H), 7.79 (d, *J* =9.0 Hz, 1H), 7.61 (dd, *J* = 9.0 Hz, *J* = 2.5 Hz, 1H), 7.53 (ddd, *J* = 7.9 Hz, *J* = 4.7 Hz, *J* = 0.9 Hz, 1H), 3.40 (s, 6H).
¹³C NMR (125 MHz, DMSO-d6) δ 162.91,155.56, 150.77, 150.10, 149.29, 148.54, 135.17, 133.95, 128.85, 127.47, 123.72, 115.45, 114.79, 41.57.
HRMS: calculated for C₁₅H₁₅O₄N₄S, 347.08085; found, 347.08086.

### Further compounds synthesized

Further synthesized compounds are shown in table 4 hereinunder. These compounds were also synthesized following the general procedures A to F, described above.

### III. Tissue Culture Tests

The compounds synthesized were tested in ALPHA screen and in YFP screen on tissue cultures.

### III.1: General Procedure of ALPHA screen

The AlphaScreen experiments are performed using Perkin Elmer Enspire plate reader in white 96-well ProxiPlates and PBS supplemented with 0.05% Tween20, 2mM DTT, 2% DMSO. First, Streptavidin-coated Donor beads are used to capture the CAI-biotin peptide. In the other solution, a fusion protein of CA with glutathione-S-transferase (CA-GST) is mixed with GSH Acceptor beads. Both mixtures were incubated and then mixed together. To test the ability of prepared organic compounds to inhibit the CAI binding to CA, they are screened at concentrations between 0.5-100 µM and IC₅₀ values are calculated from the influenced signals.

### Principle of the assay

Upon illumination by laser at 680 nm, Donor beads containing the photosensitizer phthalocyanine convert ambient oxygen to an excited form of singlet oxygen ¹O_{2.} Singlet oxygen can within its half-life of ~ 4 µs diffuse approximately 200 nm in solution. If an Acceptor bead is within that proximity, which is mostly upon CA:CAI interaction, energy is transferred from the singlet oxygen to thioxene derivates within the Acceptor and signal at 520 - 620 nm is emitted. In the absence of an Acceptor bead, i.e. a compound inhibits CA/CAI interaction, singlet oxygen falls to ground state and no signal is produced.

### III.2: General Procedure of YFP screen

For YFP-screen were used cells transfected with the YFP expressing variant of NL4.3 HIV-1. Briefly, HEK293T cells were cotransfected with pNL4.3ΔLTRΔNef and YFP-pNL4.3ΔLTRΔNef in 1 : 1 ratio by FuGENE6 method according the manufacturer instructions. Transfected cells were immediately seeded on 96-well plate by Multidrop in the density of 3 x 10⁴ cells per well in the volume of 100 µl. Compounds from parental plate (5 µl of 2,7 or 6,75 µM compound in 100 % DMSO) were diluted by addition of 95 µl of complete DMEM (Dulbecco's modified Eagle medium containing 10 % fetal calf sera and peniciline/streptorriycine combination) by Hydra™. Compounds were added to transfected cells 10 hrs post-transfection. Hydra™ transferred 8 µl of diluted compounds from parental plate, so the final concentration of the compounds in the cell media was 10 or 25 µM. Supernatant was harvested again by Hydra™ 24 hrs later and the YFP intensities in supernatant and in cells were measured on the Saphire fluorescence reader.

Several controls were distributed randomly on the plate and on the plate edges. The controls include nontransfected cells, late(-) transfected cells (HIV-1 variant with impaired release), pEYFP transfected cells and cells cotransfected with pNL4.3ΔLTRΔNef and YFP-pNL4.3ΔLTRΔNef treated with 1% DMSO.

Obtained YFP intensities were normalized and evaluated according to stringent and less stringent criteria as described further. Nontransfected cells and corresponding supernatants were taken as a background values and subtracted from all other values. DMSO treated cotransfected cells and corresponding supernatants were averaged and this value was used as the max value representing 100 % effectivity of virus release. The stringent criteria were set as follows: supernatant value must be lower than 75 % of DMSO control and concomitantly cell associated YFP intensity cannot be lower than 80 % of DMSO control. Only 7 compounds of 218 tested were evaluated as hits at three of four plates according to these stringent criteria (table 5).

Less stringent criteria involved the following normalization: norm=sup_{bcgrd}/(sup_{bcgrd}+cell_{bcgrd}) and then related to DMSO control. As hits were considered compounds that have norm value lower than 75 % of that of the DMSO control. These less stringent criteria were fulfilled by 5 additional compounds that were evaluated as hits at three of four plates according to the stringent or less stringent criteria (table 5).

Hits selected in the YFP-screen were further evaluated in the tissue culture experiments with cells infected with the wild type NL4.3 HIV-1. The virus for infection was obtained from co-culture of MT4 cells. The MT4 cells co-culture was synchronized for one week and then used for the infection of fresh MT 4 cells. 48 hrs post-infection was the culture supernatant harvested, cleared by the filtration through 45 µm filter and stored in -80 °C in 1 ml aliquots. Amount of virus was quantified by enzyme-linked immuno sorbent assay (ELISA) with antibodies against HIV-1 CA (3 ng of CA/ml) and blue cell assay (1x10⁵ IU/ml), which gives 3 x 10⁴ IU/ng of CA.

### III.3: TZM titration

For compounds testing the TZM cells were seeded on 96-well plate in the density 5 x 10³ cells per well. The culture media was aspirated approx. 16 hrs later and cells were infected by the wild type NLA.3 HIV-1 by addition of 50J..il of prepared virus stock diluted with 50 µl of complete DMEM. The compounds were added twice to ensure that there are not any preassembled viruses present at the time of their addition. Compounds were first added 8 hrs post-infection and then 24 hrs later. In both cases the old culture media were replaced by 100 µl of fresh complete DMEM prior to the compounds addition. Different compounds concentrations were tested, but the DMSO concentration never exceeded 1 % DMSO. As controls were used cells treated only with 1 % DMSO and cells treated with 0,1 µM Lopinavir (HIV-1 protease inhibitor). Supernatant was harvested 24 hrs after the second compound addition, i.e. 56 hrs post-infection. Harvested supernatants were immediately titrated on the fresh TZM cells in aim to perform the infectivity assay (TZM titration), or inactivated by addition of Triton X-100 to the final concentration 0.025 % for further ELISA analysis.

TZM titration was performed on the cells seeded day before on 96-well plate in the density 5 x 10³ cells per well. The analysis was performed for each compound concentration at three different dilutions and in duplicates. The culture media was aspired and cells were lysed 48 hrs post-infection by addition of 100 µl of SteadyGlo luciferase substrate (Promega) diluted 1 : 1 by complete DMEM. The luminescence signal was measured on Luminoskan Ascent (Thermo Labsystems) after the transfer of 80 µl of lysate to the flat bottom white polystyrene assay plates (Costar).

For ELISA measurement, the Maxisorp plates (Nunc) were precoated with monoclonal mouse anti HIV-1 CA antibodies at RT over night and blocked with 10 % FCS in PBS for at least 2 hrs at 37 °C prior the loading of inactivated supernatant. The analysis was done in three different dilutions and in duplicates for each compound concentration. For detection were used polyclonal rabbit anti HIV-1 CA antibodies and secondary antibodies labeled with horseradish peroxidase. The product of colorimetric reaction was quantified by measuring of absorbance at 450 nm at Multiscan EX reader (Thermo Scientific).

Cytotoxicity of selected hits tested in the assay with NL4.3 infected cells was determined by MTT test. TZM cells were seeded on the previous day on 96-well plate in the density 5 x 10³ cells per well. The tested compounds were added in different concentrations, but the DMSO concentration never exceeded 1 % DMSO. As controls were used cells treated only with 1 % DMSO (non-toxic concentration) and cells treated with 5 % DMSO (toxic concentration). 20 µl of the CellTiter 96 Aqueous One substrate (Promega) was added to the 100 µl of culture media 48 hrs after the compounds addition and the color development was quantified 4 hrs later by measuring the absorbance at 490 nm on Saphire reader. Compounds were consider non-cytotoxic (at given concentrations) when the measured values were higher than 80 % of the value of 1 % DMSO treated cells.

### IV. Determination of the binding of tested compounds to the target :isothermal titration calorimetry (ITC)

The binding of CA ligand AMR-346 to the HIV capsid protein and its C-terminal part was monitared using a VP-ITC microcalorimeter (MicroCal Inc., Northampton, USA) at 25 °C. The solutions of reactants were prepared in 30 mM MES, pH 6.0 containing 10 % glycerol, 2 mM 2-mercaptoethanol, 2 % DMSO, and the exact concentrations of proteins were determined by amino acid analysis. Due to low solubility of AMR-346, the precipitation was observed at concentration required for ITC measurement. The isothermal titration was used to estimate the ligand concentration and the binding parameters were calculated for situation when single AMR-346 molecule binds to monomer or dimer of protein, respectively. Typically, 9 µl aliquots of AMR-346 were injected stepwise into the sample cell containing 1.43 ml of 7 µM protein until saturation was achieved. The experiment was accompanied by a corresponding control experiment in which AMR-346 was injected into buffer alone. The association constants were determined by software developed by MicroCal.

Figure 1 shows the calorimetric titration curves from which the parameters for binding of one ligand molecule to monomeric proteins were calculated. The CA ligand AMR-346 binds to monomer of HIV capsid protein or its C-terminal part with dissociation constants of 230 nM or 50 nM, respectively. For the binding mode of single molecule AMR-346 toward dimeric capsid protein and its C-terminal part, the dissociation constants were estimated to be 120 nM and 26 nM, respectively.

**Table 4: Synthesized and tested compounds**

| | | | α screen | tissue culture | toxicity |
|---|---|---|---|---|---|
| compound code | compound structure | mol. Weight | IC50 (µM) | IC50 (µM) | LC50 (µM) |
| AMR.320 | 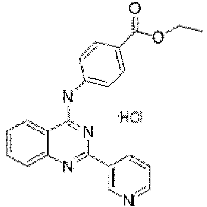 | 406,1 | 14,2 | | |
| AMR.322 | 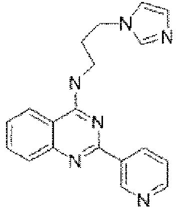 | 330,2 | > 200 | | |
| AMR.325 | 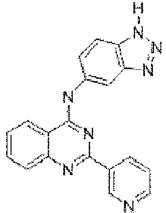 | 339,1 | > 200 | | |
| AMR.328 | 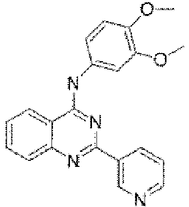 | 358,1 | 110,2 | | |
| AMR.328 .HCl | 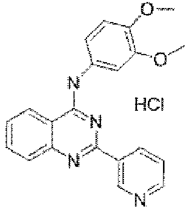 | 394,1 | > 200 | | |
| AMR.331 | 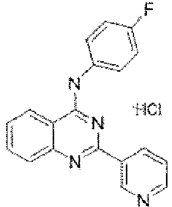 | 352,1 | 44,4 | | |
| AMR.334 | 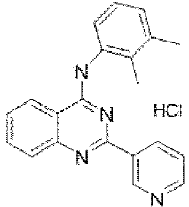 | 362,1 | 43,2 | | |
| AMR.335 | 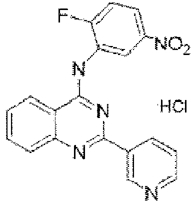 | 397,1 | 6,5 | | |
| AMR.336 | 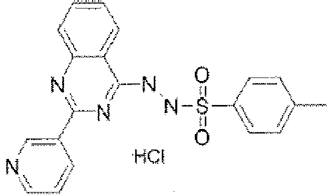 | 427,1 | 118,6 | | |
| AMR.341 | 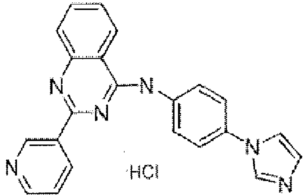 | 400,1 | 31,5 | | |
| AMR.345 | 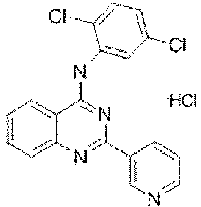 | 402,0 | 22 | | |
| AMR.346 | 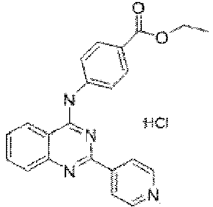 | 406,1 | 7,2 | ~15 | non-toxic |
| AMR.347 | 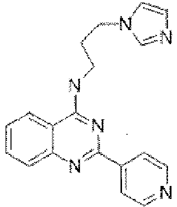 | 330,2 | > 200 | | |
| AMR.347 .HCl | 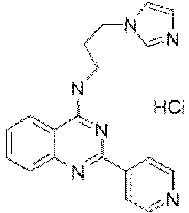 | 366,1 | 152,8 | | |
| AMR.347 .2HCl | 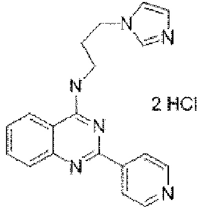 | 402,1 | > 200 | | |
| AMR.348 | 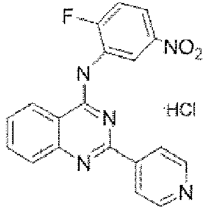 | 397,1 | 7,2 | | |
| AMR.350 | 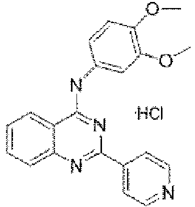 | 394,1 | > 200 | | |
| AMR.351 | 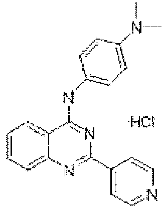 | 377,1 | 15,4 | | |
| AMR.352 | 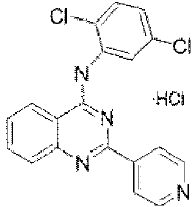 | 402,0 | 190,7 | | |
| AMR.353 | 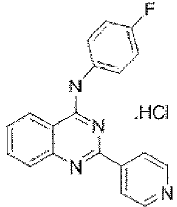 | 352,1 | > 200 | | |
| AMR.354 | 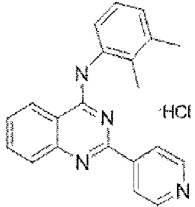 | 362,1 | 165,1 | | |
| AMR.355 | 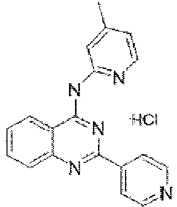 | 349,1 | 25 | | |
| AMR.356 | 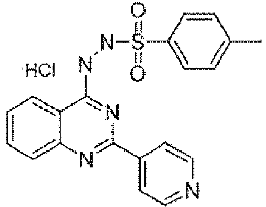 | 427,1 | 155,8 | | |
| AMR.357 | 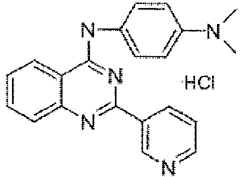 | 377,1 | 66,7 | | |
| AMR.358 | 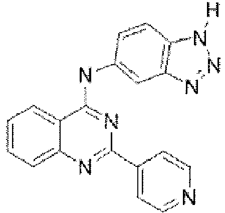 | 339,1 | 5 | | |
| AMR.359 | 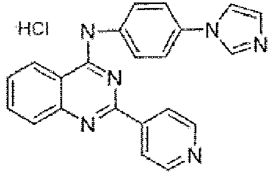 | 400,1 | 37,5 | | |
| AMR.361 | 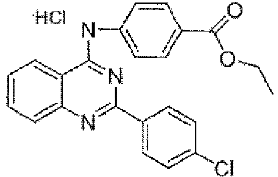 | 439,1 | >200 | | |
| AMR.362 | 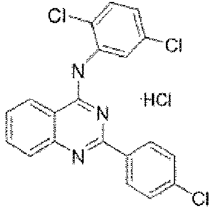 | 435,0 | > 200 | | |
| AMR.363 | 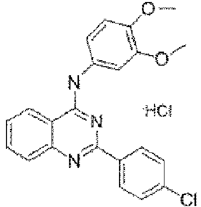 | 427,1 | 54,8 | | |
| AMR.364 | 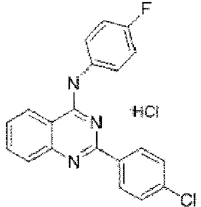 | 385,1 | > 200 | | |
| AMR.365 | 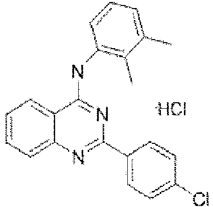 | 395,1 | > 200 | | |
| AMR.366 | 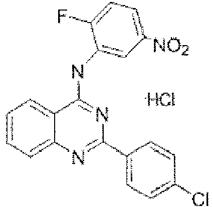 | 430,0 | > 200 | | |
| AMR.367 | 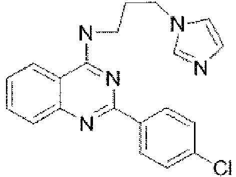 | 363,1 | > 200 | | |
| AMR.368 | 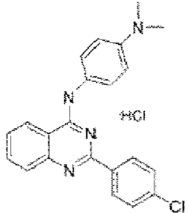 | 410,1 | 33,7 | | |
| AMR.370 | 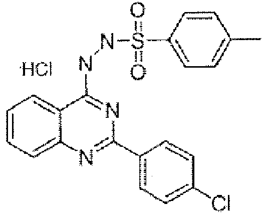 | 460,1 | > 200 | | |
| AMR.3 71 | 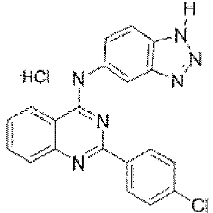 | 408,1 | 5 | | |
| AMR.372 | 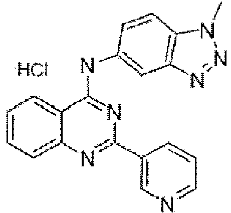 | 389,1 | 6,4 | | |
| AMR.373 | 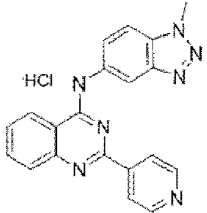 | 389,1 | 17,3 | | |
| AMR.374 | 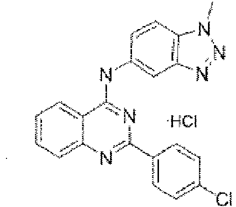 | 422,1 | 6,6 | | |
| AMR.378 | 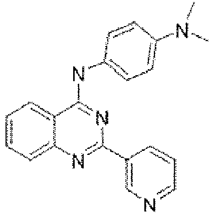 | 341,2 | > 200 | | |
| AMR.378 .HCl | 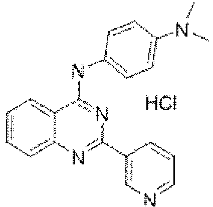 | 377,1 | 24,7 | | |
| AMR.379 | 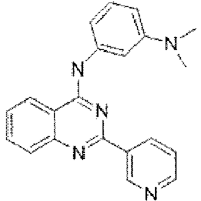 | 341,2 | 217,3 | | |
| AMR.379+HCl | 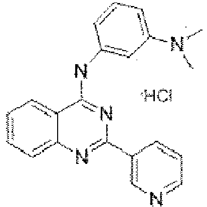 | 377,1 | 30,1 | | |
| AMR.380 | 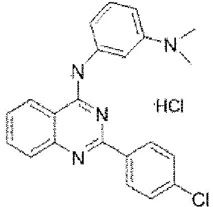 | 410,1 | 157,2 | | |
| AMR.381 | 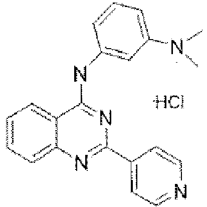 | 377,1 | 130,1 | | |
| AMR.390 | 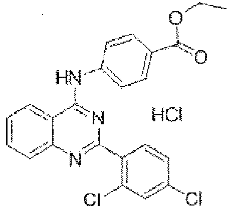 | 473,0 | > 200 | | |
| AMR.391 | 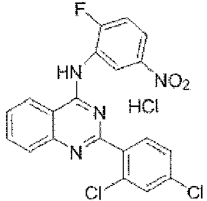 | 464,0 | > 200 | | |
| AMR.392 | 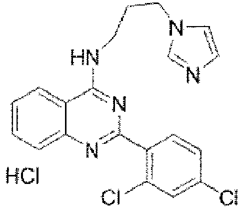 | 433,1 | > 200 | | |
| AMR.393 | 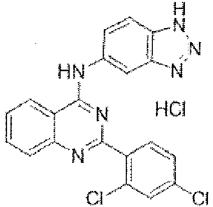 | 442,0 | 2 | | |
| AMR.395 | 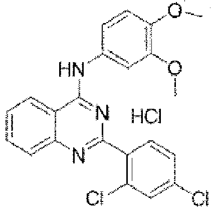 | 491,0 | 120,9 | | |
| AMR.396 | 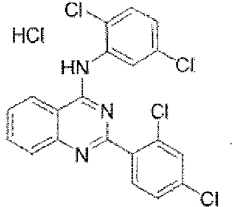 | 468,9 | > 200 | | |
| AMR.397 | 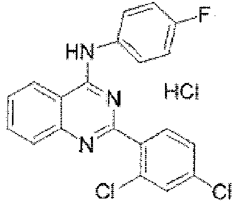 | 419,0 | > 200 | | |
| AMR.400 | 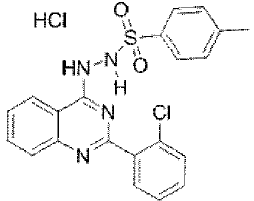 | 460,1 | > 200 | | |
| AMR.401 | 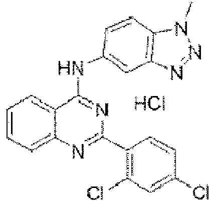 | 456,0 | 41,8 | | |
| AMR.402 | 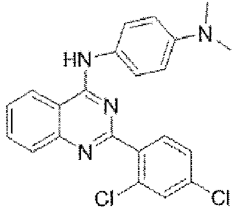 | 408,1 | 26,7 | | |
| AMR.402+HCl | 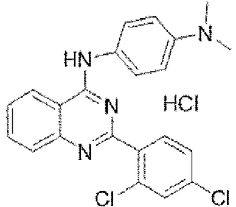 | 444,1 | 28,6 | | |
| AMR.403 | 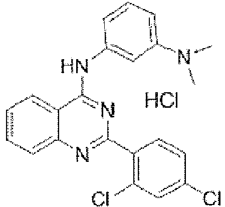 | 444,1 | 41,1 | | |
| AMR.416 | 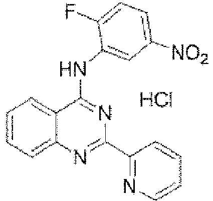 | 387,1 | 2,3 | | |
| AMR.421 | 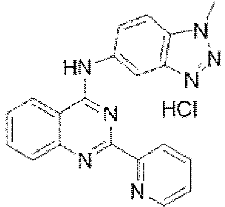 | 389,1 | 33,7 | | |
| AMR.422 | 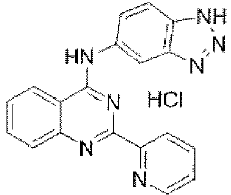 | 375,1 | 3,2 | | |
| AMR.424 | 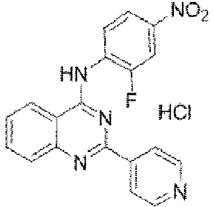 | 397,1 | 1,9 | | |
| AMR.425 | 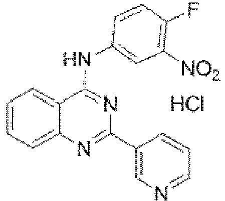 | 397,1 | 7 | | non toxic |
| AMR.426 | 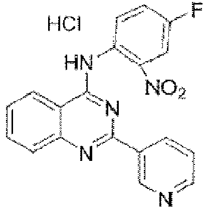 | 397,1 | 14,5 | | |
| AMR.427 | 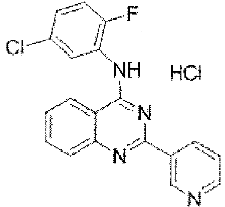 | 386,1 | 16,5 | | |
| AMR.428 | 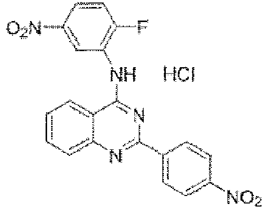 | 441,1 | 114,7 | | |
| AMR.429 | 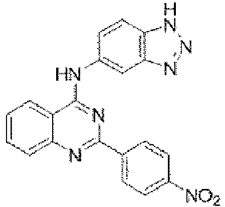 | 383,1 | 2,4 | | |
| AMR.437 | 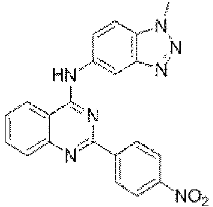 | 397,1 | 50,9 | | |
| AMR.444 | 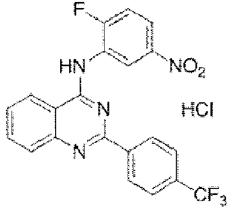 | 464,1 | > 200 | | |
| AMR.445 | 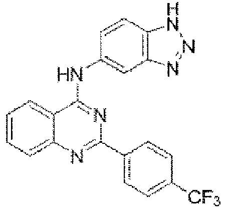 | 406,1 | 53,2 | | |
| AMR.446 | 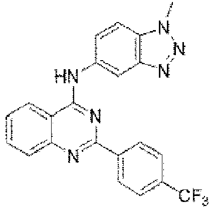 | 420,1 | 28,2 | | |
| AMR.448 | 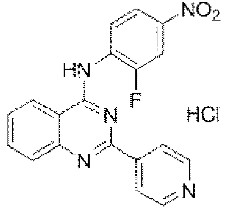 | 397,1 | 2,6 | | |
| AMR.448H | 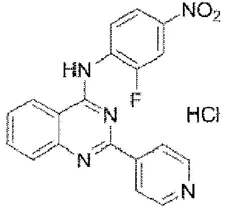 | 379,1 | 1,8 | | |
| AMR.449 | 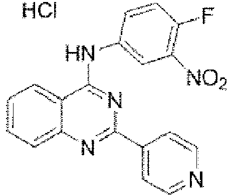 | 397,1 | 2,9 | | |
| AMR.450 | 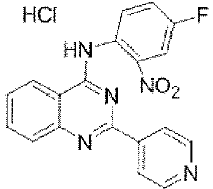 | 397,1 | 169,1 | | |
| AMR.451 | 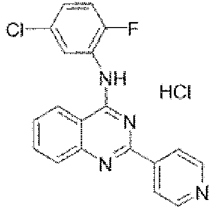 | 386,1 | 15,1 | | |
| AMR.455 | 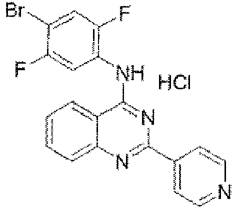 | 448,0 | 15,3 | | |
| AMR.456 | 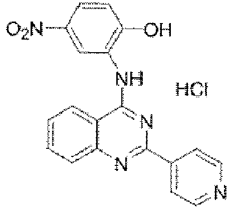 | 395,1 | 10,2 | | |
| AMR.457 | 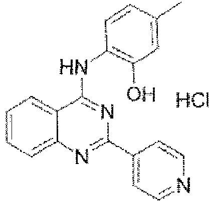 | 364,1 | > 200 | | |
| AMR.458 | 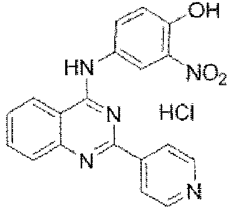 | 395,1 | 5,8 | | |
| AMR.461 | 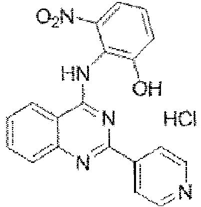 | 395,1 | 57,4 | | |
| AMR.462 | 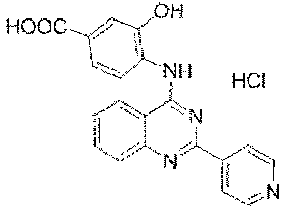 | 394,1 | 15,2 | | |
| AMR.464 | 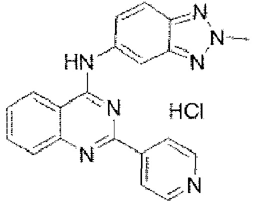 | 389,1 | 97,4 | | |
| AMR.466 | 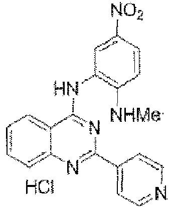 | 408,1 | 4,7 | | |
| AMR.471 | 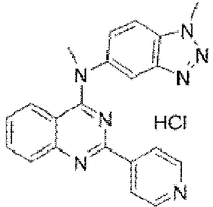 | 403,1 | > 200 | | |
| AMR.475 | 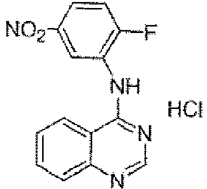 | 320,0 | > 200 | | |
| AMR.476 | 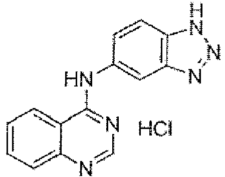 | 298,1 | 137,4 | | |
| AMR.479 | 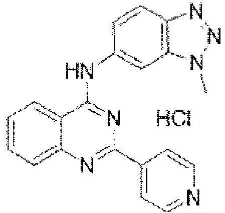 | 389,1 | 53,7 | | |
| AMR.479-MAT | 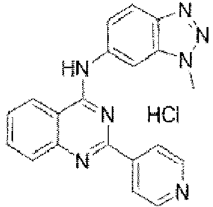 | 389,1 | 123,2 | | |
| AMR.480 | 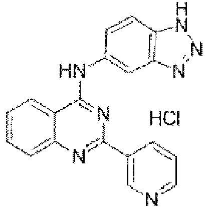 | 375,1 | 1,9 | | |
| AMR.481 | 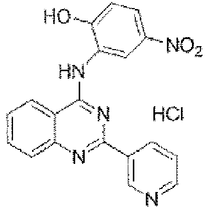 | 395,1 | 2,1 | | |
| AMR.483 | 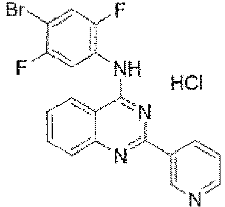 | 448,0 | 16,1 | | |
| AMR.484 | 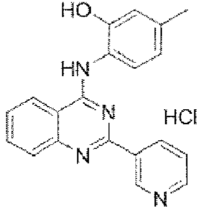 | 364,1 | 178,8 | | |
| AMR.492 | 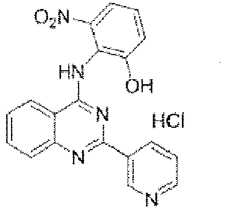 | 395,1 | 19,7 | | |
| AMR.493 | 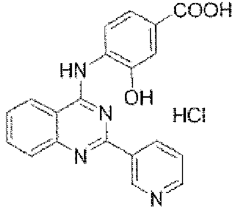 | 394,1 | 30 | | |
| AMR.494 | 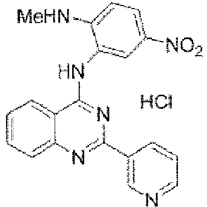 | 408,1 | 0,9 | | ~15 |
| AMR.494 HNO₃ | 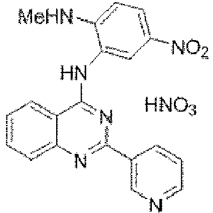 | 435,4 | | | |
| AMR.494 H₂SO₄ | 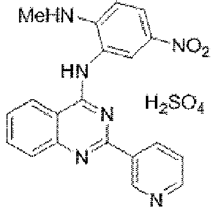 | 471,5 | | | |
| AMR.494 H₃PO₄ | 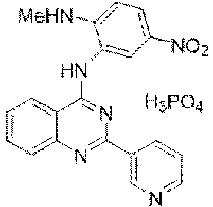 | 470,4 | | | |
| AMR.494 AcOH | 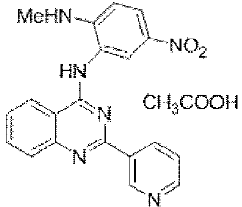 | 432,4 | | | |
| AMR.494 TFA | 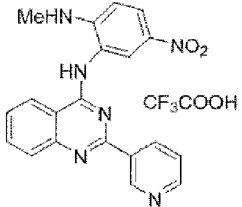 | 486,4 | | | |
| AMR.494 TfOH | 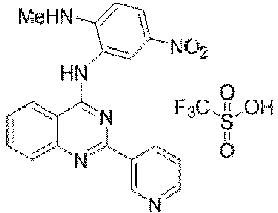 | 522,5 | | | |
| AMR.494 lactic | 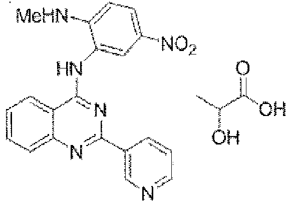 | 462,5 | | | |
| AMR.494 citric | 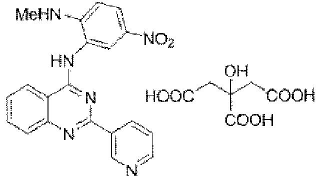 | 564,2 | | | |
| AMR.494 tartaric | 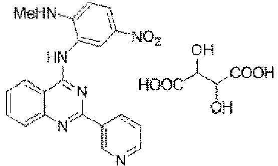 | 522,5 | | | |
| AMR.494 picolinic | 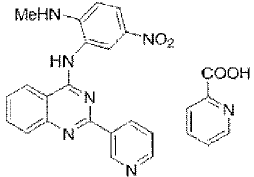 | 495,5 | | | |
| AMR.494 ascorbic | 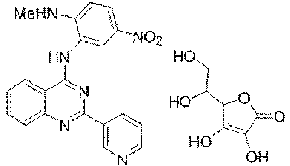 | 548,8 | | | |
| AMR.504 | 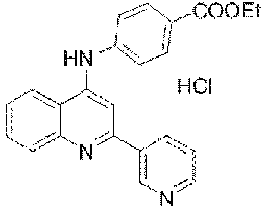 | 405,1 | 76 | | |
| AMR.505 | 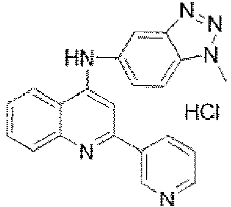 | 388,1 | 59,8 | | |
| AMR.508 | 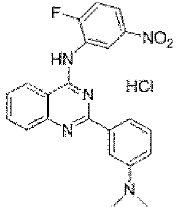 | 439,1 | > 200 | | |
| AMR.509 | 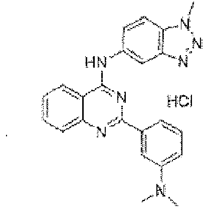 | 431,2 | > 200 | | |
| AMR.511 | 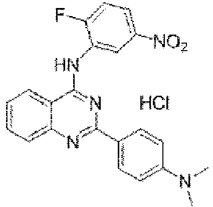 | 439,1 | > 200 | | |
| AMR.512 | 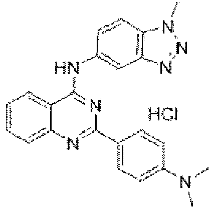 | 431,2 | 30,8 | | |
| AMR-535 | 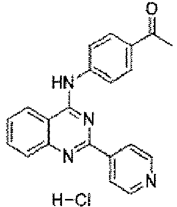 | 376,8 | | < 5 | non-toxic |
| AMR-540 | 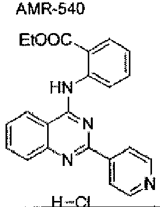 | 406,9 | | > 10 | non-toxic |
| AMR-541 | 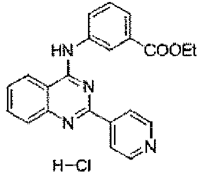 | 406,9 | | < 5 | non-toxic |
| AMR-542 | 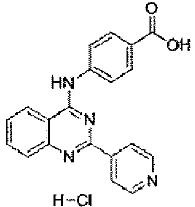 | 378,8 | | >10 | non-toxic |
| AMR-546 | 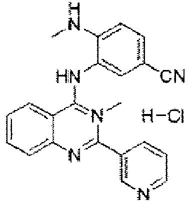 | 388,9 | | > 10 | non-toxic |
| AMR-549 | 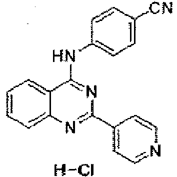 | 359,8 | | >10 | non-toxic |
| AMR-551 | 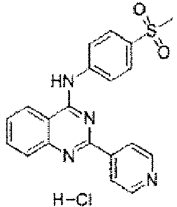 | 412,9 | | > 10 | non-toxic |
| AMR-556 | 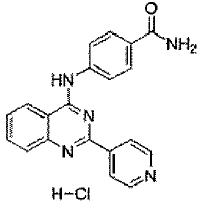 | 377,8 | | > 10 | non-toxic |
| AMR-558 | 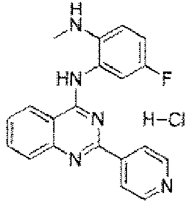 | 381,8 | | > 10 | non-toxic |
| AMR-560 | 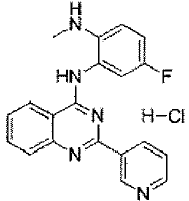 | 381,8 | | ~12 | non-toxic |
| AMR-566 | 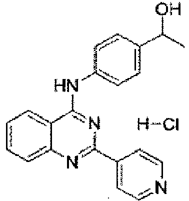 | 378,9 | | ~10 | non-toxic |
| AMR-568 | 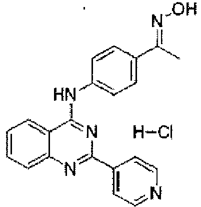 | 391,9 | | < 5 | ~7 |
| AMR-570 | 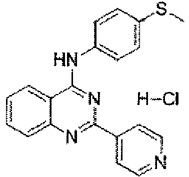 | 380,9 | | ~10 | non-toxic |
| AMR-573 | 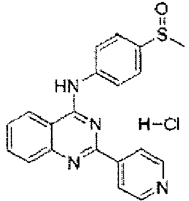 | 396,9 | | > 10 | non-toxic |
| AMR-577 | 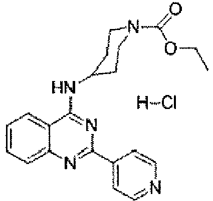 | 413,9 | | > 10 | non-toxic |
| OSR-1 | 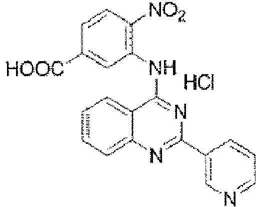 | 423,8 | | | |
| OSR-2 | 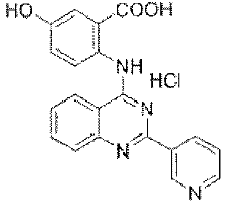 | 394,8 | | | |
| OSR-3 | 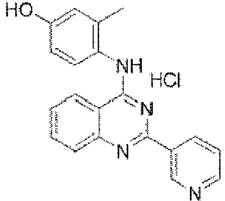 | 364,8 | | | |
| OSR-5 | 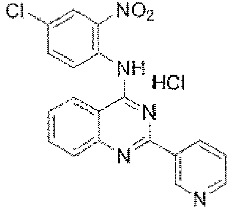 | 414,2 | | | |
| OSR-17x | 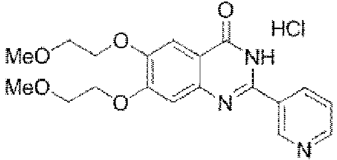 | 407,8 | | | |
| OSR-33 | 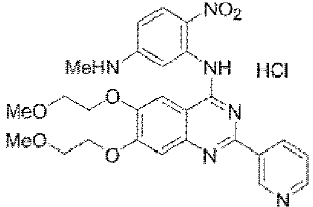 | 557,0 | | | |
| OSR-33 HNO₃ | 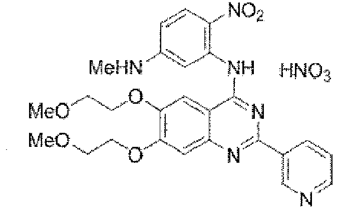 | 583,6 | | | |
| OSR-33 H₂SO₄ | 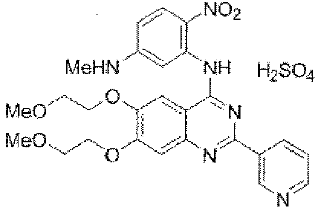 | 618,6 | | | |
| OSR-33 H3PO4 | 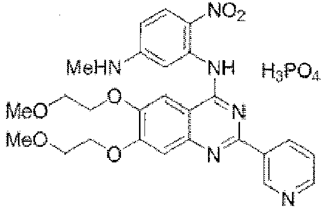 | 618,5 | | | |
| OSR-33 AcOH | 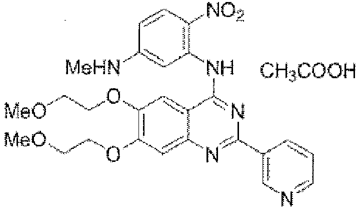 | 580,6 | | | |
| OSR-33 TFA | 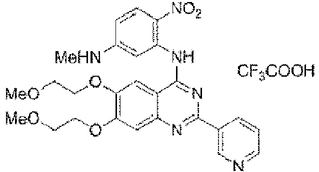 | 634,6 | | | |
| OSR-33 TfOH | 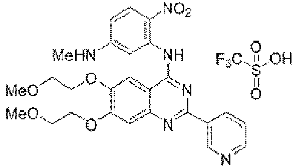 | 670,6 | | | |
| OSR-33 lactic | 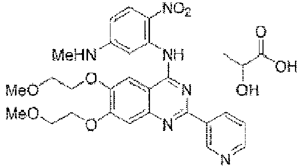 | 610,6 | | | |
| OSR-33 citric | 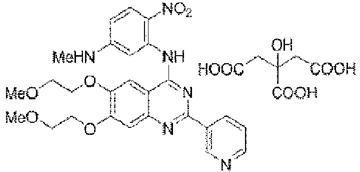 | 712,7 | | | |
| OSR-33 tartaric | 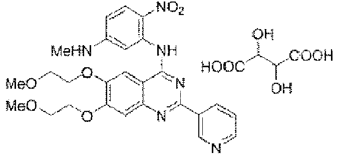 | 670,2 | | | |
| OSR-33 picolinic | 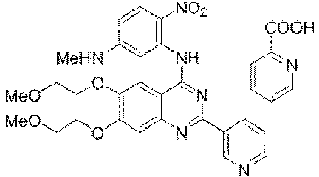 | 643,6 | | | |
| OSR-33 ascorbic | 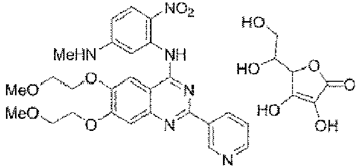 | 696,7 | | | |
| OSR-48 | 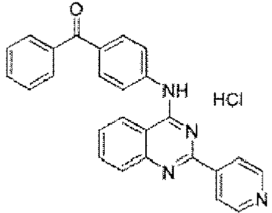 | 438,9 | | >25 | non toxic |
| OSR-72 | 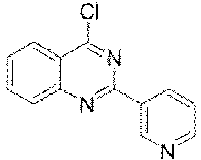 | 241,7 | | | |
| OSR-73 | 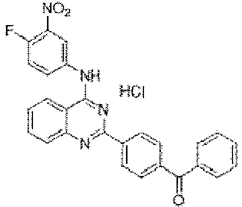 | 500,1 | | | |
| OSR-73 VI | 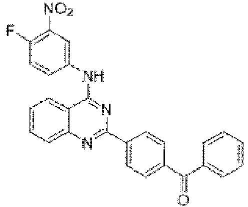 | 464,4 | | | |
| OSR-77 | 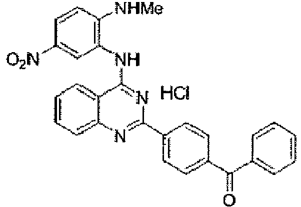 | 512,0 | | ~3 | ~7 |
| OSR-79 | 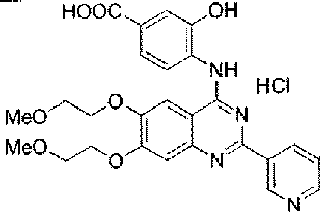 | 543,0 | | >25 | non toxic |
| OSR-79 free base | 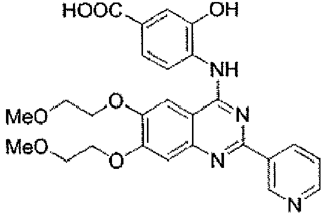 | 506,5 | | >25 | non toxic |
| OSR-85 | 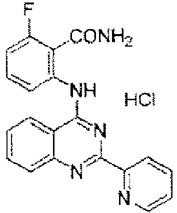 | 395,8 | | ~8 | non toxic |
| OSR-166 | 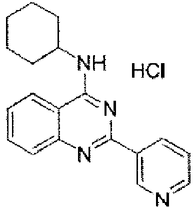 | 340,8 | | | |
| OSR-177 | 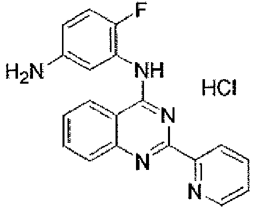 | 367,1 | | | |
| OSR-178 | 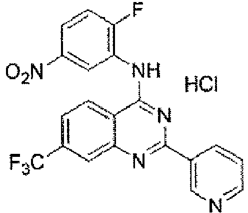 | 465,8 | | ~5 | ~11 |
| OSR-183 | 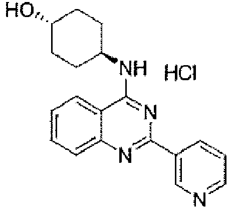 | 356,1 | | >25 | non-toxic |
| OSR-198 | 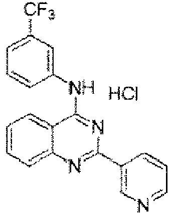 | 402,1 | | >25 | non-toxic |
| OSR-199 | 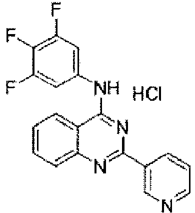 | 388,8 | | ~5 | non-toxic |
| OSR-201 | 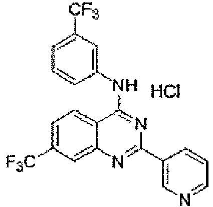 | 470,8 | | ~8 | non-toxic |
| OSR-205 | 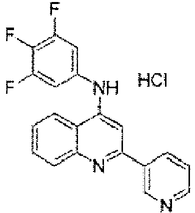 | 378,8 | | ~10 | non-toxic |
| OSR-217 | 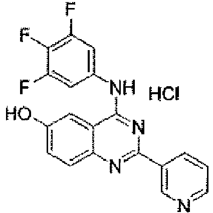 | 404,8 | | | non-toxic |
| OSR-219 | 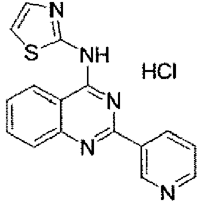 | 341,8 | | | |
| OSR-236 | 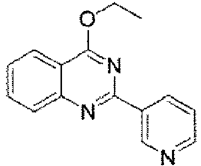 | 251,1 | | ~10-15 | non-toxic |
| OSR-242X | 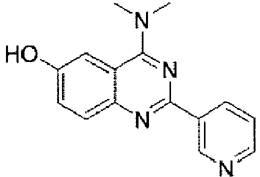 | 266,1 | | 0.5 - 1 | non-toxic |
| OSR-243 | 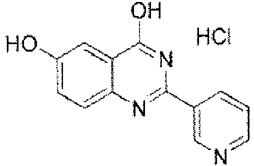 | 275,6 | | -17 | non-toxic |
| OSR-250 | 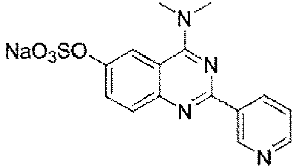 | 368,3 | | >10 | non-toxic |
| OSR-283 HCl | 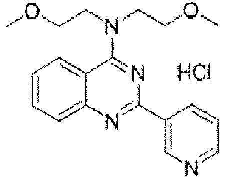 | 374,9 | | > 10 | non-toxic |
| OSR-242 HCl | 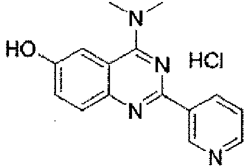 | 302,8 | | ~3 | ~20 |
| OSR-285 | 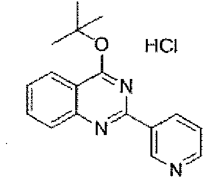 | 315,8 | | > 10 | non-toxic |
| OSR-290 | 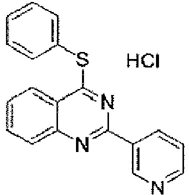 | 351,9 | | >10 | non-toxic |
| OSR-291 | 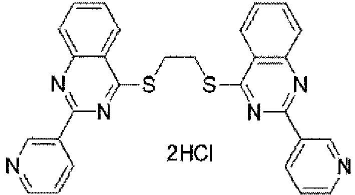 | 577,6 | insoluble in DMSO | | |
| OSR-295 | 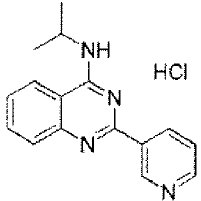 | 300,8 | | >10 | non-toxic |
| OSA-296 | 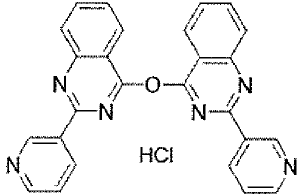 | 464,9 | | > 10 | non-toxic |
| OSR-298 | 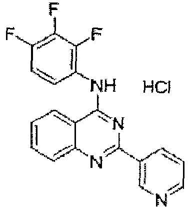 | 388,8 | | >10 | non-toxic |
| OSR-299 | 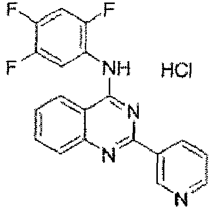 | 388,8 | | ~10 | non-toxic |
| OSR-302 | 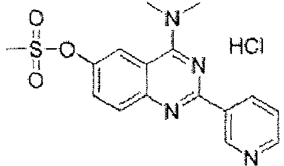 | 380,8 | | a lot of dead cells | ~7 |
| OSR-242 Na | 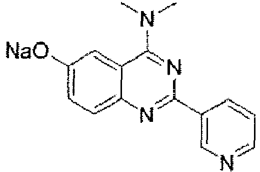 | 288,2 | | ~8 | ~20 |
| OSR-242 HCl | 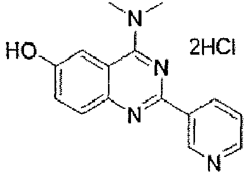 | 339,2 | | < 5 | -15 |
| OSR-306 HCl | 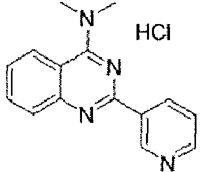 | 286,8 | | >10 | non-toxic |
| OSR-306 2HCl | 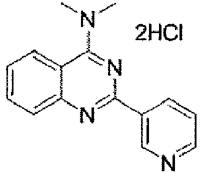 | 323,2 | | > 10 | non-toxic |
| KP-34 | 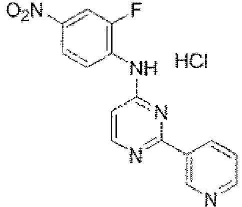 | 347,7 | | >25 | non-toxic |
| KP-43 | 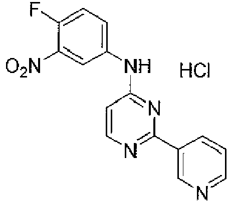 | 347,7 | | | non-toxic |
| KP-66 | 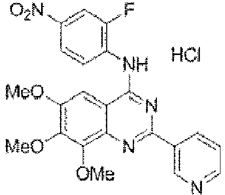 | 487,9 | | ~5 | non-toxic |
| KP-76 | 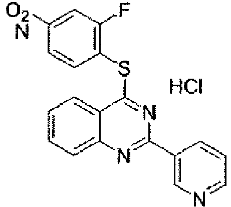 | 378,3 | | ~5 | non-toxic |
| KP-92 | 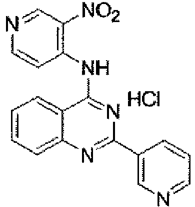 | 344,3 | | ~8 | non-toxic |
| KP-94 | 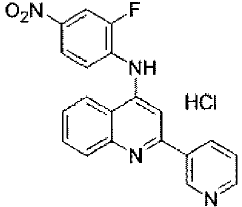 | 360,3 | | ~5 | -20 |
| KP-98 | 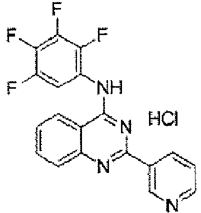 | 370,3 | | ~15 | non-toxic |
| KP-100 | 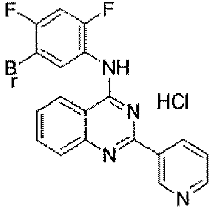 | 412,2 | | -15 | non-toxic |
| KP-107 | 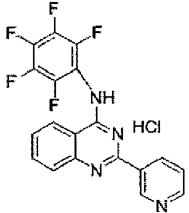 | 388,3 | | ~7 | non-toxic |
| KP-108 | 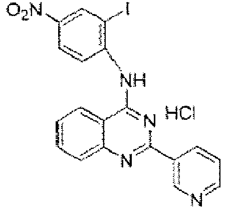 | 469,2 | | ~1.5 | ~20 |
| KP-111 | 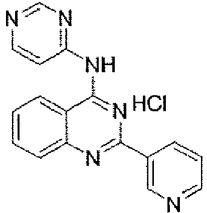 | 300,3 | | ~18 | non-toxic |
| KP-119 | 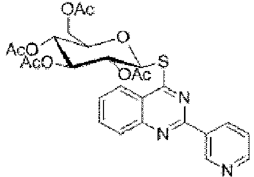 | 569,6 | | > 25 | non-toxic |
| KP-130 | 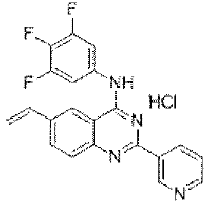 | 378,3 | | >25 | non-toxic |
| KP-139 | 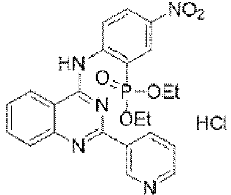 | 479,4 | | >25 | non-toxic |
| KP-152 | 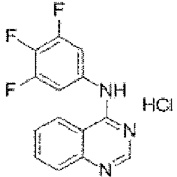 | 275,2 | | ~17 | non-toxic |
| KP-153 | 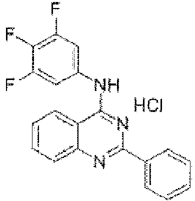 | 351,3 | | ~10-15 | non-toxic |
| KP-158 (old) | 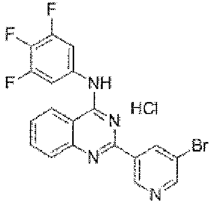 | 431,2 | | 0.5 - 1 | non-toxic |
| KP-161 | 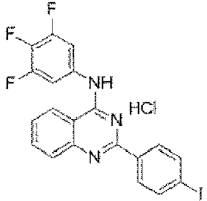 | 477,2 | | ~10 | non-toxic |
| KP-154 | 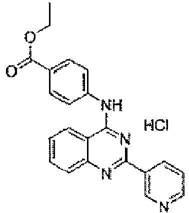 | 406,9 | | ~2 | ~15 |
| KP-158 (new pure) | 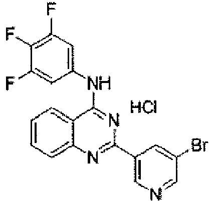 | 367,7 | | ~3 | non-toxic |
| KP-165 | 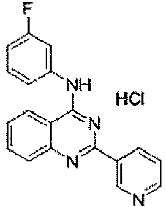 | 352,8 | | >10 | non-toxic |
| KP-179 | 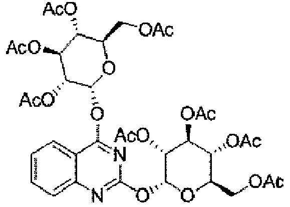 | 822,7 | | >10 | non-toxic |
| KP-187 | 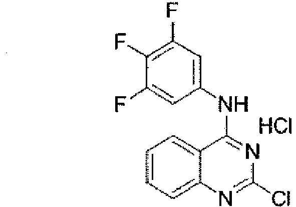 | 346,1 | | >10 | non-toxic |
| KP-190 | 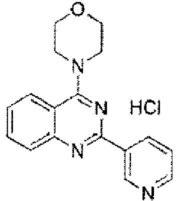 | 328,8 | | > 10 | non-toxic |
| KP-199 | 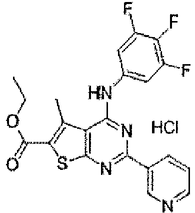 | 480,9 | | >10 | non-toxic |
| KP-207 | 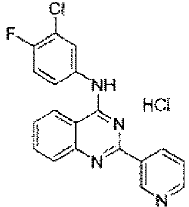 | 387,2 | | >10 | non-toxic |
| KP-216 | 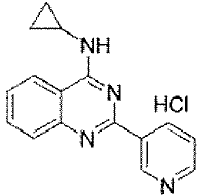 | 298,8 | | > 10 | non-toxic |
| KP-233 | 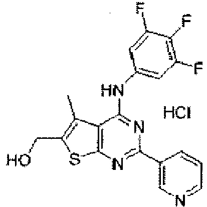 | 438,9 | | >10 | non-toxic |
| KP-236 | 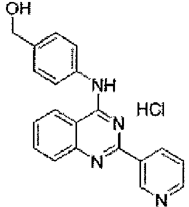 | 364,8 | | ~10 | ~10 |
| KP-237 | 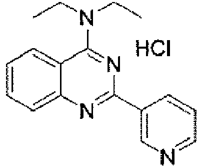 | 314,7 | | > 10 | non-toxic |
| KP-242 | 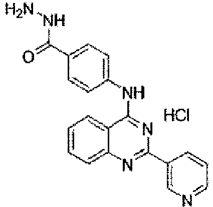 | 392,8 | | >10 | non-toxic |
| KP-251 | 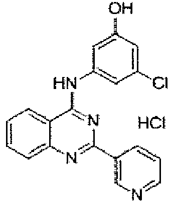 | 385,2 | | ~5 | non-toxic |
| KP-253 | 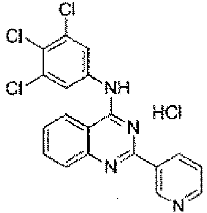 | 438,1 | insoluble in DMSO | | |
| KP-255 | 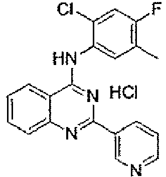 | 401,3 | | ~10 | non-toxic |
| KP-260 | 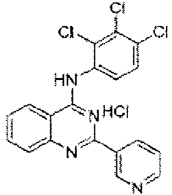 | 438,1 | | > 10 | non-toxic |
| KP-265 | 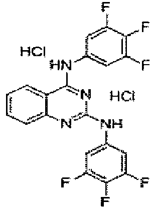 | 493,2 | | ~10 | non-toxic |
| KP-268 | 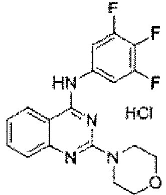 | 396,8 | | > 10 | non-toxic |
| KP-270 | 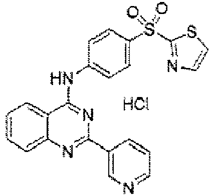 | 482 | | > 10 | non-toxic |
| KP-271 | 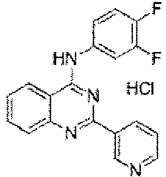 | 370,8 | | ~10 | non-toxic |
| KP-272 | 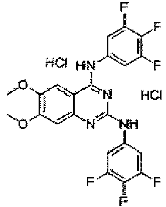 | 553,3 | | < 5 | non-toxic |
| KP-278 | 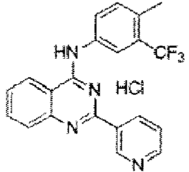 | 416,8 | | ~10 | non-toxic |
| KP-283 | 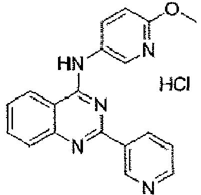 | 365,8 | | >10 | non-toxic |
| KP-288 | 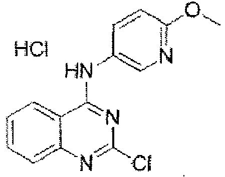 | 322,2 | | >10 | non-toxic |
| KP-291 | 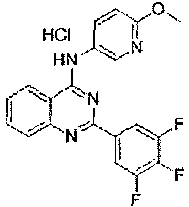 | 418,8 | | >10 | non-toxic |

**Tab. 5 IC₅₀ and CC₅₀ values of compounds inhibiting HIV-1 infectivity in tissue culture infected by the wild type NL4.3 HIV-1. Presented IC₅₀ values are based on the TZM titration, CC₅₀ values on the MTT cytotoxicity test and selectivity index (SI) was calculated as CC₅₀/IC₅₀.**

| | IC₅₀(µM) | CC₅₀(µM) | SI |
|---|---|---|---|
| OSR77 | 2 | 7.1 | 3.5 |
| OSR85 | 6.5 | 125 | 19.2 |
| OSR178 | 1.5 | 5 | 3.3 |
| OSR199 | 5.1 | 100 | 19.6 |
| OSR201 | 8.5 | 25 | 2.9 |
| AMR325 | 6.9 | 200 | 29 |
| AMR480 | 8.8 | 150 | 17.1 |
| AMR494HCl | 1.8 | 16.1 | 8.9 |

## Claims

1. As compound or a pharmaceutically acceptable salt or solvate thereof for use in inhibiting HIV capsid assembly, the compound comprising the core structure 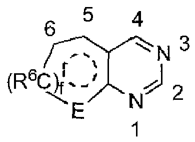 wherein E is S or CR⁷, and wherein f is 0 or 1, and wherein in case E is S, f is 0, and wherein the core structure is at least substituted in 2 and 4 position, and wherein the residue R⁶ and R⁷, are, independently of each other, selected from the group consisting of -H, -D, -alkyl, alkoxy, alkenyl, alkynyl, halides, -NO₂, - OH, - NH₂, -NHR^{4#}, -CN, -S(O)R^{4#}, -SO₂R^{4#}, -P(O)R^{4#}R^{5#}, -P(O)(OR^{4#})R^{5#}, - P(O)(OR^{4#})(OR^{5#}), -C(O)NR^{4#}R^{5#}, -C(O)SR^{4#}, -C(O)R^{4#}, -C(O)O-R^{4#}, alkoxy and glycol chains; and wherein R⁶ may optionally form a cyclic residue, with a further substituent present in 5 or 6 position, and wherein R^{4#} and R^{5#} are, independently of each other, selected from the group consisting of -H, -alkyl, -alkenyl, - heterocycloalkyl, aryl and heteroaryl.

2. The compound according to claim 1 having a structure according to formula (I) 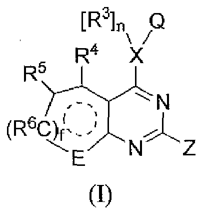 or a pharmaceutically acceptable salt or solvate thereof , wherein Z is 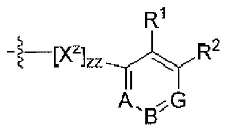 wherein X^{Z} is selected from the group consisting of O, NH, N(Me) and S, and wherein integer zz is 0 or 1, more preferably wherein Z is 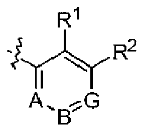 and wherein R¹ and R², are, independently of each other, selected from the group consisting of H, D, -alkyl, cycloalkyl, cycloalkenyl, heterocycloalkyl, aryl, heteroaryl, alkenyl, alkoxy, halides, -C(O)R^{1#}, -C(O)OR^{1#}, -S(O)R^{1#}, -SO₂R^{1#}, - P(O)R^{1#}R^{2#}, -P(O)(OR^{1#})R^{2#}, -P(O)(OR^{#})(OR^{2#}), -C(O)NR^{1#}R^{2#}, -C(O)NR^{1#} and - C(O)SR^{1#}, wherein R^{1#} and R2# are, independently of each other, selected from the group consisting of -H, -alkyl, -alkenyl, -heterocycloalkyl, aryl and heteroaryl,
and wherein, A is N or C-R⁸, and wherein B is N or C-R⁹, and wherein C is N or C-R¹⁰
and wherein R⁸, R⁹ and R¹⁰, are, independently of each other, selected from the group consisting of H, -D, -alkyl, cycloalkyl, cycloalkenyl, heterocycloalkyl, heterocycloalkenyl, aryl, heteroaryl, alkenyl, alkoxy, halides, -C(O)R^{1#}, -C(O)OR^{8#}, -S(O)R^{8#}, -SO₂R^{8#}, -P(O)R^{8#}R^{9#}, -P(O)(OR^{8#})R^{9#}, -P(O)(OR^{8#})(OR^{9#}), - C(O)NR^{8#}R^{9#}, -C(O)NR^{8#} and -C(O)SR^{8#}, and wherein R^{8#} and R^{9#} are, independently of each other, selected from the group consisting of -H, -alkyl, - alkenyl, -heterocycloalkyl, aryl and heteroaryl,
X is selected from the group consisting ofN, O or S,
wherein R³ is selected from the group consisting of H, alkyl, cycloalkyl, aryl, heteroaryl and heterocycloalkyl,
wherein integer n is 0 or 1,
and wherein R⁴, R⁵, are, independently of each other, selected from the group consisting of -H, -D, -alkyl, alkoxy, alkenyl, alkynyl, halides, -NO₂, -OH, -NH₂, - NHR^{4#}, -CN, -S(O)R^{4#}, -SO₂R^{4#}, -P(O)R^{4#}R^{5#}, -P(O)(OR^{4#})R^{5#},-P(O)(OR^{4#})(OR^{5#}), -C(O)NR^{4#}R^{5#}, -C(O)SR^{4#}, -C(O)R^{4#}, -C(O)O-R^{4#}, alkoxy and glycol chains;
wherein R⁴ and R⁵ or R⁵ and R⁶ can form a cyclic residue,
and wherein R^{4#} and R^{5#} are, independently of each other, selected from the group consisting of -H, -alkyl, -alkenyl, -heterocycloalkyl, aryl and heteroaryl,
and wherein Q is selected from the group consisting of alkyl, alkoxy, cycloalkyl, heterocycloalkyl, aryl, alkoxyalkyl and heteroaryl,
with the proviso that in case X is S, n is 0, and in case X is N, n is 1.

3. The compound according to claim 2, wherein R¹ and R², are, independently of each other, selected from the group consisting of C1-20alkyl, C3-10cycloalkyl, C3-10heterocycloalkyl, C3-10cycloalkenyl, C2-20alkenyl, C6-10aryl, C5-10heteroaryl, C(O)R^{1#}, C(O)OR^{1#}, -H, -D, -C1-10alkoxy, and -F, wherein R^{1#} is C₁₋₁₀alkyl.

4. The compound according to claim 2 or 3 , having a structure according to formula (II)

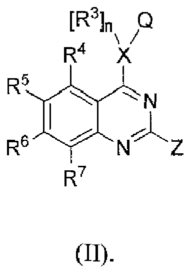

5. The compound according to any one of claims 2 to 4, wherein at least one of A, B and G is N.

6. The compound according to any one of claims 2 to 5, wherein X is N, n is 1 and R³ is H or alkyl, preferably H or methyl, more preferably H.

7. The compound according to any one of claims 2 to 6, wherein Q is 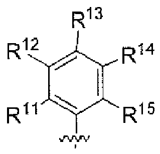 wherein R¹¹, R¹², R¹³, R¹⁴, and R¹⁵ are, independently of each other, selected from the group consisting of -H, -alkyl, -halides, alkoxy, alkynyl, alkenyl, -NO₂, -CN, - C(=Y)- R^{11#}, -C(=Y)-H, C(=Y)-Y¹-R^{11#}, -C(=Y)-OH, , -Y-R^{11#}, -NH₂, -NHR^{11#}, - CN, -F, -CF₃, -S(O)R^{11#}, -SO₂R^{11#}, -P(O)R^{11#}R^{12#}, -P(O)(OR^{11#})R^{12#}, - P(O)(OR^{11#})(OR^{12#}),
wherein Y is O, S or NH, and wherein Y¹ is O, S or NR*, wherein R* is H or alkyl, and wherein R^{11#} and R^{12#} are, independently of each other, selected from the group consisting of -H, -alkyl, -alkenyl, -heterocycloalkyl, aryl and heteroaryl.

8. The compound according to claim 5, wherein at least one of R¹¹, R¹², R¹³, R¹⁴, and R¹⁵ is selected from the group consisting of -F, -I, NO₂, -C(=O)-alkyl, -C(=O)-OH, -C(=O)-O-alkyl, -C(=Y)-OH and -NH-alkyl.

9. The compound according to claim 6 or 7, wherein at least one of R¹¹, R¹², R¹³, R¹⁴, and R¹⁵ is -F.

10. The compound according to any of claims 2 to 8, wherein Q is selected from the group consisting

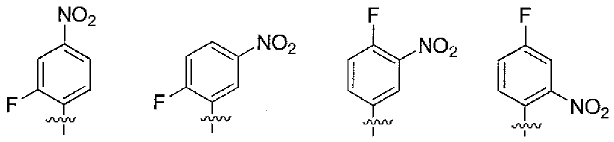

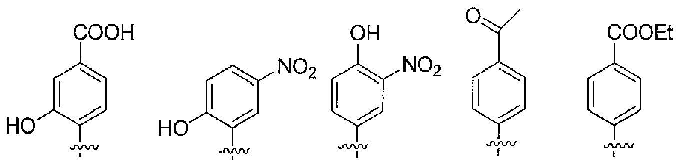

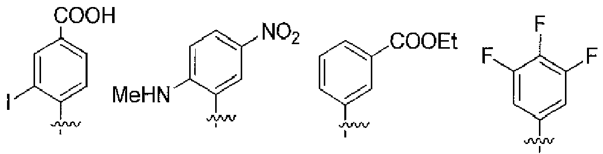

11. The compound according to any one of claims 2 to 6, wherein Q 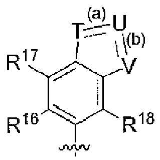 wherein T is -C-R¹⁹, N or N-Y^{T}, with Y^{T} being H or alkyl wherein U is -C-R²⁰, N or N-Y^{U}, with Y^{U} being H or alkyl wherein V is -C-R²¹, N or N-Y^{Y}, with Y^{V} being H or alkyl the bond (a) is a single or double bond,
the bond (b) is a single or double bond,
with eth proviso that one of (a) and (b) is a double bond,
R¹⁹, R²⁰, and R²¹ are, independently of each other H or -alkyl,
and wherein R¹⁶, R¹⁷ and R¹⁸, are independently of each other selected from the group consisting of -H, -alkyl, -halides, alkoxy, alkynyl, alkenyl, -NO₂, -C(=Y)-alkyl, -C(=Y)-H, -C(=Y)-Y¹-alkyl, -C(=Y)-OH and -Y-alkyl,
preferably wherein Q is selected from the group consisting of

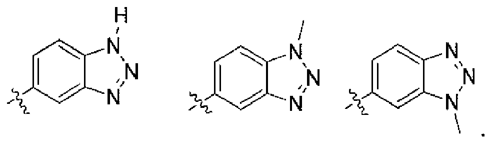

12. The compound according to any one of claims 2 to 12, wherein Z is selected from
the group consisting of

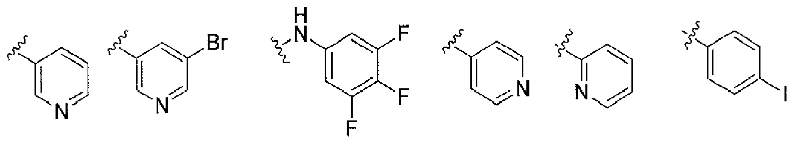

13. A compound according to any one of claims 1 to 12, wherein HIV is HIV-1 capsid assembly

14. A pharmaceutical composition comprising a compound according to any one of claims 1 to 12.

15. Use of a compound as defined in any one of claims 1 to 12 for the manufacture of a medicament for inhibiting HIV capsid assembly.

16. A method of treating a patient suffering from HIV comprising administering a therapeutically effective amount of the compound as defined in any one of claims 1 to 12.
